(19) Europäisches Patentamt
European Patent Office
Office européen des brevets

(11) **EP 4 212 538 A1**

(12) **EUROPEAN PATENT APPLICATION**
published in accordance with Art. 153(4) EPC

(43) Date of publication:
**19.07.2023 Bulletin 2023/29**

(21) Application number: **21865622.1**

(22) Date of filing: **23.06.2021**

(51) International Patent Classification (IPC):
***C07F 9/564*** *(2006.01)* ***A61K 31/06*** *(2006.01)*
***A61K 31/664*** *(2006.01)* ***A61P 35/00*** *(2006.01)*

(52) Cooperative Patent Classification (CPC):
(C-Sets available)
**C07F 9/564; A61K 31/664; A61K 45/06;**
**A61P 35/00; C07F 9/65583** (Cont.)

(86) International application number:
**PCT/CN2021/101870**

(87) International publication number:
**WO 2022/052564 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.09.2020 PCT/CN2020/114519**

(71) Applicant: **Ascentawits Pharmaceuticals, Ltd.**
**Shenzhen, Guangdong 518118 (CN)**

(72) Inventors:
- **LI, Anrong**
  **Shenzhen, Guangdong 518118 (CN)**
- **DUAN, Jianxin**
  **Shenzhen, Guangdong 518118 (CN)**
- **MENG, Fanying**
  **San Francisco, California 94121 (US)**
- **CAI, Xiaohong**
  **Shenzhen, Guangdong 518118 (CN)**
- **QI, Tianyang**
  **Shenzhen, Guangdong 518118 (CN)**

(74) Representative: **Haseltine Lake Kempner LLP**
**One Portwall Square**
**Portwall Lane**
**Bristol BS1 6BH (GB)**

(54) **HYPOXIA-ACTIVATED DNA ALKYLATING AGENT AND MEDICAL USE THEREOF**

(57) The present invention relates to hypoxia-activated DNA alkylating agents having any one of the following structural formulas, or a pharmaceutically acceptable salt, a prodrug, a solvate or an isotopic variant thereof, as well as an anticancer medical use thereof.

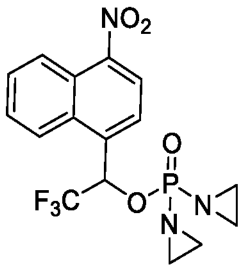 , 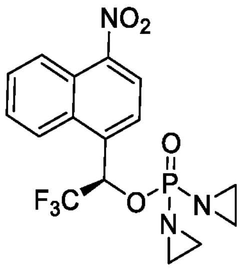 , 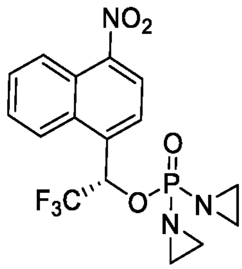 ,

EP 4 212 538 A1

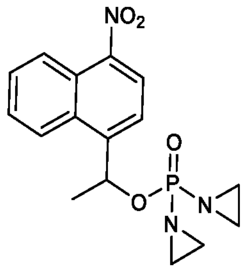,
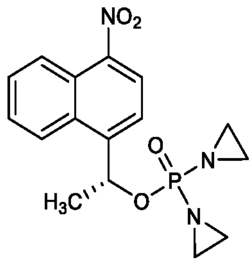,
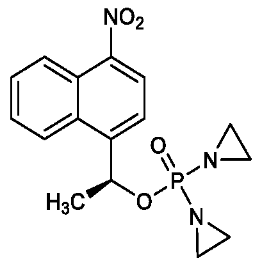.

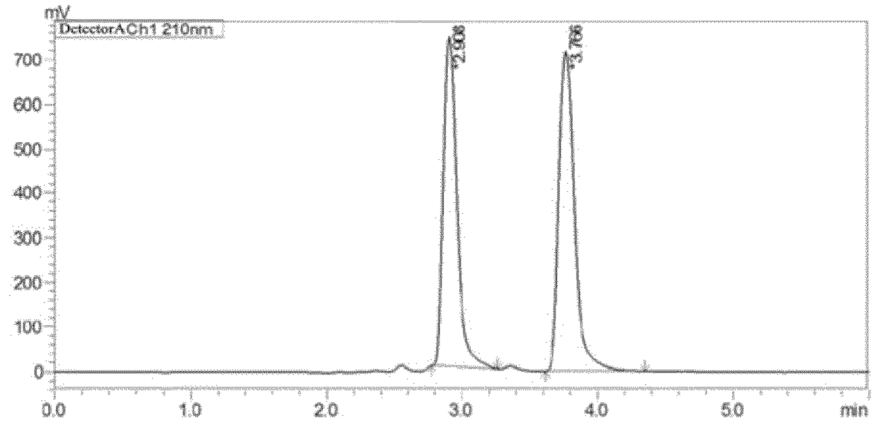


Figure 1

(52) Cooperative Patent Classification (CPC): (Cont.)

C-Sets
**A61K 31/664, A61K 2300/00**

## Description

### Technical Field

The present invention relates to compounds for cancer therapy and belongs to the field of drug small molecule development.

### Background Art

TH-302 (Evofosfamide, cas NO. 918633-87-1) is a 2-nitroimidazole-initiated hypoxic activated prodrug (HAP) bromoisophosphoramide. In the case of hypoxia, the inactive TH-302 prodrug can release highly toxic Br-IPM (as shown in Structural formula 1 below). TH-302 has a broad spectrum of biological activities in vitro and in vivo and a specific hypoxia-selective activation activity, and induces H2AX phosphorylation, DNA cross-linking activity, thereby leading to cell cycle arrest; thus this compound has been used for the development of anticancer drugs by several pharmaceutical companies and scientific research institutes.

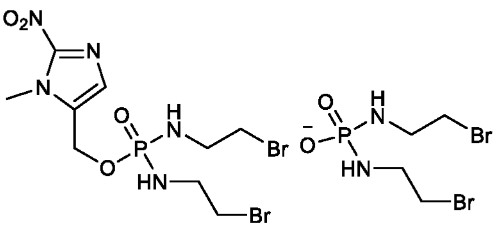
TH-302 Br-IPM

Structural formula 1

After purchasing TH-302-related research project from Threshold Pharmaceutical Company, Merck & Company, Inc. conducted a phase III clinical trial of anti-tumor drug TH-302 to investigate the therapeutic effect of this drug on soft tissue sarcoma and pancreatic cancer. However, in 2015, Merck & Company, Inc. announced that the trial failed. Threshold Pharmaceutical Company indicated that the failure of TH-302 clinical trial showed that this drug could not effectively target tumor for a long time to become a stable anti-tumor drug.

### Summary of the Invention

The present invention provides hypoxic activated small molecule compounds that can enter the brain or other central nervous system, the mechanism of action of which is to inhibit, kill tumor cells by releasing DNA alkylating agents through the hypoxic activation mechanism as described above.

In particular, during conducting structural optimization and activity testing of similar small molecules, the inventors team unexpectedly found that compounds of the following structures have excellent anticancer activity and can be activated under hypoxic conditions:

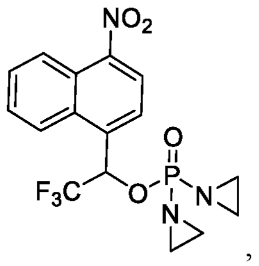
,
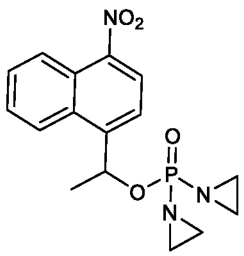
.

The tests showed that these compounds had strong inhibition activity on cancer cell proliferation in vitro, especially under hypoxic conditions; these compounds have been tested and shown not to be substrates for Pgp (P-Glycoprotein); and the tests in serum and liver microsomes and animal models further showed that the compound had better stability and removal and inhibition effect on tumor. Compared to TH-302 drugs, the hypoxia-activated DNA

alkylating agents provided by the present invention are more suitable for development as drugs for the treatment of cancers or tumors of the central nervous system, with greater efficacy.

Based on the above findings, the present invention provides the following hypoxia-activated DNA alkylating agents and makes clear that they have related anticancer, antitumor medical uses.

The hypoxia-activated DNA alkylating agent, which is a compound of the following structural formula, or a pharmaceutically acceptable salt, a solvate, or an isotopic variant thereof,

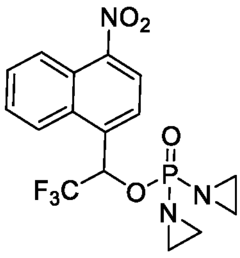 , 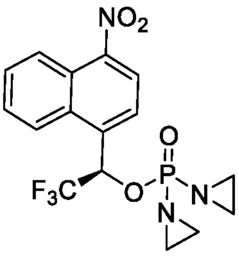 , 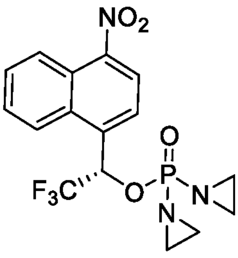 ,

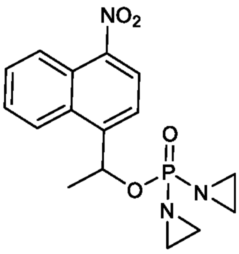 , 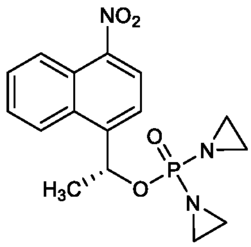 , 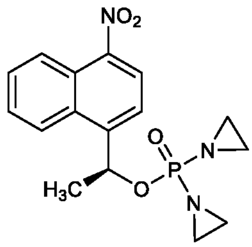 .

The present invention provides pharmaceutically acceptable salts of the compounds as described above. The salts may be basic salts, including the salts of the compounds with an inorganic base (such as alkali metal hydroxide or alkaline earth metal hydroxide, and the like) or with an organic base (such as monoethanolamine, diethanolamine or triethanolamine, and the like). Alternatively, the salts may be acid salts, including the salts of the compounds formed with an inorganic acid (such as hydrochloric acid, hydrobromic acid, hydroiodic acid, nitric acid, perchloric acid, sulfuric acid or phosphoric acid, and the like) or with an organic acid (such as methanesulfonic acid, trifluoromethanesulfonic acid, ethanesulfonic acid, benzenesulfonic acid, p-toluenesulfonic acid, fumaric acid, oxalic acid, maleic acid or citric acid, and the like). It is a well-known technology in the art to select and prepare acceptable salts, solvates, and the like of a compound.

With respect to the compounds herein, they may also be used in the form of solvates, i.e. the present invention provides pharmaceutically acceptable solvates of the above compounds. The solvates are hydrates, alcoholates, and the like. The alcoholates include ethanolates.

Since the drug API may be combined with solvents during the production and refining and purification processes, solvates are finally formed.

The isotopic variant corresponds to a deuterated compound selected from the following structures:

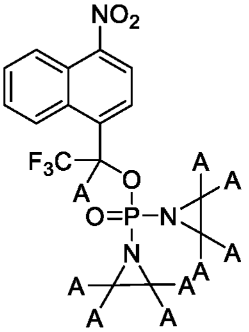 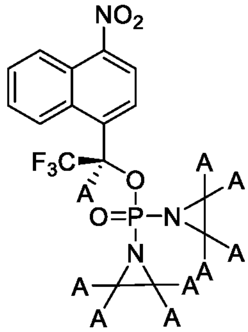 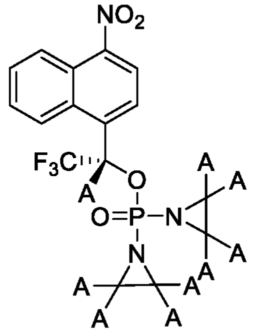 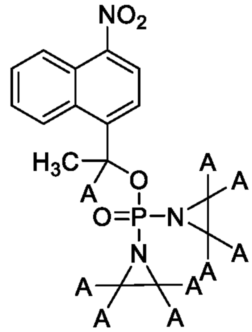 ;

wherein each A is independently H or D, and at least one of the nine A is D.

The isotopic variant corresponds to a deuterated compound selected from the following structures:

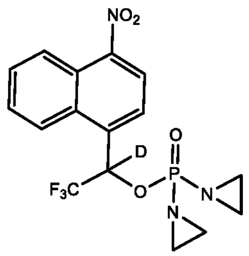, 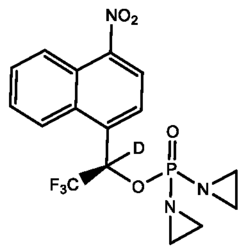, 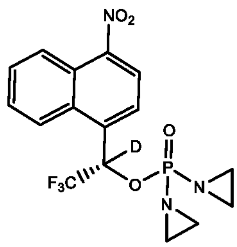, 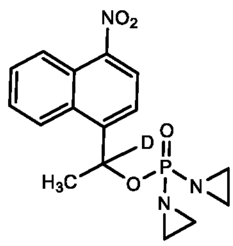,

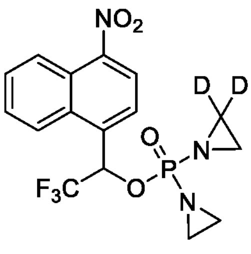, 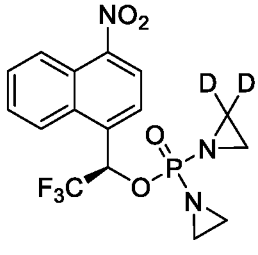, 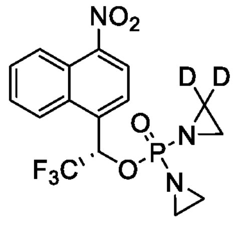, 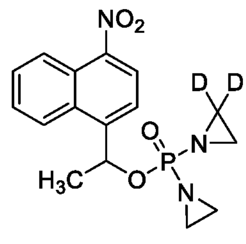,

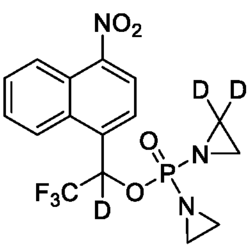, 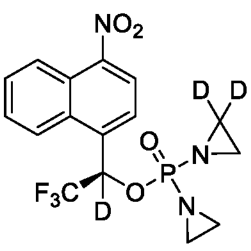, 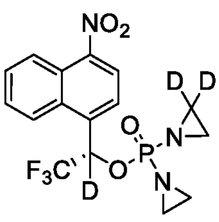, 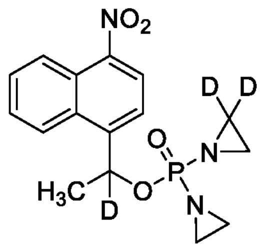,

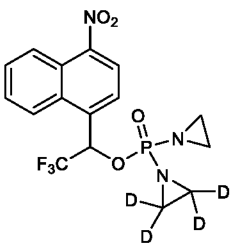, 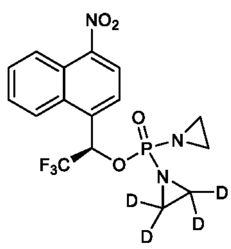, 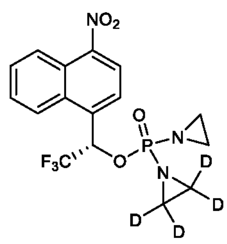, 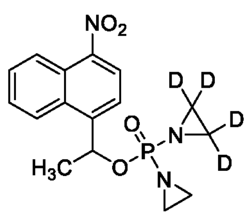,

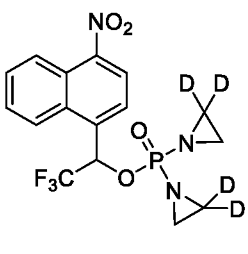, 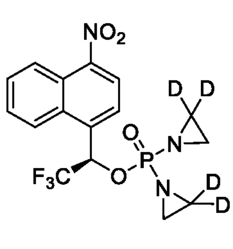, 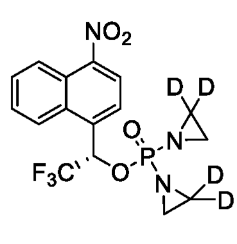, 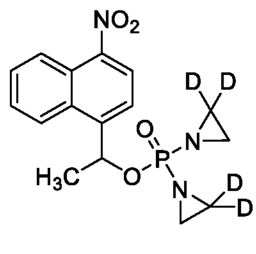,

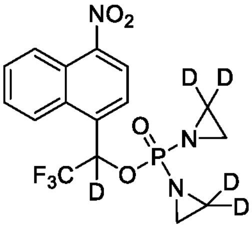, 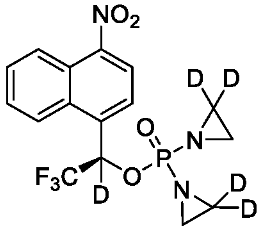, 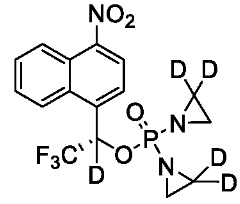, 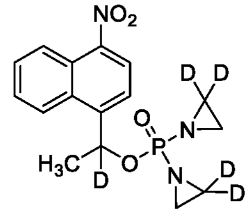,

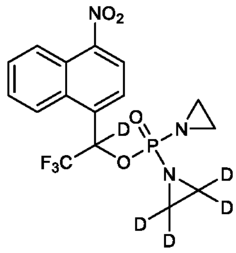 , 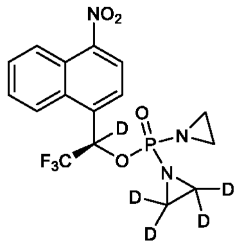 , 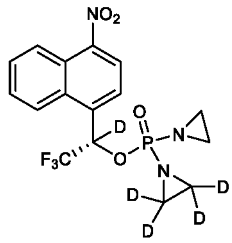 , 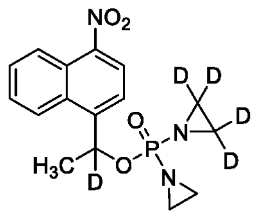 ,

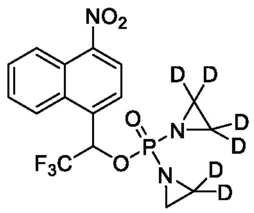 , 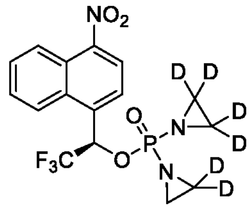 , 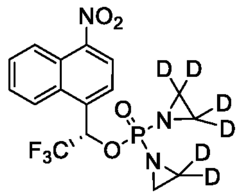 , 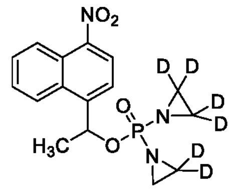 ,

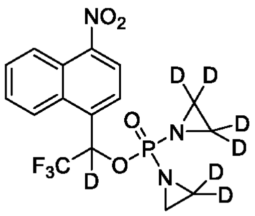 , 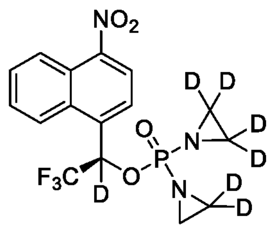 , 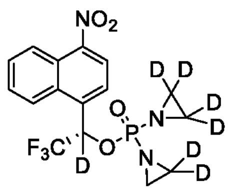 , 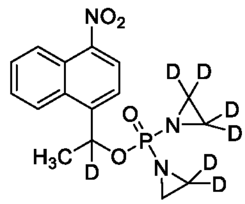 ,

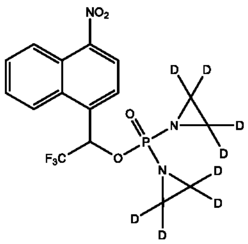 , 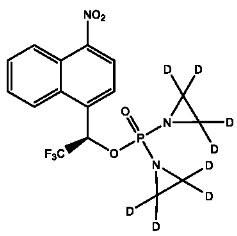 , 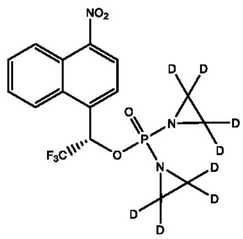 , 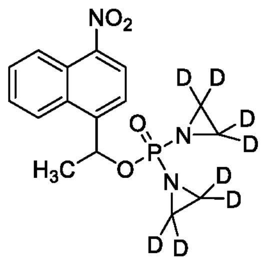 ,

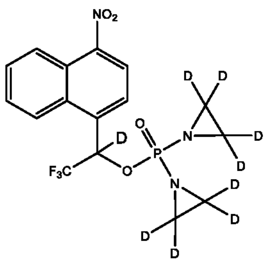 , 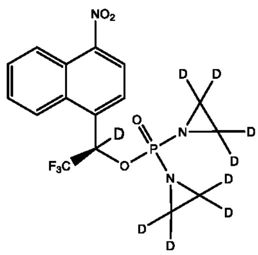 , 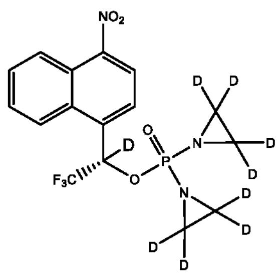 , 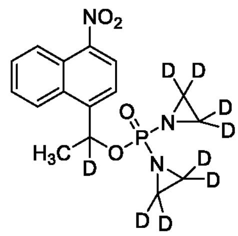 .

The present invention also provides a drug comprising the DNA alkylating agents as described above.

The drugs containing the DNA alkylating agents as described above, which are used to treat cancer, tumor, conditions caused by cancer or tumor, or cell proliferative diseases.

Among them are cancers or tumors including: lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous cell carcinoma, basal cell carcinoma, adenocarcinoma, sweat gland carcinoma, sebaceous gland carcinoma, papillary carcinoma, papillary adenocarcinoma, adenocarcinoma cystic, cystic carcinoma, medullary carcinoma, bronchial carcinoma, osteocyte carcinoma, epithelial carcinoma, carcinoma of bile duct, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cords neuroma,

meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, choriocarcinoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, endothelioma, endoblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, anemia of chronic disease, polycythemia, lymphoma, endometrial cancer, glioma, colorectal cancer, thyroid cancer, urothelial cancer or multiple myeloma.

Preferably, the cancer or tumor is preferably non-small cell lung cancer, pancreatic cancer, colorectal cancer, liver cancer, gastric cancer.

Further, the cancer or tumor is primary brain cancer or tumor, or metastatic cancer or tumor that metastasizes to the brain.

Use of the DNA alkylating agents as described above in the preparation of a drug for the treatment of cancer, tumor, conditions caused by cancer or tumor, or cell proliferative diseases.

Further, the patient with cancer or tumor is the patient with impaired DNA repair.

The patient with cancer or tumor is the patient with impaired DNA repair, wherein the impaired DNA repair is one or more of impaired homologous recombination DNA repair enzymes, impaired nucleotide excision repair enzymes, impaired non-homologous end joining enzymes, impaired base excision repair enzymes, impaired mismatch repair enzymes, or at least one impaired repair enzyme in a fanconi anemia pathway.

Further, the patient with cancer or tumor is the patient with BRCA2 gene mutation.

The present invention also provides use of the DNA alkylating agents as described above in the preparation of a drug for the treatment of cancer, tumor, conditions caused by cancer or tumor, or cell proliferative diseases.

Among them, the cancer or tumor is preferably non-small cell lung cancer, pancreatic cancer, colorectal cancer, liver cancer, gastric cancer.

The patient with cancer or tumor is the patient with impaired DNA repair.

The patient with cancer or tumor is the patient with impaired DNA repair, wherein the impaired DNA repair is one or more of impaired homologous recombination DNA repair enzymes, impaired nucleotide excision repair enzymes, impaired non-homologous end joining enzymes, impaired base excision repair enzymes, impaired mismatch repair enzymes, or at least one impaired repair enzyme in a fanconi anemia pathway.

The patient with cancer or tumor is the patient with BRCA2 gene mutation.

At the same time, the hypoxic anticancer compounds according to the present invention can be used in combination with four anticancer drugs to enhance the anticancer effect.

First, it is used in combination with traditional chemotherapy drugs. The traditional chemotherapeutic drugs can only kill tumor cells under normoxia, but have no effect on hypoxic cells. These traditional chemotherapeutic drugs such as docetaxel (Vanhoefer U, Cao S, Harstrick A, Seeber S, Rustum YM (1997) Comparative antitumor efficacy of docetaxel and paclitaxel in nude mice bearing human tumor xenografts that overexpress the multidrug resistance protein(MRP). Ann Oncol 8: 1221-1228), cisplatin(Bigioni M, Benzo A, Irrissuto C, Lopez G, Curatella B, Maggi CA, Manzini S, Crea A, Caroli S, Cubadda F, Binaschi M (2008) Antitumor effect of combination treatment with Sabarubicin (MEN 10755) and cis-platin (DDP) in human lung tumor xenograft. Cancer Chemother Pharmacol 62: 621-629), pemetrexed (Huber PE, Bischof M, Jenne J, Heiland S, Peschke P, Saffrich R, Grone HJ, Debus J, Lipson KE, Abdollahi A (2005) Trimodal cancer treatment: beneficial effects of combined antiangiogenesis, radiation, and chemotherapy. Cancer Res 65: 3643-3655), irinotecan (Houghton JA, Cheshire PJ, Hallman JD, 2nd, Lutz L, Luo X, Li Y, Houghton PJ (1996) Evaluation of irinotecan in combination with 5 -fluorouracil or etoposide in xenograft models of colon adenocarcinoma and rhabdomyosarcoma. Clin Cancer Res 2: 107-118), doxorubicin (Kraus-Berthier L, Guilbaud N, Jan M, Saint-Dizier D, Rouillon MH, Burbridge MF, Pierre A, Atassi G (1997) Experimental antitumor activity of S 16020-2 in a panel of human tumors. Eur J Cancer 33: 1881-1887), gemcitabine (Merriman RL, Hertel LW, Schultz RM, Houghton PJ, Houghton JA, Rutherford PG, Tanzer LR, Boder GB, Grindey GB (1996) Comparison of the antitumor activity of gemcitabine and ara-C in a panel of human breast, colon, lung and pancreatic xenograft models. Invest New Drugs 14: 243-247; Teicher BA, Chen V, Shih C, Menon K, Forler PA, Phares VG, Amsrud T (2000) Treatment regimens including the multitargeted antifolate LY231514 in human tumor xenografts. Clin Cancer Res 6: 1016-1023), temozolomide (temozolomide Middleton MR, Thatcher N, McMurry TB, McElhinney RS, Donnelly DJ, Margison GP (2002) Effect of O6-(4-bromothenyl)guanine

on different temozolomide schedules in a human melanoma xenograft model. Int J Cancer 100: 615-617) in combination with hypoxic activated compounds have killing effect on all tumor cells (Liu et al, TH-302, a hypoxia-activated prodrug with broad in vivo preclinical combination therapy efficacy: optimization of dosing regimens and schedules, Cancer Chemother Pharmacol, 2012, 69: 1487-1498, DOI: 10.1007/s00280-012-1852-8).

Second, in combination with anti-angiogenic drugs leads to an increase in the hypoxic areas of tumor cells and thus an increase in the number of tumor cells that are sensitive to the hypoxic compounds (Chang et al, Sorafenib (BAY43-9006) inhibits tumor growth and vascularization and induces tumor apoptosis and hypoxia in RCC xenograft models, Cancer Chemother Pharmacol, 2007, 59(5): 561-74. DOI: 10.1007/s00280-006-0393-4). Anti-angiogenic drugs that have been demonstrated by experiments include sorafenib (Chang et al, Sorafenib (BAY43-9006) inhibits tumor growth and vascularization and induces tumor apoptosis and hypoxia in RCC xenograft models, Cancer Chemother Pharmacol, 2007, 59(5): 561-74. DOI: 10.1007/s00280-006-0393-4), rapamycin (Su D, Stamatakis L, Singer EA, Srinivasan R.Renal cell carcinoma: molecular biology and targeted therapy. Curr Opin Oncol 2014(26): 321-7), mTOR inhibitor (Sun et al Combination treatment with hypoxia-activated prodrug evofosfamide (TH-302) and mTOR inhibitors results in enhanced antitumor efficacy in preclinical renal cell carcinoma models, Am J Cancer Res. 2015(5): 2139). These drugs combined or administered in combination with the hypoxic compounds of the present invention can induce an increase in the hypoxic areas of tumor cells and thus an increase in the number of tumor cells that are sensitive to the hypoxic compounds. Thus, the combined administration can exert a synergistic anti-cancer effect.

Third, in combination with the cell checkpoint inhibitor leads to an increase in the sensitivity of tumor cells to DNA alkylating agents (Meng et al, Enhancement of hypoxia-activated prodrug TH-302 anti-tumor activity by Chk1 inhibition, BMC Cancer, 2015, 15: 422, DOI: 10.1186/s12885-015-1387-6). Cell checkpoint inhibitor drugs that have been demonstrated by experiments include LY2603618 (Wang F Z, Fei H R, Cui, Y J et al. The checkpoint 1 kinase inhibitor LY2603618 induces cell cycle arrest, DNA damage response and autophagy in cancer cells. Apoptosis, 2014(19): 1389-1398), PF477736 (Balsina et al Breaching the DNA damage checkpoint via PF-00477736, a novel small-molecule inhibitor of checkpoint kinase, Mol Cancer Ther 2008; 7(8): 2394-404), AZD7762 (Morgan et al; Mechanism of radiosensitization by the Chk1/2 inhibitor AZD7762 involves abrogation of the G2 checkpoint and inhibition of homologous recombinational DNA repair, Cancer Researh 2010(70): 4972). These drugs combined or administered in combination with the hypoxic compounds of the present invention can induce an increase in the sensitivity of tumor cells to DNA alkylating agents (the compound of the present invention is a prodrug compound, and the prodrugs that act after metabolism are DNA alkylating agents are the active compounds that act after metabolism of the prodrug compound), thereby enhancing the effect of the DNA alkylating agents of the present invention. Thus, the combined administration can exert a synergistic anti-cancer effect.

Fourth, it is used in combination with immunosuppressive agents. Hypoxic compounds can decrease the tumor hypoxic areas and thus increase the infiltration of lymphocytes in the hypoxic areas, leading to an increased effect on immunotherapy (Jayaprakash et al, Targeted hypoxia reduction restores T cell infiltration and sensitizes prostate cancer to immunotherapy, JCI, 2018(128): 5137, DOI: 10.1172/JCI96268.). Immunosuppressive agent drugs that have been demonstrated by experiments include $\alpha$CTLA-4/$\alpha$PD-1(Curran et al. PD-1 and CTLA-4 combination blockade expands infiltrating T cells and reduces regulatory T and myeloid cells within B16 melanoma tumors. Proc Natl Acad Sci USA. 2010, 107(9): 4275-4280), these drugs combined or administered in combination with the hypoxic compounds of the present invention can increase the infiltration of lymphocytes in the hypoxic areas, leading to increased effect on immunotherapy.

The present invention also provides a combined drug comprising the DNA alkylating agent as described above and

a. a conventional chemotherapeutic drug;

b. an anti-angiogenic drug;

c. a cell checkpoint inhibitor; or

d. an immunosuppressive agent.

The above combined drug can be a combined drug containing two active ingredients comprising the DNA alkylating agent according to the present invention and any one of a, b, c, or d, or a combined drug containing three active ingredients comprising the DNA alkylating agent according to the present invention and any two of a, b, c, or d, or a combined drug containing four active ingredients comprising the DNA alkylating agent according to the present invention and any three of a, b, c, or d, or a combined drug containing five active ingredients comprising the DNA alkylating agent according to the present invention and any four of a, b, c, and d.

The present invention also provides a combined therapy for the treatment of cancers or tumors, which comprises

administering to the patient a drug containing the DNA alkylating agent as described above; and

a. administering a conventional chemotherapeutic drug;

b. administering an anti-angiogenic drug;

c. administering a cell checkpoint inhibitor; or

d. administering an immunosuppressive agent.

The above combined therapy is a combined therapy in which the patient is administered the DNA alkylating agent of the present invention and any one of a, b, c, d, which may be administered in any order in which the DNA alkylating agent is administered first or any one of a, b, c, d is administered first; or a combined therapy in which the patient is administered the DNA alkylating agent of the present invention and any two of a, b, c, d, the order of administration of which may be adjusted in any order; or a combination therapy in which the patient is administered the DNA alkylating agent of the present invention and any three of a, b, c, d, the order of administration of which may be adjusted in any order; or a combination therapy in which the patient is administered the DNA alkylating agent of the present invention and four of a, b, c, d, the order of administration of which may be adjusted in any order.

The concepts of conventional chemotherapeutic drugs, anti-angiogenic drugs, cell checkpoint inhibitors, and immunosuppressive agents are as described above.

The present invention also provides a method for preparing a hypoxia-activated DNA alkylating agent of the following structure I, comprising subjecting compound I-l to a ring closure reaction to give the corresponding compound I:

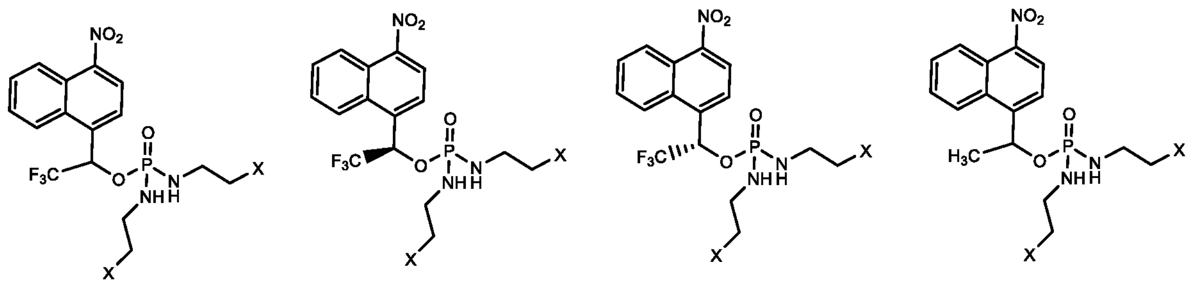

I-1

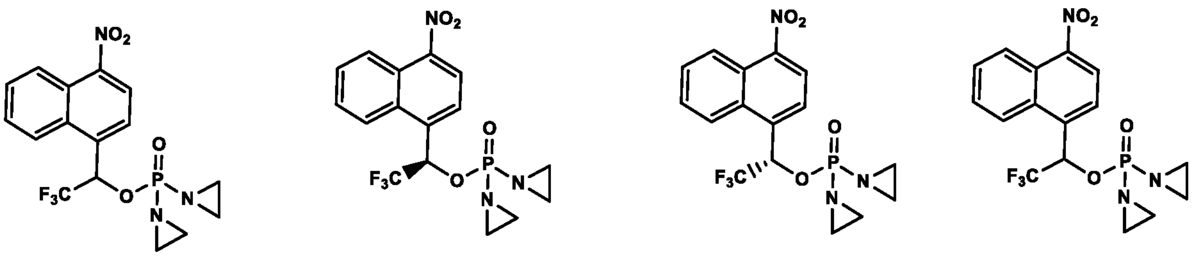

I

wherein X is independently F, Cl, Br, or I. Preferably, all of X is Br.

Preferably, the ring closure reaction uses silver oxide or silver nitrate as a catalyst, with or without the addition of a base. Preferably, the base is an organic base.

Preferably, the organic base is N, N-diisopropylethylamine DIEA or triethylamine TEA.

The present invention also provides a compound having the following structure 1-1:

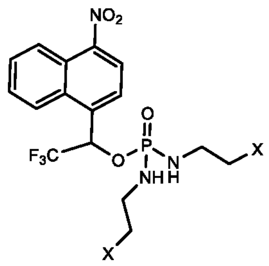 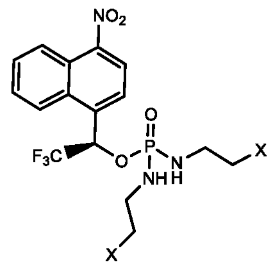 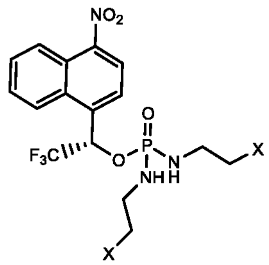 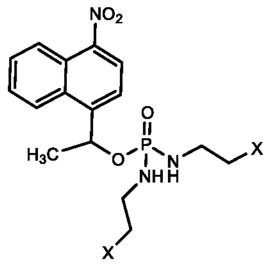

I-1

wherein X is independently F, Cl, Br, or I.

The present invention also provides use of compound I-1 as an intermediate for the synthesis of the hypoxia-activated DNA alkylating agents.

The method for synthesis of a compound of the following structure I-1 comprises reacting a compound I-2 as the starting reactant with phosphorus oxychloride $POCl_3$ respectively to obtain an intermediate, and then reacting the intermediate with haloethanamine or the hydrohalide salt of haloethanamine, and finally obtaining the corresponding compound I-1:

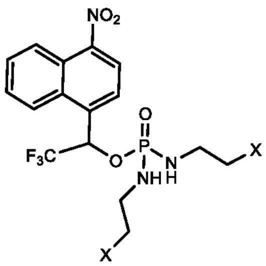 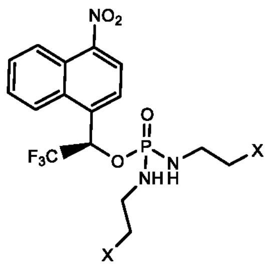 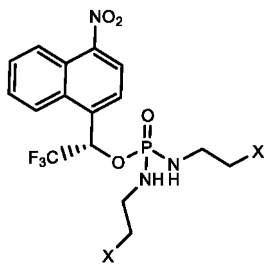 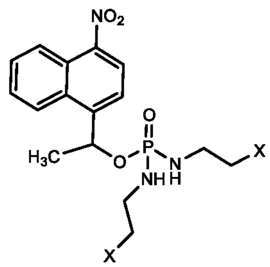

I-1

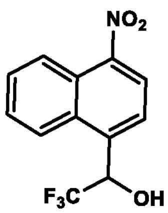 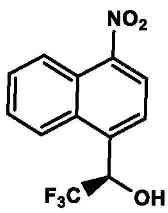 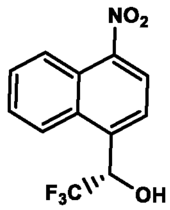 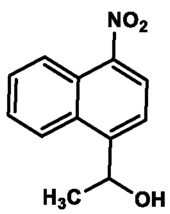

I-2

wherein X is independently F, Cl, Br, or I.

Preferably, the haloethylamine is bromoethylamine and the hydrohalide of haloethylamine is the hydrobromide of bromoethylamine.

The method for preparing a compound of the following structure I-2:

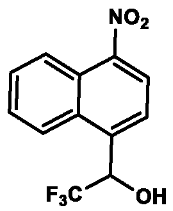 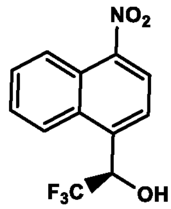 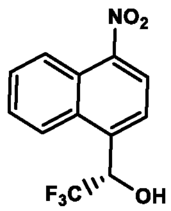 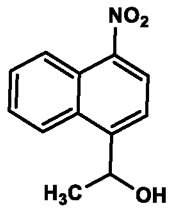

I-2

Compound I-3 was reacted in contact with $TMSCF_3$ and hydrolyzed after deprotection operation to obtain the racemic alcohols I-2-1 and I-2-4. Chiral resolution operation was performed on the racemic alcohol I-2-1 to obtain chiral alcohol I-2-2 and chiral alcohol I-2-3, respectively,

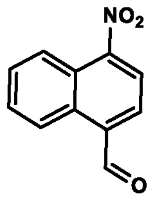

I-3

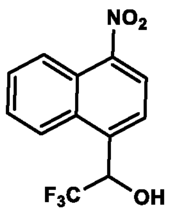
I-2-1

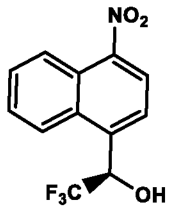
I-2-2

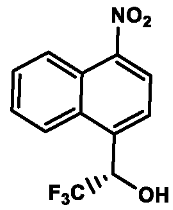
I-2-3

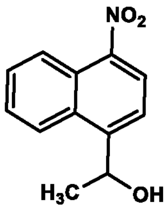
I-2-4

.

Preferably, the deprotection reagent used in the deprotection operation is tetrabutylammonium fluoride TBAF, and ammonium fluoride, aqueous ammonium chloride or hydrochloric acid was used for the hydrolysis operation.

Preferably, the methods of chiral resolution include crystallization method and chemical resolution method.

The present invention also provides a method for separating the racemate of compounds

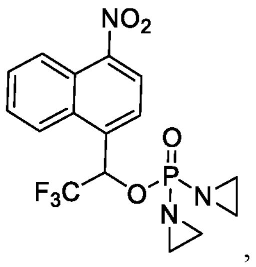 , 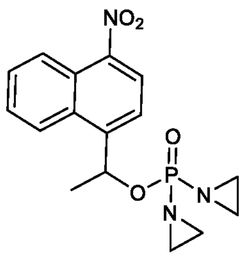

to obtain one of the enantiomers or to increase the concentration of any enantiomeric excess of the compound in the mixture, comprising the step of subjecting the racemic compound to an optical resolution processing by chiral chromatography comprising stationary phase and mobile phase, wherein the stationary phase comprises silica gel impregnated with a functionalized polysaccharide and the mobile phase comprises an alcohol and a further solvent.

Preferably, the alcohol is ethanol and the further solvent is n-hexane.

Among others, the temperature of the chromatography column during the operation of the chiral chromatography is 38°C.

Among others, the functionalized polysaccharide is linear starch-tris(3, 5-dimethylphenylcarbamate), i.e. the stationary phase is silica gel particles coated with linear starch-tris(3, 5-dimethylphenylcarbamate).

The method for preparing a deuterated compound of the following structure I-4 comprises subjecting compound I-5 to a ring closure reaction to give the corresponding compound I-4

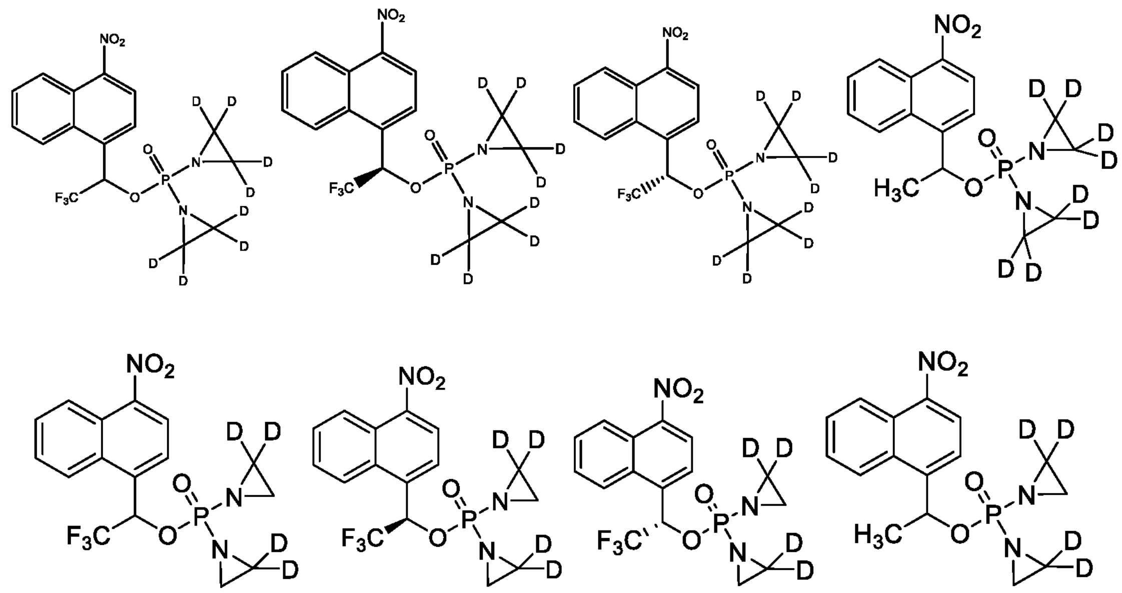

I-4

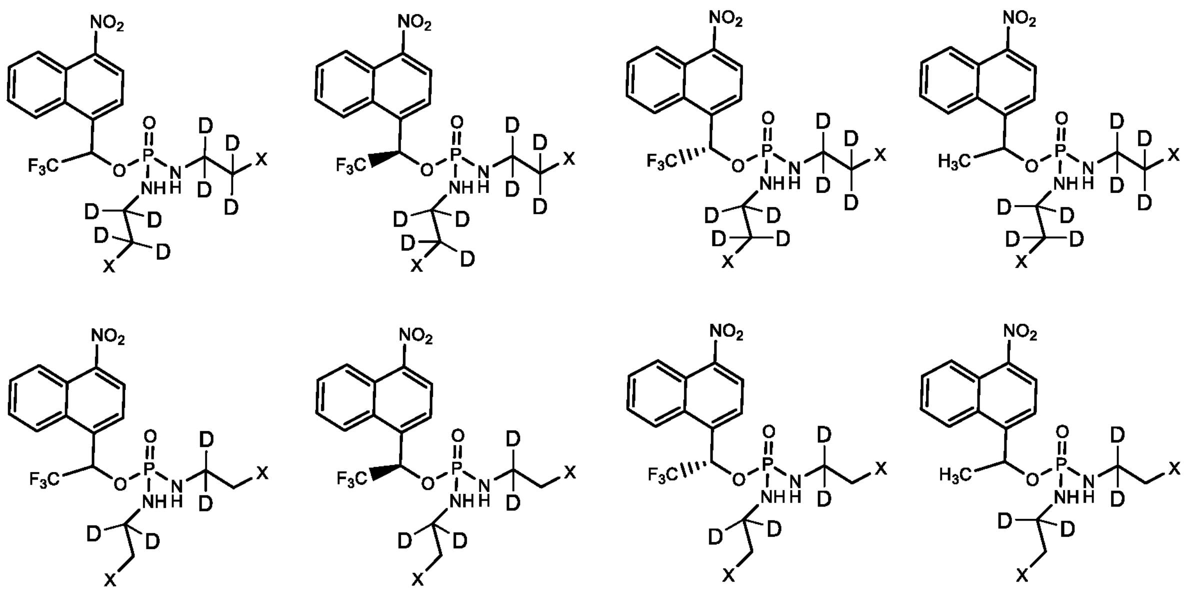

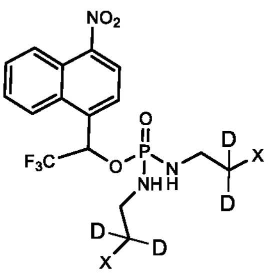
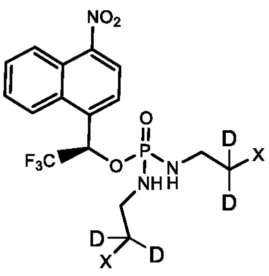
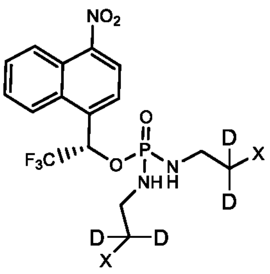
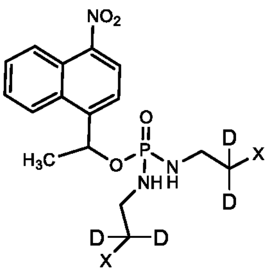

I-5

wherein X is independently F, Cl, Br, or I, preferably Br;

the ring closure reaction uses silver oxide or silver nitrate as a catalyst, with or without the addition of an organic base.

The method for the preparation of a deuterated compound of the following structure I-5 comprises reacting a compound I-2 as the starting reactant with phosphorus oxychloride $POCl_3$ respectively to obtain an intermediate, and then reacting the intermediate with the corresponding deuterated haloethanamine I-6 or the hydrohalide salt of deuterated haloethanamine, and finally obtaining the corresponding compound I-5:

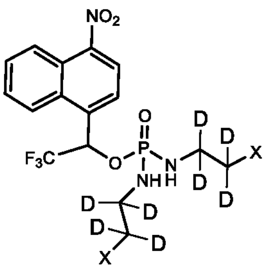
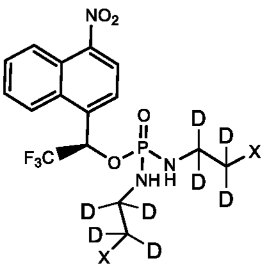
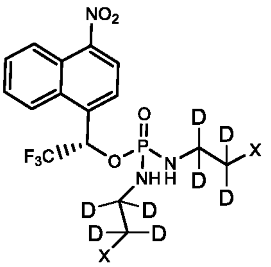
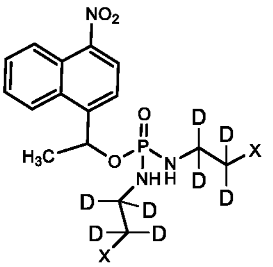
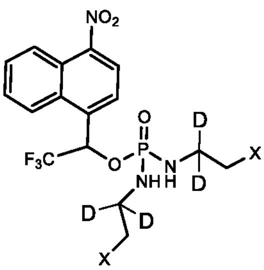
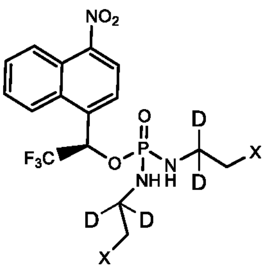
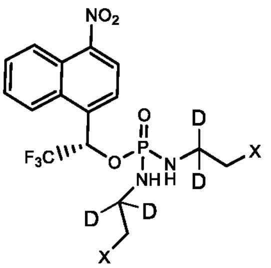
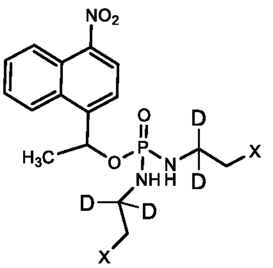
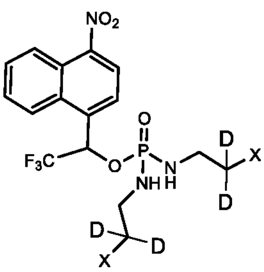
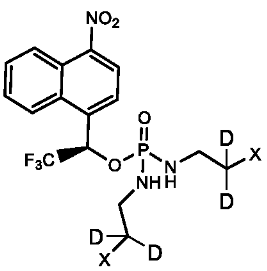
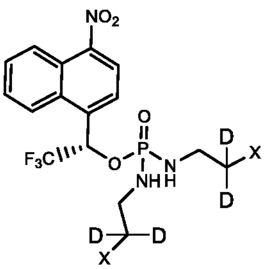
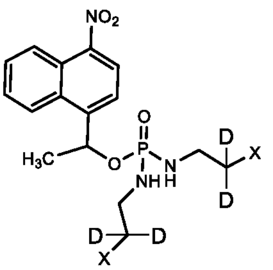

I-5

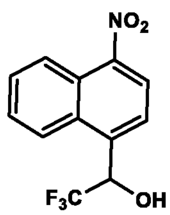 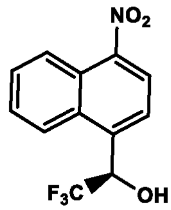 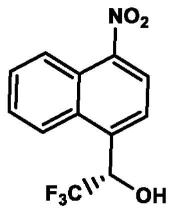 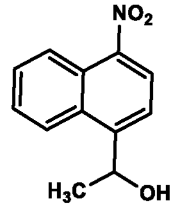

I-2

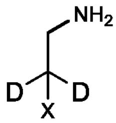 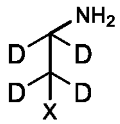

I-6

wherein X is independently F, Cl, Br, or I;

the halogen in the hydrohalide salt of the deuterated haloethylamine is the same as the halogen in the deuterated haloethylamine;

preferably, the haloethylamine is bromoethylamine and the hydrohalide salt of the haloethylamine is the hydrobromide salt of the bromoethylamine.

Regarding the drug or preparation described herein, the prepared drug contains a specific dosage range of the shown compounds or salts or solvates thereof, and/or the prepared drug is in a specific dosage form and is administered using a specific mode of administration.

Regarding the use described herein, the prepared drug may also contain pharmaceutically acceptable auxiliaries or excipients. The drug can be any dosage form for clinical administration, such as tablets, suppositories, dispersible tablets, enteric-coated tablets, chewable tablets, orally disintegrating tablets, capsules, sugar coated agents, granules, dry powders, oral solutions, a small needle for injection, lyophilized powder for injection, or infusion solutions. According to the specific dosage form and the mode of administration, the pharmaceutically acceptable auxiliaries or excipients in the drug may include one or more of the following: diluent, solubilizer, disintegrant, suspension, lubricant, adhesive, filler, flavoring agent, sweetener, antioxidant, surfactant, preservative, wrapping agent and pigment.

Preferably, the patient is a mammal, more preferably a human.

## Brief description of the drawings

Figure 1 shows the chiral HPLC spectrum of compound A.

Figure 2 shows the chiral HPLC spectrum of the optical isomer corresponding to peak 1 obtained after chiral separation of compound A.

Figure 3 shows the chiral HPLC spectrum of the optical isomer corresponding to peak 2 obtained after chiral separation of compound A.

Figure 4 shows a schematic diagram of the absolute stereo configuration of the optical isomer corresponding to peak 1 obtained after the chiral separation of compound A.

Figure 5 shows the inhibition rate curves of three compounds A, B and C at different concentrations on H460 cells under the normoxic condition, wherein inhibition is the inhibition rate, and Log.con (nM) represents the logarithmic value with base 10 of the concentration value in nmol/L.

Figure 6 shows the inhibition rate curves of TH-302 and compound A (left figure), compound D (middle figure), and

TH-302 (right figure) at different concentrations on H460 cells under the normoxic condition air and hypoxic condition $N_2$, wherein inhibition is the inhibition rate, and Log.con (μM) represents the logarithmic value with base 10 of the concentration value in nmol/L. The curve on the left corresponds to the data under anoxic conditions, and the curve on the right corresponds to the data under normoxic conditions.

Figure 7 shows the inhibition rate curves of TH-302 and compound A (left figure), compound D (middle figure), and TH-302 (right figure) at different concentrations on HepG2 cells under the normoxic condition air and hypoxic condition $N_2$, wherein inhibition is the inhibition rate, and Log.con (μM) represents the logarithmic value with base 10 of the concentration value in nmol/L. The curve on the left corresponds to the data under anoxic conditions, and the curve on the right corresponds to the data under normoxic conditions.

Figure 8 shows the inhibition rate curves of compound A at different concentrations on AA8 and UV41 cells under normoxic condition air, wherein inhibition is the inhibition rate, and Log.con (μM) represents the logarithmic value with base 10 of the concentration value in nmol/L. The curve on the left corresponds to the data under anoxic conditions, and the curve on the right corresponds to the data under normoxic conditions.

Figure 9 shows the average remaining percentage of TH-302 and compound A relative to the start at different times after enzymatic reduction of TH-302 and compound A (compound 1 in the figure) by adding 53.2 μg/ml human NADPH coenzyme at 37°C under hypoxia (< 0.1% $O_2$) and air conditions.

Figure 10 shows the average remaining percentage of TH-302 and compound A relative to the start at different times after enzymatic reduction of TH-302 and compound A (compound 1 in the figure) by adding 104.6 μg/ml human NADPH coenzyme at 37°C under hypoxia (< 0.1% $O_2$) and air conditions.

Figure 11 shows the tumor volume change curves at different days under different dosage regimens of compound A in the PDX gastric cancer mouse model test, wherein the abscissa represents the number of days and the ordinate represents the number of volume in $mm^3$.

Figure 12 shows the change curves of body weight of mice relative to the start at different days under different dosage regimens of compound A in the PDX gastric cancer mouse model test, wherein the abscissa represents the number of days and the ordinate represents the percentage change in body weight.

Figure 13 shows the tumor volume change curves at different days under different dosage regimens of compound A in the CDX mouse model test with transplantation of H460 cell line, wherein the abscissa represents the number of days and the ordinate represents the number of volume in $mm^3$.

Figure 14 shows the change curves of body weight of mice relative to the start at different days under different dosage regimens of compound A in the CDX mouse model test with transplantation of H460 cell line, wherein the abscissa represents the number of days and the ordinate represents the percentage change in body weight.

Figure 15 shows the tumor volume change curves at different days under different dosage regimens of compound B and compound C in the CDX mouse model test with transplantation of H460 cell line, wherein the abscissa represents the number of days and the ordinate represents the number of volume in $mm^3$.

Figure 16 shows the change curves of body weight of mice relative to the start at different days under different dosage regimens of compound B and compound C in the CDX mouse model test with transplantation of H460 cell line, wherein the abscissa represents the number of days and the ordinate represents the percentage change in body weight.

Figure 17 shows the tumor volume change curves of the single drugs and combined drugs of compound C and Sorafenib at different days in the *in vivo* drug efficacy test in the human liver cancer HepG2 subcutaneous xenograft model, wherein the abscissa represents the number of days and the ordinate represents the number of volume in $mm^3$.

Figure 18 shows the change curves of body weight of mice relative to the start of the single drugs and combined drugs of compound C and Sorafenib at different days in the *in vivo* drug efficacy test in the human liver cancer HepG2 subcutaneous xenograft model, wherein the abscissa represents the number of days and the ordinate represents the percentage change in body weight.

Figure 19 shows the survival rate curves of the single drugs and combined drugs of compound C and Anti-mouse-PD1 antibody at different days in the drug efficacy test in the murine intestinal cancer CT26 subcutaneous model.

Figure 20 shows the change curves of body weight of mice relative to the start of the single drugs and combined drugs of compound C and Anti-mouse-PD1 antibody at different days in the *in vivo* drug efficacy test in the murine intestinal cancer CT26 subcutaneous model, wherein the abscissa represents the number of days and the ordinate represents the percentage change in body weight.

Figure 21 shows the tumor volume change curves of the single drugs and combined drugs of compound C and Gemcitabine at different days in the *in vivo* drug efficacy test in HuPrime® pancreatic cancer PA3546 human pancreatic cancer subcutaneous xenograft PDX tumor model, wherein the abscissa represents the number of days and the ordinate represents the number of volume in $mm^3$.

Figure 22 shows the change curves of body weight of mice relative to the start of the single drugs and combined drugs of compound C and Gemcitabine at different days in the *in vivo* drug efficacy test in HuPrime® pancreatic cancer PA3546 human pancreatic cancer subcutaneous xenograft PDX tumor model, wherein the abscissa represents the number of days and the ordinate represents the percentage change in body weight.

Figure 23 shows the fluorescent intensity change curves of tumors at different days in the *in vivo* drug efficacy test of compound C alone in the NCI-H460-Luc2 intracranial inoculation model, wherein the abscissa represents the number of days and the ordinate represents the volume fluorescence intensity (intensity size indicates tumor size).

Figure 24 shows the change curves of body weight of mice relative to the start at different days in the *in vivo* drug efficacy test of compound C alone in the NCI-H460-Luc2 intracranial inoculation model, wherein the abscissa represents the number of days and the ordinate represents the percentage change in body weight.

Figure 25 shows the curves of concentration change of compound B and compound C versus time in blood and brain tissues.

Figure 26 shows the corresponding test result curves of ratio of compound Band C concentrations versus time in brain tissues and blood.

Figure 27 shows the change curves of compound A after reaction with liver microsomes from different animals in the presence of NADPH.

Figure 28 shows the curves of content change of compound A versus time in plasma of different animals.

## Detailed Description of the Invention

The present invention will be described below with reference to specific examples. Those skilled in the art could understand that these examples are only used for describing the invention and do not in any way limit its scope.

The experimental methods in the following examples are all conventional methods unless otherwise specified. The raw materials of the medicaments, the reagents and the like used in the following examples are all commercially available products unless otherwise specified.

"Patient" and "subject" are used interchangeably to refer to a mammal in need of treatment for cancer. Generally, the patient is a human. Generally, the patient is a human diagnosed with cancer. In certain embodiments, a "patient" or "subject" may refer to a non-human mammal used in screening, characterizing, and evaluating drugs and therapies, such as, a non-human primate, a dog, cat, rabbit, pig, mouse or a rat.

"Prodrug" refers to a compound that, after administration, is metabolized or otherwise converted to a biologically active or more active compound (or drug) with respect to at least one property.

A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor (for example, see the reference Nogrady, 1985, Medicinal Chemistry A Biochemical Approach, Oxford University Press, New York, pages 388-392, incorporated herein by reference). A prodrug may be synthesized using reactants other than the corresponding drug.

"Solid tumor" refers to solid tumors including, but not limited to, primary or metastatic tumors in bone, brain, liver, lungs, lymph node, pancreas, prostate, skin and soft tissue (sarcoma).

"Therapeutically effective amount" of a drug refers to an amount of a drug that, when administered to the patient with cancer, will have the intended therapeutic effect, e.g., alleviation, amelioration, palliation or elimination of one or more manifestations of cancer in the patient. A therapeutic effect does not necessarily occur by administration of one dose, and may occur only after administration of a series of doses. Thus, a therapeutically effective amount may be administered in one or more administrations.

"Treatment" of a condition or patient refers to taking steps to obtain beneficial or desired results, including clinical results. For purposes of this invention, beneficial or desired clinical results include, but are not limited to, alleviation or improvement of one or more symptoms of cancer; diminishment of extent of disease; delay or slowing of disease progression; alleviation, palliation, or stabilization of the disease state; or other beneficial results. Treatment of cancer may, in some cases, result in partial response or stable disease.

"Tumor cells" refers to tumor cells of any appropriate species, e.g., mammalian such as murine, canine, feline, equine or human.

The above description of embodiments of the present invention does not limit the present invention. Those skilled in the art can make various modifications and changes according to the present invention, and any modification and change within the spirit of the present invention shall be covered in the scope of the claims appended to the present invention.

**I. Synthesis, preparation and structure determination of compounds**

English Abbreviation Description

THF, tetrahydrofuran; DCM, dichloromethane; EA, ethyl acetate; TEA, triethylamine; HPLC, high performance liquid chromatograph; MTBE, methyl tert-butyl ether; DMAP, 4-dimethylaminopyridine; DBAD, di-tert-butyl azodicarboxylate; TFA, trifluoroacetic acid; MS, mass spectrometry; EtOH, ethanol; t-BuOH, tert-butanol; DMF, N, N-dimethylformamide; PE, petroleum ether; DMF-DMA, N, N-dimethylformamide dimethyl acetal; TBAF, tetrabutylammonium fluoride; DIPEA or DIEA, N, N-diisopropylethylamine; DIAD, diisopropyl azodiformate; t-BuOK, potassium tert-butoxide.

Calculated, theoretical calculating value of mass spectrometry; found, measured value of mass spectrometry; eq, equivalent, i.e. molar ratio.

Other abbreviations or terms not specified are to be interpreted or operated according to the definitions or instructions in the Handbook for Organic Chemistry or Organic Synthesis.

All unspecified reagents or drugs are purchased commercially.

For $^1$H-NMR NMR test, if not specified, 400MHz instrument is used for determination; for MS mass spectrometry test, LC-MS liquid chromatography-mass spectrometry instrument is used for determination.

1.1 Synthesis, preparation, and Structure Determination of

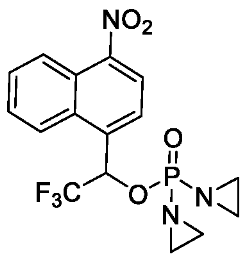
(A),

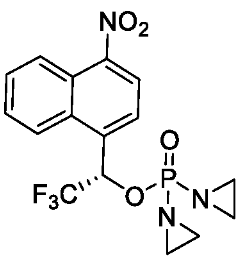
(B),

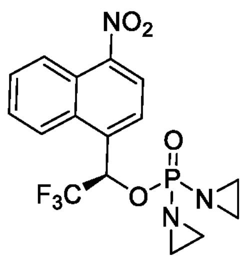
(C)

1.1.1 Synthesis and preparation

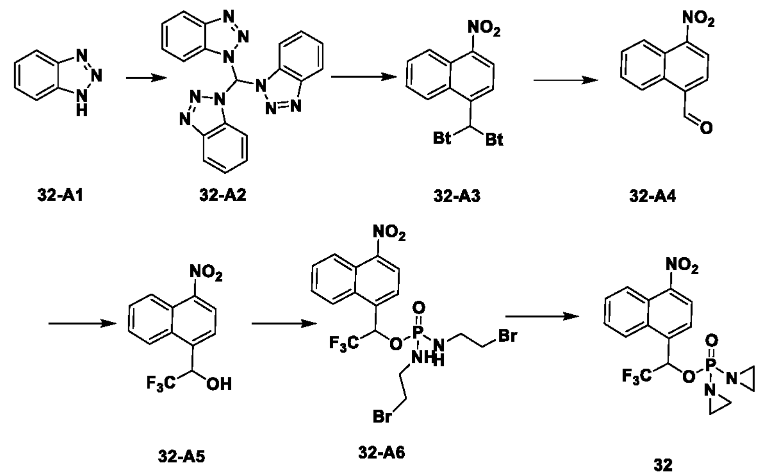

32-A1 32-A2 32-A3 32-A4

32-A5 32-A6 32

Synthetic Route of Compound A

Under nitrogen protection, 32-A1 (20.0g, 167.9mmol), $CHCl_3$ (116.1g, 335.9mmol), tetrabutylammonium bromide (1.6g, 1.68mmol), and 40% NaOH (50mL) were added to a 500mL four-necked flask. The mixture was heated to 62 °C until the reaction was complete. Post-treatment: after cooling to room temperature, the mixture was suction filtered over celite, and the mother liquor was extracted with $CHCl_3$. After drying and concentration, the product 32-A2 (7.0g, 34.0% yield) was preliminarily isolated as an off-white solid. $^1$H-NMR(400MHz, DMSO): $\delta$11.29(s, 1H), 8.22(d, $J$=8.4Hz, 3H), 7.63-7.61(m, 3H), 7.55-7.51(m, 6H).

32-A2 (6.0g, 16.3mmol), 1-nitronaphthalene (2. 8g, 16.3mmol), KOH (5. 8g, 102. 9mmol) were added to DMSO (80mL) and the reaction was completed after overnight at room temperature.

Post-treatment: The system was poured into hydrochloric acid, and a large amount of solid was precipitated. The organic phase was dissolved and extracted with chloroform, dried and concentrated to give 32-A3 (7. 3g), which was used in the next step without further purification.

Under nitrogen protection, 32-A3 (7g crude, 16.33mmol) and $ZnBr_2$ (7.4g, 32.66mmol) were added to dioxane. The mixture was stirred for a period of time after the temperature was increased. Then, concentrated hydrochloric acid was added to the system dropwise. The heating and stirring is continued until the reaction was completed. Post-treatment: after cooling to room temperature, the system was concentrated, dissolved by adding water, extracted with DCM, washed with basic solution, dried and concentrated, and isolated by chromatography to give 32-A4 (1.3g) as a yellow solid. $^1$H-NMR (400MHz, $CDCl_3$): $\delta$10.53 (s, 1H), 9.30 (dd, $J_1$=7.2Hz, 2.0Hz, 1H), 8.37 (dd, $J_1$=8.0Hz, 1.8Hz, 1H), 8.17 (d, $J$=7.6Hz, 1H), 8.09 (d, $J$=7.6Hz, 1H), 7.86-7.80 (m, 2H).

Under nitrogen protection, 32-A4 (200mg, 0. 99mmol) and $TMSCF_3$ (283mg, 1.98mmol) were added to THF. The system was cooled to 0°C. TBAF (0.1mL) was added dropwise to the system, and the reaction was continued until the conversion of the starting material was complete. Appropriate amount of hydrochloric acid was added dropwise to the system, and the mixture was extracted with DCM. The organic phase was washed with water and brine, dried and concentrated, and isolated by chromatography to give the product 32-A5 (190mg, 70.5%) as a yellow solid. $^1$H-NMR(400MHz, $CDCl_3$): $\delta$8.49(d, $J$=8.0Hz, 1H), 8.15(d, $J$=8.0Hz, 1H), 7.98(d, $J$=7.2Hz, 1H), 7.40-7.32(m, 2H), 5.98(m, 1H), 2.98(s, 1H).

Under nitrogen protection, $POCl_3$ (210mg, 1.36mmol) was added to DCM. The system was cooled to -30°C. 32-A5 (185mg, 0.68mmol) was dissolved in DCM and then added dropwise to the system. TEA (173mg, 1. 71mmol) was dissolved in DCM and added dropwise to the system. The mixture was incubated at -30°C until the starting material disappeared. Bromoethylamine hydrobromide (1.1g, 5.46mmol) and TEA (552mg, 5. 46mmol) were added to the system, and the reaction was completed after a period of time. Saturated aqueous ammonium chloride solution was added at 0°C, and the mixture was extracted with DCM. The organic phase was washed with water and brine, dried and concentrated, and isolated by chromatography to give the product 32-A6 (170mg, 44. 3%) as a yellow solid. $^1$H-NMR(400MHz, $CDCl_3$): $\delta$8.49 (d, $J$=1.2Hz, 1H), 8.26(d, $J$=7.6Hz, 1H), 8.17(d, $J$=8.0Hz, 1H), 7.90(d, $J$=8.0Hz, 1H), 7.80-7.75(m, 2H), 6.63-6.61(m, 1H), 3.56-2.94(m, 8H).MS: Calculated 562.9, found 563.9([M+H]$^+$). MS: Calculated 562.9, found 563.9([M+H]$^+$).

Under nitrogen protection, 32-A6 (160mg, 0.28mmol) was dissolved in THF (15mL). Then, silver oxide (329mg, 1.42mmol) and DIEA (167mg, 1.42mmol) were added to the system. The system was heated to 65°C and then stirred until the reaction was completed. The mixture was suction filtered over celite. The solid was washed with DCM, and the mother liquor was concentrated. Upon high performance liquid phase preparation, compound 32 (i.e. compound A) in pure form as a pale yellow solid was obtained. $^1$H-NMR (400MHz, $CDCl_3$): δ8.57-8.44(m, 1H), 8.31-8.10(m, 2H), 7.96(d, *J*=7.9Hz, 1H), 7.84-7.67(m, 2H), 6.65(s, 1H), 2.33-2.21(m, 2H), 2.20-2.09(m, 2H), 2.06-1.96(m, 4H). MS: Calculated 401.1, found 402.0($[M+H]^+$).

1.1.2 Chiral Resolution of Compound

The above compound 32 (i.e. compound A) in pure form was subjected to chiral analysis HPLC chiral analysis conditions are:

| | |
|---|---|
| Analytical chromatographic column | CHIRALPAK AD-3(AD30CD-SK010) chromatographic column |
| Column size | internal diameter 0.46 cm, length 15 cm |
| Injection volumn | 0.5ul |
| Mobile phase | n-hexane: ethanol = 60/40 (volume ratio) |
| Flow rate | 1.0 ml/min |
| Detection Wave length | UV 210nm |
| Column temperature | 35 °C |

The HPLC spectrum obtained upon test is shown in Figure 1, and the specific results are shown in the following table.

| Separation Peak | Ret. Time(Retention time) | Area | Area% |
|---|---|---|---|
| peak 1 | 2.908 | 5110974 | 47.096 |
| peak 2 | 3.766 | 5741368 | 52.904 |

4.9971 g of compound A was taken and dissolved in chromatographic grade ethanol. Chiral separation preparation was carried out using the following chiral separation preparation method and chiral separation preparation conditions.

HPLC chiral separation preparation method: The above chiral isomers (i.e., compound A) were separated by HPLC method using HPLC preparation equipment and a chiral column, and the corresponding fractions were collected. The solvent was removed by rotary evaporation to obtain the pure product of the optical isomer.

The HPLC chiral separation preparation conditions were:

| | |
|---|---|
| Resolution chromatographic column | CHIRALPAK AD chromatographic column |
| Column size | internal diameter 5.0 cm, length 25 cm, filler particle size 10μm |
| Mobile phase | n-hexane: ethanol = 60/40 (volume ratio) |
| Flow rate | 60 ml/min |
| Detection Wave length | UV 254 nm |
| Column temperature | 38 °C |

After the separation preparation, 2.5377 g of pure product of the optical isomer corresponding to peak 1 was obtained, and the measured ee value was 99.7%; 2.4653 g of pure product of the optical isomer corresponding to peak 2 was obtained, and the measured ee value was 99.4%.

The optical isomer corresponding to peak 1 and the optical isomer corresponding to peak 2 from the chiral separation preparation were further analyzed using HPLC chiral analysis conditions, and the results were obtained as shown in Figures 2 and 3.

The results of the HPLC analysis of the optical isomer corresponding to peak 1 are shown in the following table:

| Separation Peak | Ret. Time(Retention time) | Area | Area% |
|---|---|---|---|
| peak 1 | 2.908 | 7125381 | 99.837 |
| peak 2 | 3.758 | 11633 | 0.163 |

The results of the HPLC analysis of the optical isomer corresponding to peak 2 are shown in the following table:

| Separation Peak | Ret. Time(Retention time) | Area | Area% |
|---|---|---|---|
| peak 1 | 2.897 | 20323 | 0.320 |
| peak 2 | 3.765 | 6340121 | 99.680 |

1.1.3 Determination of the absolute configuration of the compounds

The absolute configuration of compound 1 was analyzed by single crystal X-ray diffraction results.

Single crystal cultivation. A sample of about 5 mg of the optical isomer corresponding to the above peak 1 was weighed, dissolved in methanol (2 mL, analytical purity), sonicated at room temperature for 1 min, filtered through an organic filter membrane, and left to stand at room temperature for two days to obtain colorless crystals.

Single crystal structure analysis.

The above colorless crystals were single-crystal diffracted using X-ray single-crystal diffractometer (D8 Venture, Bruker) with the following parameters:

| | |
|---|---|
| Light source: Mo target | X-ray: Mo-K□ (=0.71073 Å) |
| Detector: CMOS area detector | Resolution: 0.80 Å |
| Current and Voltage: 50 kV, 1.2 A | Exposure time: 30 s |
| Distance from area detector to sample: 50 mm | Test temperature: 298 K (25 °C) |

After collecting the data, the SAINT program was used to perform an integral reduction on the diffraction data and then the SADABS program was used to perform an empirical absorption correction on the data, the SHELXT2014 was used to analyze the single crystal structure by the direct method, and the least squares method was used to refine the structure. The refinement of the hydrogen atoms was obtained by isotropic calculation, and the hydrogen atoms on C-H were obtained by calculation of dehydrogenation and were refined by a riding model. The flack constant of 0.03 (10) can be obtained from the data, and the absolute configuration can be determined. The C5 configuration in the structure is the S-configuration as shown in Figure 4.

Therefore, it can be seen that the optical isomer corresponding to the peak 1 is the S-configuration, and the structural formula is as follows:

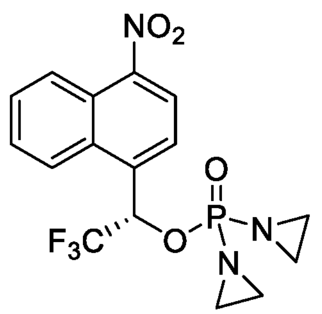

Structural formula of the optical isomer corresponding to peak 1

Therefore, it can be seen that the peak 1 in the spectrogram using chiral HPLC corresponds to the compound

32 (compound A) in the S-configuration and is compound B.

1.2 Synthesis, preparation and structure determination of

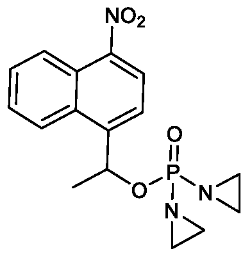

(D),

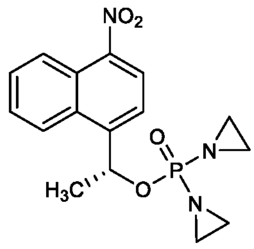

(E),

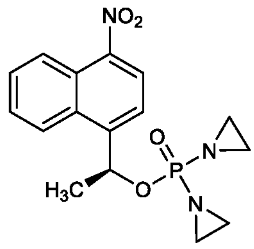

(F)

Preparation of compound 26 (i.e., compound D above)

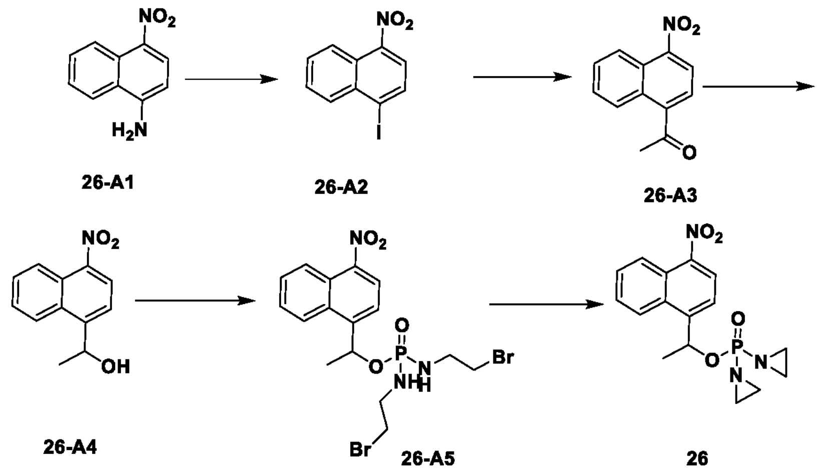

26-A1 26-A2 26-A3 26-A4 26-A5 26

p-Toluenesulfonic acid (20.0g, 112.0mmol) was added to acetonitrile (160mL), followed by 26-A1 (7.4g, 39.32mmol). The system was cooled to 0°C. Aqueous solution (24mL) of KI (16g, 98.30mmol) and $NaNO_2$ (5.4g, 78.64mmol) was added dropwise to the system. The reaction was completed over 2h. $H_2O$ (600mL) was added to dilute, and the pH was adjusted to 9-10 with 1N aqueous $NaHCO_3$ solution. Then, 2M sodium thiosulfate solution (50mL) was added and a large amount of solid was precipitate, suction filtered and dissolved with ethyl acetate, dried and concentrated and isolated by column chromatography (200-300 mesh silica gel, n-heptane: ethyl acetate = 20: 1) to obtain product 26-A2 (11.0g, 93.5% yield) as a yellow solid. $^1$H-NMR(400MHz, $CDCl_3$): $\delta$8.50(d, $J$=8.0Hz, 1H), 8.24(d, $J$=1.2Hz, 1H), 8.20(d, $J$=8.4Hz, 1H), 7.86(d, $J$=8.0Hz, 1H), 7.76-7.70(m, 2H).

26-A2 (3.0g, 10.00mmol) and tributyl(1-ethoxyethylene)tin (5.0g, 14.00mmol) were added to dioxane (40mL) under nitrogen, and tetrakis(triphenylphosphine)palladium (277mg, 0.25mmol) was added to the system after pumping and charging nitrogen three times. The system was heated to 105°C overnight after another pumping and charging nitrogen three times, and after that the reaction was completed. The system was cooled to room temperature and

quenched by adding dropwise 50mL of saturated ammonium chloride, extracted with ethyl acetate (50mL×4), concentrated, added with 100mL of 3N HCl and stirred for 12h, then extracted with DCM (50mL×4), washed with water and brine, dried and concentrated, and isolated by column chromatography (200-300 mesh silica gel, n-heptane: ethyl acetate = 20: 1) to obtain product 26-A3 (1.1g, 51.5% yield) as a yellow solid. $^1$H-NMR (400MHz, $CDCl_3$): $\delta$8.55(d, *J*=6.4Hz, 1H), 8.45(d, *J*=6.4Hz, 1H), 8.10(d, *J*=7.6Hz, 1H), 7.83(d, *J*=7.6Hz, 1H), 7.78-7.69(m, 2H), 2.77(s, 3H). MS: Calculated 215.1, found 216.0([M+H]$^+$).

Under nitrogen protection, 26-A3 (200 mg, 0.93 mmol) was dissolved in THF (5 mL). The system was cooled to 0°C and $BH_3$-THF (4.7 mL, 4.65 mmol) was added dropwise. The reaction was completed over 30 min. The system was cooled down to 0 °C. The reaction was quenched by adding dropwise MeOH (6 mL). The system was concentrated and then dissolved with DCM, washed 3 times with 1N HCl, dried and concentrated to give product 26-A4 (185 mg, 91.6% yield) as a yellow oil. $^1$H-NMR (400MHz, $CDCl_3$): $\delta$8.55(d, *J*=6.4Hz, 1H), 8.20-8.11(m, 2H), 7.79(d, *J*=6.4Hz, 1H), 7.73-7.62(m, 2H), 5.75-5.70(m, 1H), 1.66(d, *J*=6.4Hz, 3H). MS: Calculated 217.1, found 218.0([M+H]$^+$).

Under nitrogen protection, 26-A4 (650mg, 2.99mmol), Br-IPM (1.4g, 4.49mmol) and $PPh_3$ (1.8g, 5.99mmol, commercially purchased) were added to THF (50mL). The system was cooled to 0 °C and DIAD (1.2g, 5.99mmol, commercially purchased) was added dropwise. The reaction was kept at 30°C for 30 minutes and then completed. 50 mL of water was added dropwise to the system. The system was extracted with DCM (30 mL×3). The organic phase was washed with water and brine, dried, concentrated and then isolated by column chromatography (200-300 mesh silica gel, n-heptane: ethyl acetate = 1: 1) to obtain product 26-A5 (400 mg, 26.3% yield) as a yellow oil. $^1$H-NMR(400MHz, $CDCl_3$): $\delta$8.52(d, *J*=6.4Hz, 1H), 8.26-8.15(m, 2H), 7.75-7.68(m, 2H), 7.54-7.52(m, 1H), 6.35-6.28(m, 1H), 3.49-3.15(m, 8H), 1.79(d, *J*=6.4Hz, 3H).

Under nitrogen protection, 26-A5 (250mg, 0.49mmol) was dissolved in THF (25mL) and then silver oxide (569mg, 2.46mmol) and DIEA (217mg, 2.46mmol) were added to the system. After the system was heated to 65 °C and then stirred overnight, the reaction was completed. The mixture was suction filtered over celite. The solid was washed with DCM and the mother liquor was concentrated. Compound 26 (30.7mg, 18.1%) i.e.

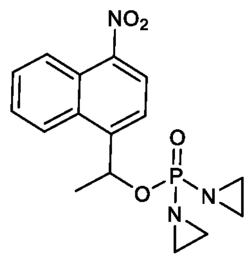

as a yellow oil was obtained by high performance liquid phase preparation. $^1$H-NMR(400MHz, $CDCl_3$): $\delta$8.56-8.54(m, 1H), 8.208.17(m, 2H), 7.81(d, *J*=8.0Hz, 1H), 7.74-7.67(m, 2H), 6.57-6.50(m, 1H), 2.25-1.99(m, 8H), .79(d, *J*=6.4Hz, 3H). MS: Calculated 347.1, found 348.0([M+H]$^+$).

The two chiral isomers

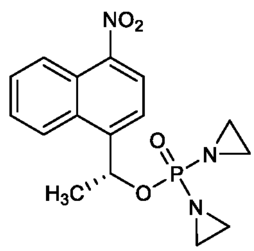

(E) and

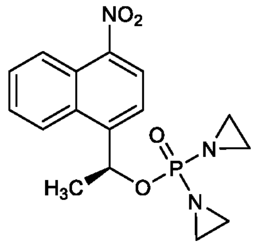

(F) of compound 26 can be obtained by chiral resolution and chiral synthesis and their configurations can be determined in a similar method to that of compound 32 above.

1.3 Synthesis and preparation of deuterated compounds

Deuterated compound I-4 was prepared using the following synthetic routes and steps.

Step 1 comprises synthesizing a deuterated compound of the following structure I-5, which comprises first reacting a compound I-2 as the starting reactant to react with phosphorus oxychloride $POCl_3$ respectively to obtain an intermediate, and then second reacting the intermediate with the corresponding deuterated haloethanamine I-6 or the hydrohalide salt of deuterated haloethanamine, and finally obtaining the corresponding compound I-5:

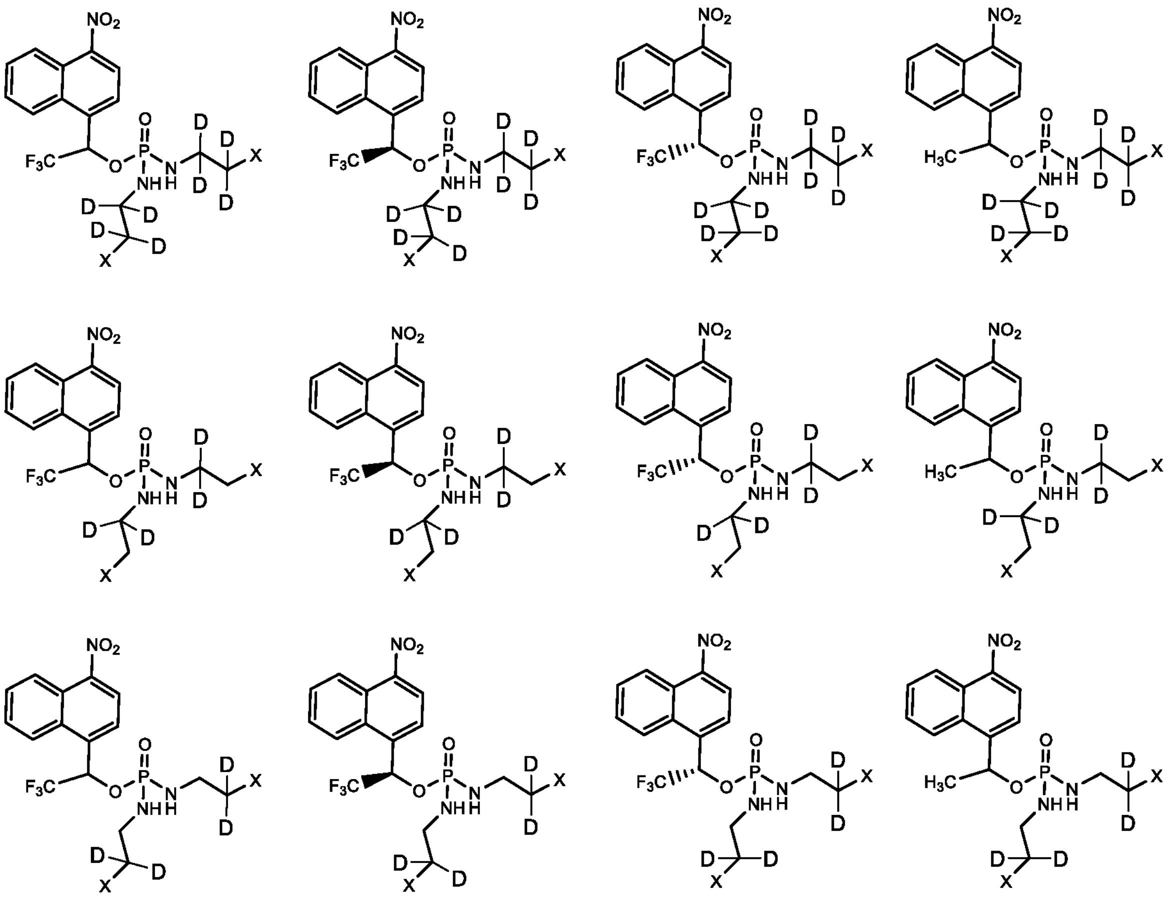

I-5

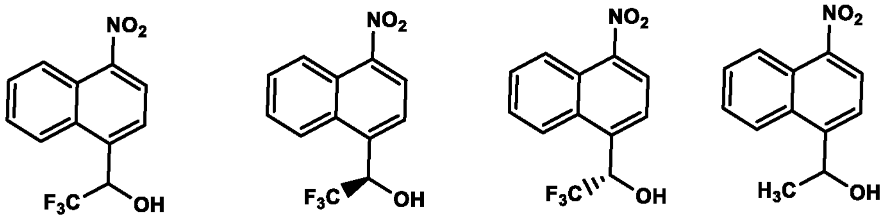

I-2

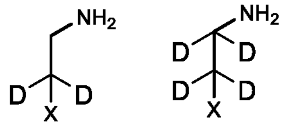

I-6

wherein X is independently F, Cl, Br, I; the halogen in the hydrohalide salt of the deuterated haloethylamine is the same as the halogen in the deuterated haloethylamine;

preferably, the haloethylamine is bromoethylamine and the hydrohalide salt of the haloethylamine is the hydrobromide salt of the bromoethylamine.

Step 2 comprises preparing a deuterated compound of the following structure I-4, which comprises subjecting compound I-5 to a ring closure reaction to obtain the corresponding compound I-4

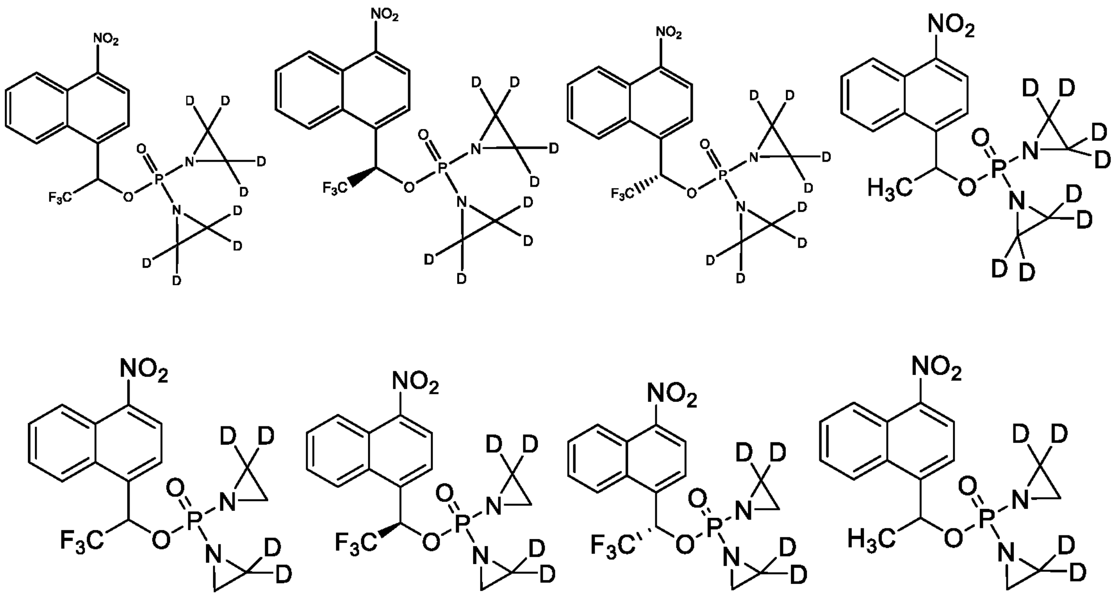

I-4

wherein X is independently F, Cl, Br, I, preferably Br;

the ring closure reaction used silver oxide or silver nitrate as a catalyst, with or without the addition of an organic base.

The specific operations are carried out with reference to the steps for compound 26 and compound 32 as described above.
The following deuterated compounds:

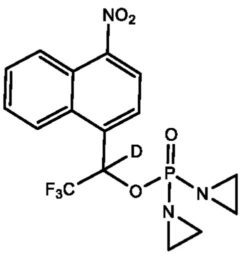 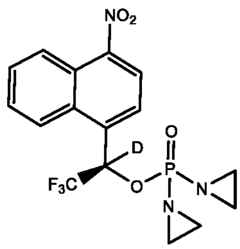, 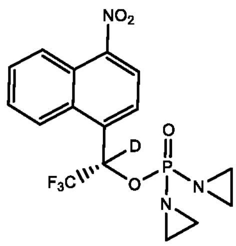, 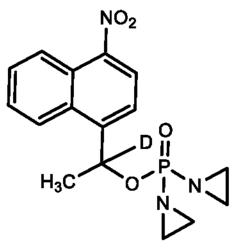,

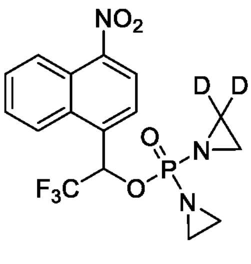, 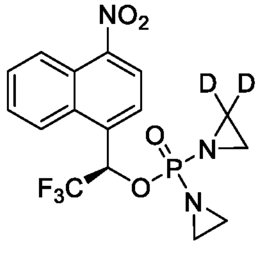, 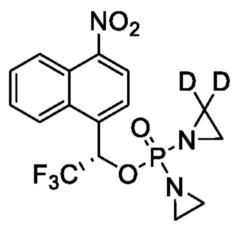, 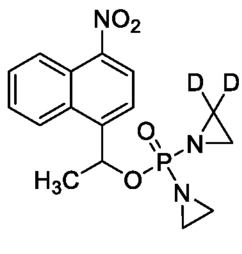,

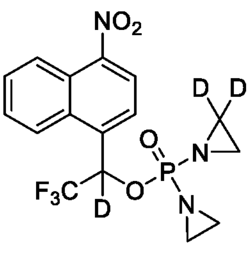, 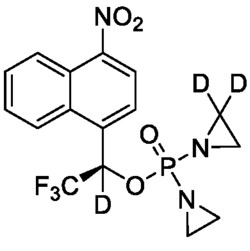, 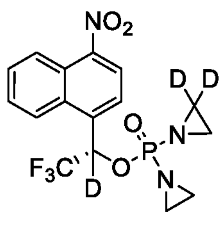, 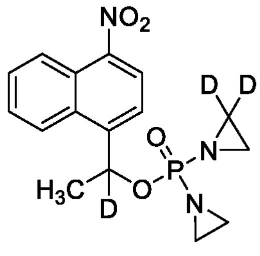,

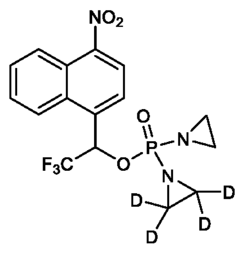, 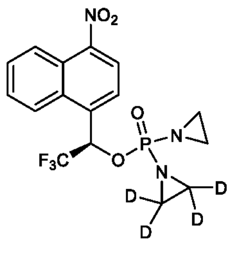, 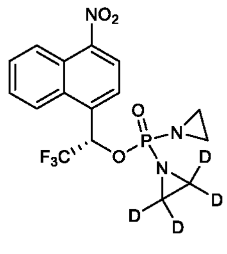, 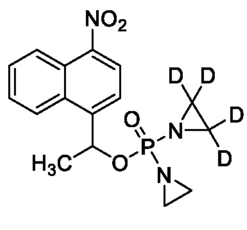,

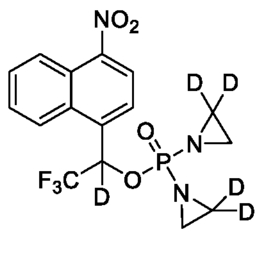, 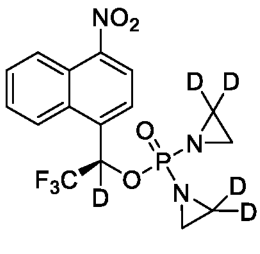, 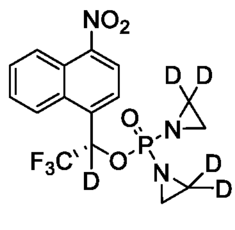, 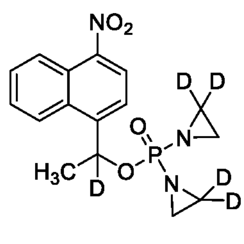

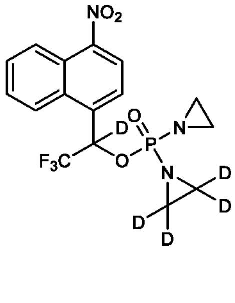, 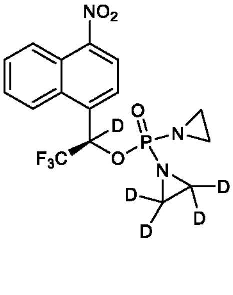, 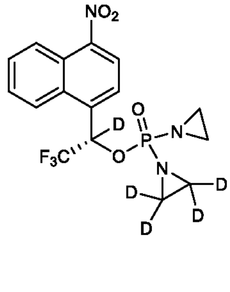, 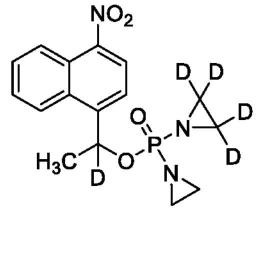

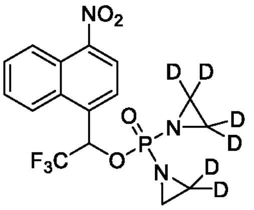 , 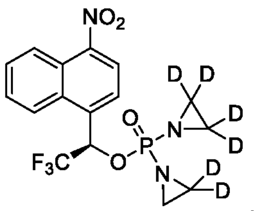 , 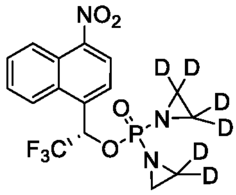 , 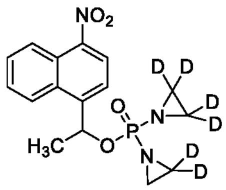 ,

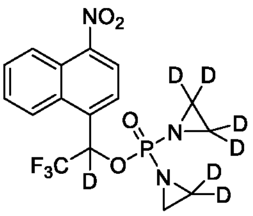 , 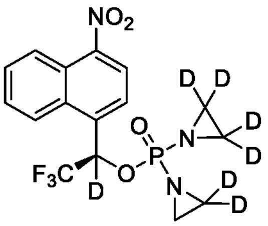 , 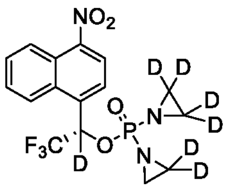 , 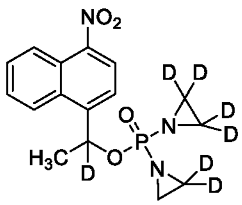 ,

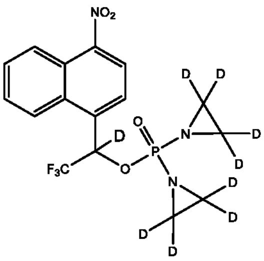 , 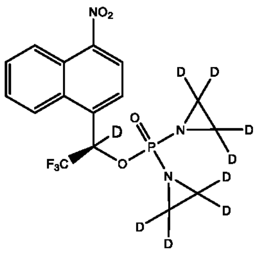 , 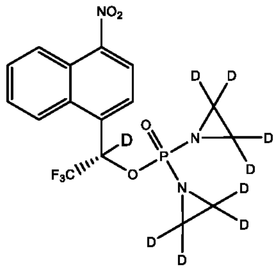 , 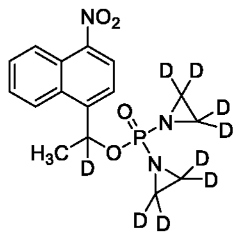 ,

can be obtained by using the following compounds of formula I-2 after deuterium substitution as the starting material for step 1, and performing the appropriate group protection and deprotection procedures

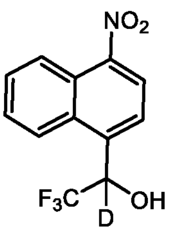 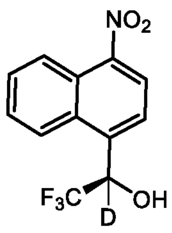 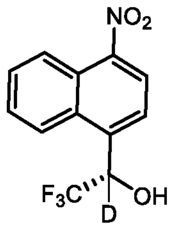 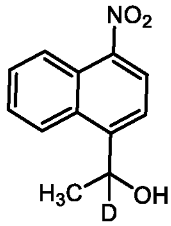 .

The meanings of foreign abbreviations or words involved in the in vivo and in vitro experiments of sections 2 to 8 below are to be interpreted according to the meanings of the words in textbooks of medicinal chemistry, biochemistry, physiology, pharmacology, or medicine.

## II. Inhibition test of Compounds on Cancer Cell

The quantification value $IC_{50}$ for cytotoxicity of a human tumor cell line *in vitro* was used to compare the cancer cell proliferation inhibition of compounds

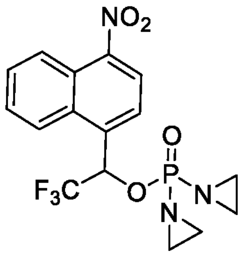,
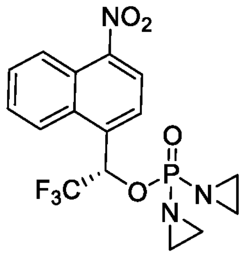,
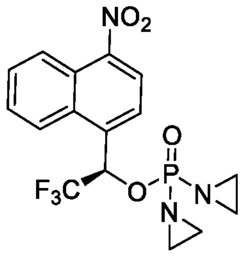

A (compound 32) B C.

If not otherwise specified, the test method is as follows:
Compounds were exposed at various concentrations for 72 hours, followed by a wash step. Fresh medium was added for growth. The cell was stained to determine the cell survival rate and compared with controls treated with medium only.

In particular, exponentially grown cells were inoculated in 96-well plates at a density of $2 \times 10^3$ cells/100 μl/well and incubated for 24 hours at 37°C and under the conditions of 5% $CO_2$, 95% air and 100% relative humidity before addition of 99 μl of culture solution. The compounds were dissolved in 100% DMSO at 200 times of the desired final test concentration. When the drug was added, an aliquot of 1 μl of the compound at the specified concentration was added to microwells already containing 199 μl of medium to obtain the reported final drug concentration. After adding the drug, plates were incubated for an additional 72 hours at 37°C, 5% $CO_2$, 95% air and 100% relative humidity. The cell assay plate was left to balance for 30 minutes at room temperature and 100 μL of medium was removed from per well. 100 μl CTG reagent (CelltiterGlo kit) was added into each well. The plate was placed on a fast shaker and shaked for 2 min, and kept in dark at room temperature for 30 min. The chemiluminescence signal value was read by Envision instrument. The drug concentration resulting in 50% growth inhibition ($IC_{50}$) was calculated using computer software and the results are showed in the table below.

Table 1: $IC_{50}$ data for inhibitory effect of the three compounds on H460 cancer cells

| Compound | $IC_{50}$(nm/L) |
|---|---|
| A | 128 |
| B | 103.9 |
| C | 76.85 |

The resulting $IC_{50}$ curve upon test is shown in Figure 5.

The test results indicated that the two optical isomers (compounds B and C) had H460 inhibitory toxicity similar to the racemate (compound A).

Compound A was further tested for cytotoxicity against different non-small cell lung cancer cells NSCLC and the results were shown in Table 2 below:

Table 2: $IC_{50}$ data for inhibitory effect of compound A on different non-small cell lung cancer cells NSCLC

| Different NSCLC cell lines | $IC_{50}$ (nM) |
|---|---|
| NCI-H1944 | 538.1 |
| NCI-H2228 | 263.9 |
| NCIH1755 | 6465 |
| NCI-H1563 | 4451 |
| CAL12T | 30470 |
| NCIH2106 | 14778 |
| NCI-H23 | 6409 |
| NCI-H522 | 19155 |
| NCI-H2110 | 382 |

(continued)

| Different NSCLC cell lines | $IC_{50}$ (nM) |
|---|---|
| NCI-H1792 | 4785 |

Compound A was further tested for cytotoxicity against different pancreatic cancer PA cells and the results were shown in Table 3 below:

Table 3: $IC_{50}$ data for inhibitory effect of compound A on different pancreatic cancer PA cells

| **PA cell lines** | **$IC_{50}$(nM)** |
|---|---|
| AsPC-1 | 12575 |
| QGP-1 | 6271 |
| HPAF-II | 26058 |
| KP-2 | 29741 |
| CFPAC1 | 2863 |
| KP-3 | 26165 |
| Capan-1 | 5970 |
| Pane 02.03 | 45662 |
| MIA PaCa-2 | 6900 |
| PL45 | 17085 |
| Pane 10.05 | 27735 |
| PANC-1 | 29293 |
| KP-4 | 6099 |

**III. Hypoxia-dependent cytotoxicity test**

3.1 Proliferation inhibition test of different cell lines

To determine the effect of the drug A according to the present invention on cancer cell proliferation, the antiproliferative activity of these compounds against the cancer cell was detected in a porous CTG-based assay. The effects of test compounds on cell proliferation under normoxic (21% $O_2$) and hypoxic (<0.01% $O_2$) conditions were compared. H460 cells were routinely cultured to 80%-90% of cell saturation, and when the number reached the requirement, resuspended with the corresponding culture medium, counted and prepared into cell suspension with appropriate density. The cell suspension was added to two types of 24-well plates, 495 μL per well with $1 \times 10^4$/well of cell density. 24-well plates inserted with glass insert were used for hypoxic tests, and ordinary plastic 24-well plates were used for normoxic tests. 5 μL of each compound was added to the hypoxic and normoxic reaction systems, respectively. After reacting for 3 hours in the hypoxic workstation or the normoxic incubator, the compounds were washed away using the culture medium and the system was placed in an incubator for another 72 hours. The final concentrations of each compound tested under normoxic conditions were as follows: TH-302 was 1000, 200, 40, 8, 1.6, 0.32 μM; test compound A (compound 32) was 1000, 200, 40, 8, 1.6, 0.32 μM; compound 26 was 500, 50, 5, 0.5, 0.05 μM. The final concentrations of each compound tested under hypoxic conditions were as follows: TH-302 was 10, 2, 0.4, 0.08, 0.016, 0.0032 μM; test compound A (compound 32) was 50, 5, 0.5, 0.05, 0.005, 0.0005 μM; compound 26 was 500, 50, 5, 0.5, 0.05 μM. After 72 hours of cell culture, 300 μL of culture medium was removed from each well of 24-well plate. 50 μL of CTG was added into each well. The plate was mixed and shaked for 2 min, and kept in dark for 15 minutes at room temperature. 100 μL per well of culture medium was transferred from 24-well plate to 96-well white plate, and the chemiluminescence signal value was read with Multifunctional enzyme marker with a reading time of 1000ms. The $IC_{50}$ was calculated using GraphPad Prism 5 software. The 50% growth inhibitory concentration ($GI_{50}$, also referred to herein as $IC_{50}$) of the test compounds was calculated and listed in Table 4 below. The compound TH-302 (a hypoxic-activated prodrug entering phase III clinical studies, cas No. 918633-87-1, see the literature Sun J D, Liu Q, Wang J, et al. Selective tumor hypoxia targeting by hypoxia-activated prodrug TH-302 inhibits tumor growth in preclinical models of cancer.[J]. Clinical Cancer

Research An Official Journal of the American Association for Cancer Research, 2012, 18(3): 758-70, structural formula is

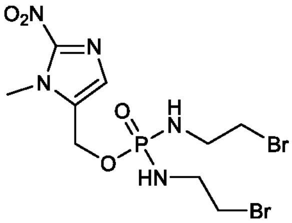

TH-302

) was used as a control.

Table 4: $IC_{50}$ data of inhibitory effect of two compounds on H460 cancer cells under hypoxic and normoxic conditions

| Compound | Normoxic $IC_{50}$ (μM) | $N_2$ $IC_{50}$ (μM) | $IC_{50}$ ratio: Normoxia/ $N_2$ |
|---|---|---|---|
| A(Compound 32) | 0.50 | 0.04 | 12.5 |
| TH-302 | 13.60 | 0.02 | 680 |
| Compound 26 | 0.80 | 0.04 | 20 |

The resulting $IC_{50}$ curve of the test is shown in Figure 6.

Compound A according to the present invention had a stronger inhibitory effect on H460 cancer cells than TH-302 under normoxic conditions, and TH-302 had a stronger inhibitory effect on H460 cancer cells than compound A under hypoxic conditions, with the $IC_{50}$ ratio of hypoxic activation reaching or exceeding 12.5.

Similarly, the above-mentioned three compounds were tested for the proliferation inhibition effect on hepatoma cell HepG2, and the results are shown in the following table 5.

Table 5: $IC_{50}$ data of inhibitory effect of two compounds on HepG2 cancer cells under hypoxic and normoxic conditions

| Compound | $IC_{50}$ Normoxia(μM) | $IC_{50}$ $N_2$ (μM) | HCR |
|---|---|---|---|
| A(compound 32) | 20.59 | 4.78 | 4.31 |
| TH-302 | 918.20 | 12.39 | 72.82 |
| D(compound 26) | 16.69 | 1.50 | 11.13 |
| Note: HCR (hypoxic cytotoxicity ratio) means hypoxic activation ratio, i.e., the $IC_{50}$ ratio under normoxia/ $N_2$ conditions. | | | |

The resulting $IC_{50}$ curve of the test is shown in Figure 7.

Compound A according to the present invention has a stronger inhibitory effect on H460 cancer cells than TH-302 under both normoxic and hypoxic conditions and still has hypoxic activation properties.

To further compare the cytotoxicity of TH-302 with that of compound A, the following further provide test results for H460 cell line and cells from different species at different test times in the same laboratory, with the same operator and under the same experimental conditions.

The comparison data of the test results of the same cell line at different times are shown in Table 6 below:

Table 6: Inhibition Test Data for TH-302 and Compound A on normoxic, hypoxic Cancer Cell

| Different test times in the same laboratory, with the same operator and under the same experimental conditions | Exposure for 2 hours; Air; $IC_{50}$ (μM); H460 cell line | | Multiplier |
|---|---|---|---|
| | TH-302 | Compound A | TH-302/Compound A |
| Time 1 | 23 | 0.7 | 32.9 |
| Time 2 | 8 | 0.3 | 26.7 |
| Time 3 | 3.3 | 0.5 | 6.6 |

The comparison data of the test results of cells from different species are shown in Table 7 below.

Table 7: Cell inhibition test data of different species under normoxic conditions of TH-302 and compound A

| Cell line | Species | $IC_{50}$ (μM) | |
|---|---|---|---|
| | | A (exposure time of 3 days) | TH-302 (exposure time of 2h) |
| H460 | Human | 0.1 | 20 |
| AA8 | Rat | 11 | 830 |
| Multiplier: Rat/Human | | 110 | 41.5 |

Cell inhibition test data at different time points, from different species, from different cancer cell lines in table 6 and table 7 again demonstrate that compound A had a stronger inhibitory effect (under both normoxic and hypoxic conditions) on cancer cells than TH-302.

3.2 Cytotoxicity test on cell line UV41 with DNA repair gene mutation

Further, cytotoxicity tests were performed using cell line UV41 with DNA repair gene mutation.

Particularly, exponentially growing cells were inoculated in a 96-well plate at a density of $4 \times 10^3$ cells/well and incubated for 24 h at 37°C and under the conditions of 5% $CO_2$, 95% air and 100% relative humidity. The tested compound was then added. The compound was dissolved in 100% DMSO at 200 times of the desired final test concentration. When the drug was added, the compound was further diluted to 4 times of the desired final test concentration by using the complete medium. 50 μL aliquots of the compound with specific concentration were added to the microwells containing 150 μL of medium, and then the final drug concentration was obtained. After the drug was added, the plate was further incubated for 2 h at 37°C and under the conditions of 5% $CO_2$, 95% air and 100% relative humidity. The drug was then washed. The fresh medium was added, and the plate was further incubated for 70 h at 37°C and under the conditions of 5% $CO_2$, 95% air and 100% relative humidity. After the incubation was finished, the Alamar Blue was used to analyze and quantify the alive cells. The drug concentration resulting in 50% growth inhibition ($IC_{50}$) was calculated by the computer software. The results found that compound A had 10.85 μmol/L of $IC_{50}$ against AA8 cell line and 0.34 μmol/L of $IC_{50}$ against UV41 cell line with DNA gene repair mutation, and the results are shown in FIG. 8.

UV41 cell line is a derivative of CHO-AA8 cell line and is derived from the ultraviolet-sensitive line of AA8. This cell line is a cell line with impaired homologous recombination DNA repair enzymes and impaired nucleotide excision repair enzymes caused by DNA ERCC4/XPF gene mutation.

It has been shown in the literature that the UV41 cell line, relative to AA8, is a cell with a defect in a nucleotide excision repair enzyme. It is sensitive to bulky adduct mutagens and belongs to the excision repair complement group 4. UV41 is extremely sensitive to DNA cross-linker (see Thompson LH, et al. Repair of DNA adducts in asynchronous CHO cells and the role of repair in cell killing and mutation induction in synchronous cells treated with 7-bromomethylbenz[a]anthracene. Somatic Cell Mol. Genet. 10: 183-194, 1984. PubMed: 6584989; Thompson LH, et al. Genetic diversity of UV-sensitive DNA repair mutants of Chinese hamster ovary cells. Proc. Natl. Acad. Sci. USA 78: 3734-3737, 1981. PubMed: 6943579; Hoy CA, et al. Defective DNA cross-link removal in Chinese hamster cell mutants hypersensitive to bifunctional alkylating agents. Cancer Res. 45: 1737-1743, 1985. PubMed: 3919945; Busch D, et al. Summary of complementation groups of UV-sensitive CHO cell mutants isolated by large-scale screening. Mutagenesis 4: 349-354, 1989. PubMed: 2687628; Bessho T, et al. Initiation of DNA interstrand cross-link repair in humans: the nucleotide excision repair system makes dual incisions 5" to the cross-linked base and removes a 22- to 28-nucleotide-long damage-free strand. Mol. Cell. Biol. 17: 6822-6830, 1997. PubMed: 9372913; Thompson LH, et al. Hypersensitivity to mutation and sister-chromatid-exchange induction in CHO cell mutants defective in incising DNA containing UV lesions. Somatic Cell Genet. 8: 759-773, 1982. PubMed: 7163954). Impaired DNA repair enzymes of UV41 cells can cause mutations in any one or more of the genes corresponding to BRCA1, BRCA2, FANCA, FANCD1, FANCD2, ATM, ATR, CHEK1, CHEK2, CTP, BARD1, BRIP1, PALB2, RAD51D, RAD51C, RAD52, RAD54, RAD55, RAD57, FAM175, NBN, Rad50, MRE11, p53, NBS1, XRS2, XRCC2, XRCC3, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA Ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RAD51B, RBBP8, SMC5, USP11, WRN and AP endonucleases, End processing enzymes, DNA polymerases, and Flap endonuclease.

There is a higher sensitivity to cell lines with DNA repair gene mutations. Specifically, compound A has a higher sensitivity to cell lines with impaired homologous recombination DNA repair enzymes and impaired nucleotide excision repair enzymes.

3.3 Hypoxia test of different cancer cell lines (clonogenic assay)

The following PSN-1 cells, PANC1 cells, MiaPaCa-2 cells, H460 cells, DLD-1 WT (wild type) cells, DLD-1 BRCA2 KO (BRCA2 deletion) cells were seeded in 10cm vitreous cell culture plates at specific cell numbers and incubated for 2 days at 37°C and under the conditions of 5% $CO_2$, 95% air and 100% relative humidity. On day 3, test compounds TH302 or AST-002-P2 at the specific concentrations were added to the cells, and incubated for 3 hours together with the cells under normoxic (21% $O_2$) and hypoxic conditions (<0.01% $O_2$), respectively. After cells were stimulated by compounds, the cells were washed once with 1×PBS and digested into single cells with pancreatic enzymes for cell counting. The above cells in specific cell numbers were reseeded in a new culture plate and cultured for 7 days to perform single cell colony formation. When single cells in compound culture plates stimulated under normoxic conditions proliferated to about 50 cells, the cultures were removed and stained with 0.5% crystal violet for 40 minutes. After staining, cells were counted for colonies using Colony Counter (VWR) and the $IC_{90}$ was calculated.

Results are shown in Table 8 below, and there is a large difference in the inhibitory effect of compound C on MiaPaCa-2 cells and DLD-1 BRCA2 KO cells under normoxic and hypoxic conditions, with fold change (HCR) of > 63-fold and > 89-fold, respectively. This indicates that hypoxic conditions can well activate the cytotoxicity of compound C in both tumor cell lines.

Table 8: Inhibition $IC_{90}$ values of the compound C on different cancer cell lines cultured under normoxic and hypoxic conditions

| Cell line | TH-302 | | | Compound C | | |
|---|---|---|---|---|---|---|
| | $IC_{90}$ Normoxia (μM) | $IC_{90}$ Hypoxia (μM) | HCR | $IC_{90}$ Normoxia (μM) | $IC_{90}$ Hypoxia (μM) | HCR |
| PSN-1 | 30.505 | 0.164 | 186 | >1 | 0.286 | >3.5 |
| PANC1 | >100 | 7.289 | >13.7 | >10 | >1 | ND |
| MiaPaCa-2 | 9.678 | 0.388 | 25 | >10 | 0.159 | >63 |
| H460 | 15.554 | 0.021 | 741 | 0.066 | 0.041 | 1.6 |
| DLD-1 WT | 26.155 | 0.21 | 125 | >0.14 | >0.14 | ND |
| DLD-1 BRCA2 KO | 21.917 | 0.003012 | 7277 | >10 | 0.112 | >89 |

Note: HCR means hypoxic activation ratio, i.e., $IC_{90}$ ratio under normoxic/$N_2$ conditions.
PSN-1, a human pancreatic cancer cell line.
PANC1, a human pancreatic cancer cell line, which is derived from pancreatic cancer ductal cells.
Miapaca-2, a human pancreatic cancer cell line.
H460, a human large cell lung cancer cell, which is established from the pleural fluid of the patient with large cell lung cancer.
DLD-1 WT, a wild type of colorectal cancer cell line.
DLD-1 BRCA2 KO, a colorectal cancer cell line with a deletion of the BRAC2 gene.

**IV. Hypoxia-dependent reduction experiments**

The ingredients were added to each test tube without the addition of NADPH. Then 50 μl of sample from condition B was transferred to 100 μl acetonitrile (T = 0). For samples to be tested under hypoxic conditions, the test tubes were placed in an hypoxic chamber (BACTRON anaerobic/environmental chamber). The reaction was initiated by adding NADPH to test tubes A and C and the sample was then placed at 37 °C. For samples to be tested in air, the test tubes were placed in a hot plate at 37°C. After 30 minutes and 2 hours of incubation, 50 μl of samples were taken from each test tube and mixed with 100 μl acetonitrile. Any protein precipitate was removed by spining all test tubes and placed into HPLC vials for analysis. The results were compared to the calibration curve of the real standard. The specific reagent composition in each sample reaction tube is shown in Table 9 below.

Table 9: The reactant composition of each sample tube in the hypoxia-dependent reduction experiments

| Sample tube | PBS solution | Test drug | CYP450R enzyme solution | NADPH solution |
|---|---|---|---|---|
| A | 450 μL | 10 μL | 20 μL | 20 μL |

(continued)

| Sample tube | PBS solution | Test drug | CYP450R enzyme solution | NADPH solution |
|---|---|---|---|---|
| B | 470 μL | 10 μL | 20 μL | 0 μL |
| C | 470 μL | 10 μL | 0 μL | 20 μL |

The amount of test drug (TH-302, compound A) at different times (0/30min/120min) was compared to the initial amount in three different sample reaction tubes of A/B/C to obtain the data in Figure 9 (53.2 μg/ml human reductase) and Figure 10 (106.4 μg/ml human reductase), respectively.

Test conclusion:

In the presence of the NADPH and CYP 450R enzymes, compound A showed a hypoxia-dependent reduction reaction, and the reduction rates of compound A and TH-302 were similar.

## V. Animal model experiments with the single drugs and combined drugs of PDX and CDX

The animal experiment illustrated that all the animal studies in this application are performed by the professional CRO (Contract Research Organization) company, whose experimental protocols are approved by the laboratory animal ethics committee within the institution or a similar institution, and the treatment of animals meets the requirements.

5.1 *In vivo* drug efficacy test of compound A in HuPrime® model GA6201 human gastric cancer subcutaneous transplantation PDX tumor model

BALB/c nude mice were subcutaneously inoculated with HuPrime® model GA6201 tumor blocks to establish human gastric cancer subcutaneous transplantation tumor model. The test was divided into groups of test drug Ifosfamide (i.e. TH-302) 60 mg/kg group, test drug compound A 5-50 mg/kg group and normal saline (pH 7.0-7.6) vehicle control group, with five mice per group. Among them, the normal saline (pH 7.0-7.6) vehicle control group was administered by tail vein injection once a week for 3 weeks, and observed for 4 weeks. Ifosfamide 60 mg/kg group was administered by intraperitoneal injection for consecutive 5 days followed by 2 days off every week for 2 weeks, and observed for 5 weeks. Test drug 5 mg/kg group was administered by intraperitoneal injection for consecutive 5 days followed by 2 days off every week for 2 weeks (QDx5/week × 2 wks). Then the dose was changed to 10 mg/kg by intraperitoneal injection administration daily for 4 days (QDx4, Day14-Day17). The dose and mode of administration were then changed to 20 mg/kg via tail vein injection for one day (QDx1, Day18). After drug withdrawal for 10 days, the dose was changed again, and 50 mg/kg was administered via tail vein injection once a week for two weeks QWx2 (Day28, Day35). The group was observed for one week. The efficacy was evaluated according to the relative tumor inhibition rate TGI (%), and the safety was evaluated according to the change in body weight and the death of the animals. The specific dosage regimens for each group are shown in Table 10 below.

Table 10: The dosage regimens for each group in the *in vivo* drug efficacy test of compound A in HuPrime® model GA6201 human gastric cancer subcutaneous transplantation PDX tumor model

| Group | Dosage Regimen |
|---|---|
| Group 01 | Group 01, normal saline, pH 7.0-7.6, 0 mg/kg, Q7Dx3, i.v. |
| Group 02 | Group 02, TH-302, 60 mg/kg, QDx5/week x 2 wks, i.p. |
| Group 06 | Group 06, Test drug, 5 mg/kg, QDx5/week x 2 wks, i.p., Test drug (10mg/kg), 10 mg/kg, QDx(day14-day17), i.p., |
| | Test drug (20mg/kg), 20 mg/kg, QDxday18, i.v., Test drug (50mg/kg), 50 mg/kg, QDx(day28, day35), i.v. |

The tumor volumes of mice from different groups were measured at different days and the average values were obtained. The results shown in Table 11 below.

Table 11: Changes of tumor volume at different days of inoculation in HuPrime® model GA6201 human gastric cancer subcutaneous graft PDX tumor model in *in vivo* drug efficacy tests of compound A

| Group Day | Changes of tumor volume at different days of inoculation, the unit of volume is $mm^3$ | | | | | | | | | | | | | | |
|---|---|---|---|---|---|---|---|---|---|---|---|---|---|---|---|
| | 0 | 3 | 7 | 10 | 14 | 17 | 21 | 24 | 28 | 31 | 35 | 38 | 42 | 45 | 49 |
| Group 01 | 94.22 | 123.26 | 161.08 | 182.31 | 242.26 | 297.28 | 398.37 | 479.52 | 579.90 | 648.12 | 831.15 | 905.75 | 1026.01 | 1130.43 | 1283.11 |
| Group 02 | 94.15 | 122.22 | 129.39 | 175.00 | 190.74 | 189.23 | 217.96 | 225.66 | 247.04 | 265.76 | 324.51 | 371.02 | 403.47 | 484.83 | 578.60 |
| Group 06 | 94.24 | 121.79 | 123.19 | 153.46 | 191.17 | 219.91 | 246.57 | 271.72 | 280.86 | 270.68 | 299.31 | 277.17 | 300.46 | 342.08 | 287.47 |

The data in the above table was made into a curve graph, and Figure 11 can be obtained.

The total amount of mice from different groups were weighed at different days and the average values were taken to obtain the results of the change rate of body weight of each group of mice at different days. The results are shown in Figure 12.

In the above dosage regimen, isocyclophosphamide was designed according to the best dosage regimen and maximum safe dosage as described in the literature (Jessica D. Sun, Qian Liu, Dharmendra Ahluwalia, Damien J. Ferraro, Yan Wang, Don Jung, Mark D. Matteucci, and Charles P. Hart. Comparison of hypoxia-activated prodrug evofosfamide (TH-302) and ifosfamide in preclinical non-small cell lung cancer models[J]. Cancer Biology & Therapy, 2016, 17(4): 371-380.), while the compound A was administered in a gradient increasing dosage regimen designed empirically based on the previous cytotoxicity tests, MTD tests, etc. Conclusion: The results showed that compound A of the present invention had significant advantages over the classical chemotherapeutic drug isocyclophosphamide (also drug with a DNA alkylating agent mechanism) in terms of the effect of tumor growth inhibition.

*5.2 In vivo* drug efficacy test of compound A in human lung cancer NCI-H460 subcutaneous xenograft model

BALB/c nude mice were subcutaneously inoculated with human lung cancer NCI-H460 cells to establish human lung cancer subcutaneous transplantation tumor model. The test was divided into groups of test drug compound A 20 mg/kg group and 40 mg/kg group, and 10% ethanol + 10% polyoxyethylene ether (35) castor oil Cremophor EL + 80% of 5% glucose injection (pH 7.4) vehicle control group, with 6 mice per group. Among them, 10% ethanol + 10% polyoxyethylene ether (35) castor oil Cremophor EL + 80% of 5% glucose injection (pH 7.4) vehicle control group was administered by tail vein injection once a week for three weeks and observed for one week. The test drug compound A 40 mg/kg group was administered by tail vein injection once every three weeks for 2 times, and observed for one week after the second administration. The test drug compound A 20 mg/kg group was administered by intraperitoneal injection once a day for 5 days continuously, and observed for 24 days. Efficacy was evaluated according to the relative tumor inhibition rate TGI (%), and safety was evaluated according to the change in body weight and the death of the animals. The administration dosage of compound A was determined according to the MTD test.

The tumor volumes of mice from each group were measured at different days and the average values were taken. The results are shown in Table 12 below and Figure 13.

Table 12: Changes of tumor volume at different days of inoculation in human lung cancer NCI-H460 subcutaneous xenograft model in *in vivo* drug efficacy tests of compound A

| Group | Changes of tumor volume at different days of inoculation, the unit of volume is $mm^3$ | | | | | | | | |
|---|---|---|---|---|---|---|---|---|---|
| | 7 | 10 | 14 | 17 | 21 | 24 | 28 | 31 | 35 |
| Control Group | 144.68 | 226.31 | 339.60 | 493.01 | 774.49 | 977.77 | 1370.471 | 1818.22 | 2299.37 |
| Test Group 7 | 143.84 | 184.06 | 153.75 | 159.72 | 218.13 | 291.07 | 520.17 | 580.70 | 447.32 |
| Test Group 8 | 144.63 | 173.09 | 152.99 | 130.06 | 129.66 | 164.63 | 272.20 | 447.50 | 651.58 |

In addition, the change rates of body weight of mice from each group at different days were calculated, and the results are shown in Figure 14.

Conclusion: The test single drug compound A 40mg/kg (Q3Wx2, IV, test group 7) treatment group showed significant inhibitory effect on tumor on day 31 (Day31) after tumor cell inoculation, which had statistically significant difference compared to the control group (p=0.00561), and the relative tumor inhibition rate TGI (%) was 66.87%. The test single drug compound 01 20mg/kg (QDx5xlweek, IP, test group 8) treatment group showed significant inhibitory effect on tumor on day 31 (Day31) after tumor cell inoculation, which had statistically significant difference compared to the control group (p= 0.000464), and the relative tumor inhibition rate TGI (%) was 76.22%.

5.3 *In vivo* drug efficacy tests of compound B and compound C in human lung cancer NCI-H460 subcutaneous xenograft model

BALB/c nude mice were subcutaneously inoculated with human lung cancer NCI-H460 cells to establish human lung cancer subcutaneous transplantation tumor model. The test was divided into groups of test drug Irinotecan 40 mg/kg group, compound B 20 mg/kg group, compound C 20 mg/kg group and 40 mg/kg group, and 10% ethanol+10%

Cremophor EL+80% glucose injection D5W (pH 7.4) vehicle control group, with 5 mice per group. Among them, 10% ethanol + 10% Cremophor EL + 80% glucose injection D5W (pH 7.4) vehicle control group, test drug compound B 20 mg/kg group, compound C 20 mg/kg group and 40 mg/kg group were administered by intraperitoneal injection once a day for 5 days continuously and 2 days off each week, then 2 weeks off with a total of 2 dosage cycles and observed for 3 days. The positive drug Irinotecan 40 mg/kg group was administered by intraperitoneal injection once every 4 days for 7 times, and observed for 4 days. Efficacy was evaluated according to the relative tumor inhibition rate TGI (%), and safety was evaluated according to the change in body weight and the death of the animals.

The tumor growth of each treatment group and control group is shown in Figure 15.

In addition, the rate of body weight change of mice in each group at different days is shown in Figure 16.

Conclusion: The test drug Irinotecan 40 mg/kg (Q4Dx7, i.p., Group 2) treatment group showed significant inhibitory effect on tumor on day 28 after tumor cell inoculation, which had statistically significant difference compared to the control group (p= 7.44E-05), and the relative tumor inhibition rate TGI (%) was 79.03%. The 20 mg/kg of test drug B (Group 5), 20 mg/kg of C (Group 6) and 40 mg/kg of C (Group 7) treatment groups showed significant inhibitory effects on tumor cells on day 28 (Day 28) after tumor cell inoculation, which all had statistically significant difference compared to the control group (p= 2.90E-06, 5.15E-10 and 1.08E-11), and the relative tumor inhibition rates TGI (%) were 88.51%, 90.56% and 94.33%, respectively. At the end of the overall experiment (Day35), the complete cure rate on tumors in mice of group 6 was 20%. However, all these test drugs exhibited a certain drug toxicity at the tested dosage at the same time, causing significant weight loss or even death in individual or some mice. The test drug Irinotecan (Irinotecan) also had significant inhibition on tumor growth in the human lung cancer NCI-H460 model at a dosage of 40 mg/kg, with statistically significant difference compared to the control group. It also exhibited a certain drug toxicity, causing significant weight loss in mice.

5.4 *In vivo* drug efficacy test of the single drugs and combined drugs of compound C and Sorafenib in human liver cancer HepG2 subcutaneous xenograft model

BALB/c nude mice were subcutaneously inoculated with human liver cancer HepG2 cells to establish human liver cancer subcutaneous transplantation tumor model. The test was divided into a total of 6 groups comprising test drug Sorafenib 30 mg/kg (QDx21 Days) group, test drug compound C 20 mg/kg (QWx3), 40 mg/kg (QWx3) single drug group, compound C 40 mg/kg (QW×3) and Sorafenib (30 mg/kg, administered one week prior to compound C, QDx21 Days) combination administration group, compound C 40 mg/kg (QWx3) and Sorafenib (30 mg/kg, administered simultaneously, QDx21 Days) combination administration group, and the control group of 10% anhydrous ethanol + 10% polyoxyethylene (35) castor oil + 80% glucose injection D5W (pH 7.4), with 5 mice per group. 10% anhydrous ethanol + 10% polyoxyethylene (35) castor oil + 80% glucose injection D5W (pH 7.4) control group, test drug compound C 20 mg/kg, 40 mg/kg single drug groups and their respective treatment groups in combination with Sorafenib were administered by tail vein injection once a week for 3 weeks continuously. The single test drug Sorafenib 30 mg/kg and in combination with compound C were administered by intragastric administration once a day for 21 continuous days. Efficacy was evaluated according to the relative tumor inhibition rate TGI (%), and safety was evaluated according to the change in body weight and the death of animals.

The tumor growth of each treatment group and control group is shown in Figure 17.

In addition, the rate of body weight change of mice in each group at different days is shown in Figure 18.

Conclusion: The relative tumor inhibition rate TGI (%) of the test single agent Sorafenib 30 mg/kg (Group 2) treatment group was 48.11% on day 45 (Day 45) after tumor cell inoculation, which had no statistically significant difference compared to the control group (p= 0.573). The tumor was completely cured in one mouse in this group, with a cure rate of 20%. The test single drug compound C at 20 mg/kg (Group 5) exhibited inhibitory effect on tumor on day 45 (Day 45) after inoculation, with a relative tumor inhibition rate TGI (%) of 33.10%, but no statistically significant difference compared to the control group (p= 0.85). The tumor was completely cured in one mouse in this group, with a cure rate of 20%. The test single compound C 40 mg/kg (Group 6) treatment group exhibited inhibitory effect on tumor on day 45 (Day 45) after tumor cell inoculation, with a relative tumor inhibition rate TGI (%) of 75.59 %, but no statistically significant difference compared to the control group (p= 0.0903). The tumor was completely cured in one mouse in this group, with a cure rate of 20%.

The test combined drug of compound C 40 mg/kg and Sorafenib (30 mg/kg, administered one week before compound C) combination administration group (Group 3) exhibited inhibitory effect on tumor on day 45 (Day 45) after tumor cell inoculation, with a relative tumor inhibition rate TGI (%) of 49.87%, but no statistically significant difference compared to the control group (p= 0.72). The test drug compound C 40 mg/kg and Sorafenib (30 mg/kg, administered simultaneously) combination administration group (Group 4) exhibited significant inhibitory effect on tumor on day 45 (Day 45) after tumor cell inoculation, with statistically significant difference compared to the control group (p= 0.0105) and a relative tumor inhibition rate TGI (%) of 94.53%. The tumor was completely cured in one mouse in this group, with a cure rate of 20%. The results showed that the inhibitory effect on tumors of the test drug compound C 40 mg/kg and

Sorafenib (30 mg/kg, administered simultaneously) combination administration group was better than the test single drug (Groups 2 and 6) treatment groups in corresponding doses, but with no statistically significant difference between the groups.

5.5 Drug efficacy of single drugs of compound C and Anti-mouse-PD1 antibody and combined drugs of compound C and Anti-mouse-PD1 antibody in murine intestinal cancer CT26 subcutaneous model

BALB/c mice were subcutaneously inoculated with murine intestinal cancer CT26 cells to establish a murine intestinal cancer subcutaneous transplantation model. The test was divided into a total of 7 groups comprising test drug compound C 30 mg/kg single drug group (Day 1, Day 4, Day 8, Day 11, Day 15, Day 18), Anti-mouse PD1 Ab 10 mg/kg single drug group (Day 2, Day 5, Day 9, Day 12, Day 16, Day 19), the combination treatment group of compound C 30 mg/kg (Day 1, Day 4, Day 8, Day 11, Day 15, Day 18) and Anti-mouse PD1 Ab 10 mg/kg (Ab second, start dosing on Day 2) (Day 2, Day 5, Day 9, Day 12, Day 16, Day 19), the combination treatment group of compound C 30 mg/kg (Day 1, Day 4, Day 8, Day 11, Day 15, Day 18) and Anti-mouse PD1 Ab 10 mg/kg (Ab second, start dosing on Day 3, Day 3, Day 6, Day 10, Day 13, Day 17, Day 20), the combination treatment group of compound C 30 mg/kg (Day 1, Day 4, Day 8, Day 11, Day 15, Day 18) and Anti-mouse PD1 Ab 10 mg/kg (Ab first, Day 0, Day 3, Day 7, Day 10, Day 14, Day 17), the combination treatment group of compound C 30 mg/kg (Day 1, Day 4, Day 8, Day 11, Day 15, Day 18) and Anti-mouse PD1 Ab 10 mg/kg (Ab simultaneously, Day 1, Day 4, Day 8, Day 11, Day 15, Day 18) and 10% anhydrous ethanol + 10% polyoxyethylene (35) castor oil + 80% glucose injection D5W (pH 7.4) vehicle control group, with 6 mice per group. Among them, in the 10% anhydrous ethanol + 10% polyoxyethylene (35) castor oil + 80% glucose injection D5W (pH 7.4) vehicle control group, the single treatment group of the test drug compound C 30 mg/kg and the combination treatment group of the test drug compound C 30 mg/kg and Anti-mouse PD-1 Ab, the administration was by tail vein injection twice a week. Anti-mouse PD-1 Ab 10 mg/kg in the single treatment group and in the combination treatment group with compound C 30 mg/kg was administered by intraperitoneal injection twice a week. The efficacy of each test drug was evaluated using median survival time, and safety was evaluated according to the change in body weight and the death of animals.

The statistical results of the median survival time at the end of the trial were that the median survival time in the control group was 24 days; the median survival time in the test drug compound C, 30 mg/kg group was 19 days; the median survival time in the test drug Anti-mouse PD1 10 mg/kg group was 28 days; the median survival time in the combination treatment group of compound C 30 mg/kg and Anti-mouse PD1 10 mg/kg (Ab second, start dosing on Day 2) was 28 days; the median survival time in the combination treatment group of compound C 30 mg/kg and Anti-mouse PD1 10 mg/kg (Ab second, start dosing on Day 3) was 28 days; the median survival time in the combination treatment group of compound C 30 mg/kg and Anti-mouse PD1 10 mg/kg (Ab first) was 33 days; and the median survival time in the combination treatment group of compound C 30 mg/kg and Anti-mouse PD1 10 mg/kg (Ab simultaneously) was 28 days. The specific results are shown in Figure 19.

In addition, the rate of body weight change of mice in each group at different days is shown in Figure 20.

The above results showed: Anti-mouse PD1 10 mg/kg antibody single drug and the combined administration of compound C 30 mg/kg and Anti-mouse PD1 10 mg/kg could prolong the median survival time of mice compared to the control group. Comparison between the combined administration group and the PD-1 single drug group showed that the median survival time was prolonged (33 days) only in the combination group of the test drug compound C 30 mg/kg and Anti-mouse PD1 10 mg/kg (Ab first), while the median survival time was not prolonged in other combined administration groups and were all 28 days.

5.6 *In vivo* drug efficacy tests of the single drugs and combined drugs of compound C and Gemcitabine in the HuPrime® pancreatic cancer PA3546 human pancreatic cancer subcutaneous xenograft PDX tumor model

BALB/c nude mice were subcutaneously inoculated with HuPrime® model PA3546 tumor blocks to establish human pancreatic cancer subcutaneous transplantation tumor model. The test was divided into a total of 6 groups comprising test drug Gemcitabine (gemcitabine) 120mg/kg group, test drug compound C 10mg/kg, 20mg/kg and 40mg/kg groups, the combination treatment group of Gemcitabine 120mg/kg and compound C 40mg/kg, and 10% anhydrous ethanol + 10% polyoxyethylene (35) castor oil + 80% glucose injection D5W (pH 7.4) vehicle control group, with 6 mice per group. Among them, 10% anhydrous ethanol + 10% polyoxyethylene (35) castor oil + 80% glucose injection D5W (pH 7.4) vehicle control group, test drug compound C 10 mg/kg, 20 mg/kg and 40 mg/kg groups, and compound C 40 mg/kg in the combination treatment group of Gemcitabine 120 mg/kg and compound C 40 mg/kg were administered by tail vein injection once a week for 3 continuous weeks and observed for 8 days. The test drug Gemcitabine 120 mg/kg in both the single-drug group and the combined-drug group was administered by intraperitoneal injection once a week for 3 consecutive weeks and observed for 8 days. Efficacy was evaluated according to the relative tumor inhibition rate TGI (%), and safety was evaluated according to the change in body weight and the death of animals.

The tumor growth of each treatment group and control group is shown in Figure 21.

In addition, the rate of body weight change of mice in each group at different days is shown in Figure 22.

Conclusion: The test drug Gemcitabine 120mg/kg (Group 2) treatment group showed significant inhibitory effect on tumor on day 25 (Day 25) after the first administration with a statistically significant difference (p= 0.025100) compared to the control group and the relative tumor inhibition rate TGI (%) was 45.31%. The relative tumor inhibition rates (TGI (%)) of the test drug compound C 10 mg/kg (Group 3), 20 mg/kg (Group 4) and 40 mg/kg (Group 5) treatment groups were 32.35%, 36.79% and 35.93% on day 25 (Day 25) after the first administration, respectively, which showed no quantitative effect relationship and statistically no significant difference compared to the control group (p= 0.187000, 0.128000 and 0.193000). The combination treatment group of test drug compound C 40mg/kg and Gemcitabine 120mg/kg (Group 6) exhibited significant inhibitory effect on tumor on day 25 (Day 25) after the first administration, with a statistically significant difference compared to the control group (p= 0.000877) and a relative tumor inhibition rate TGI (%) of 71.16%. In addition, the combination treatment group of compound C 40 mg/kg and Gemcitabine 120 mg/kg (Group 6) showed superior inhibitory effects on tumor compared to the single drug compound C 10 mg/kg (Group 3), 20 mg/kg (Group 4), 40 mg/kg (Group 5) and Gemcitabine 120 mg/kg (Group 2) treatment groups. There was no statistically significant difference between Group 6 and Group 2 and between Group 6 and Group 5.Group 2 vs. group 6, p=0.526000; group 5 vs. group 6, p=0.294000.

Except for Gemcitabine 120 mg/kg (Group 2) treatment group, control group (Group 1) and test drug compound C 10 mg/kg (Group 3) treatment group, test drug compound C 20 mg/kg (Group 4) and 40 mg/kg (Group 5) treatment groups and the combination treatment group of Gemcitabine 120 mg/kg and compound C 40 mg/kg (Group 6) all appeared significant weight loss and even death of individual or part of mice. All of these body weight loss of mice and death of mice may be related to the potential drug toxicity of the test drug at the tested dosage. Mice in the control group (Group 1) and the test drug Gemcitabine 120 mg/kg (Group 2) and the test drug compound C 10 mg/kg (Group 3) treatment groups showed no significant body weight loss and were well tolerated during treatment.

5.7 *In vivo* drug efficacy tests of a single drug of compound C in NCI-H460-Luc2 intracranial inoculation model

BALB/c nude mice were inoculated intracranially with human lung cancer NCI-H460-Luc2 cells to establish human lung cancer intracranial transplantation tumor model. The test was divided into the following groups: test drug Paclitaxel 20 mg/kg group, compound C 10 mg/kg, 20 mg/kg and 40 mg/kg groups and 10% ethanol+10% Cremophor EL+80% glucose injection D5W (pH 7.4) vehicle control group, with 6 mice per group. Among them, 10% ethanol+10% Cremophor EL+80% glucose injection D5W (pH 7.4) vehicle control group, test drug compound C 10mg/kg, 20mg/kg and 40mg/kg groups were administered by tail vein injection once a week for 3 weeks.

The positive drug Paclitaxel 20 mg/kg was administered by intraperitoneal injection once a week for 3 weeks. The relative tumor inhibition rate TGI (%) was calculated based on tumor biofluorescence value and the efficacy was evaluated. The safety was evaluated according to the change in body weight and death of animals.

The tumor growth of each treatment group and control group is shown in Figure 23.

In addition, the rate of body weight change of mice in each group at different days is shown in Figure 24.

Conclusion: At 11 days after grouping, the fluorescence value of the vehicle control group reached 2576.80 E+05 photon/second. Compared with the vehicle control group, the positive control group Paclitaxel (20 mg/kg) had no significant inhibitory effect on tumors, with a mean biofluorescence value of 4184.52 E+05 photon/second (TGI=-62.45, p=0.2278 ); and each treatment group of compound C single drug had extremely statistical significant inhibitory effect on tumors. Among them, the mean biofluorescence value of compound C (10 mg/kg) treatment group was 7.07E+05 photon/second (TGI=99.82%, p<0.01), the mean biofluorescence value of compound C (20 mg/kg) treatment group was 1.79E+05 photon/second (TGI=100.03%, p<0.01) and the mean biofluorescence value of compound C (40 mg/kg) treatment group was 1.04E+05 photon/second (TGI=100.05%, p<0.01). Compared with the Compound C (10 mg/kg) group, Compound C (20 mg/kg) and Compound C (40 mg/kg) groups had statistically significant inhibitory effects on tumors (p=0.0446 and p=0.0241) with dose-dependent. Meanwhile, NCI-H460-Luc2 intracranial inoculation model mice were well tolerated to single drug of compound C.

**VI. Pgp inhibition assay and BBB blood-brain barrier assay**

P-glycoprotein (P-GP, also known as multidrug resistance protein) is a high-molecular-weight protein discovered on the plasma membrane of the multidrug resistant tumor cell and has a transport-pump-like structure. P-GP pumps out a variety of chemotherapy drugs out of cells and reduces intracellular drug concentration. Therefore, P-GP is closely related to the resistance in clinical chemotherapy.

The role of the chemotherapy drug for treating the tumor or cancer of the central nervous system (such as the brain) is limited. The limitation is mainly due to the blood-brain barrier (BBB), which leads to the low permeability of the chemotherapy drug to the tumor system, or the tumor tissue, i.e., the chemotherapy drug may not eliminate the cancer cells by passing through the blood-brain barrier and entering the brain.

The process that the small molecules (e.g., the chemotherapy drug) pass through the blood-brain barrier is

more complicated. The blood-brain barrier, located between the systemic blood circulation and the cerebrospinal fluid, is formed by specialized brain microvascular endothelial cells, along with surrounding cells and perivascular astrocytes through tight junctions between adjacent cells, and it forced to form selective barriers when most molecules pass through, rather than forming a physical barrier around vascular endothelial cells. The small hydrophilic molecules are allowed to pass through the membrane transportation system of the BBB, whereas the bulky hydrophilic molecules, such as many chemotherapy drugs and macromolecular drugs, are excluded from the central nervous system unless they may be actively transported by specific proteins. More particularly, the "efflux pumps" of BBB for protecting the brain tissue (e.g., P-gp) may actively exclude some chemotherapy drugs and bulky molecular drugs from the brain. Therefore, even that the small hydrophilic molecular chemotherapy drugs, including small molecule targeted antitumor drug molecule, may pass through the blood-brain barrier and enter the brain to play a role. However, the small hydrophilic molecular chemotherapy drugs may not work because they are excluded from the central nervous system under the action of the P-GP. In other words, the transmembrane structure of P-GP has the function of the energy dependent "drug pump" and may pump out the hydrophobic lipophilic drugs (e.g., Vincristine (VCR), adriamycin (Dox) or pedialyte glycoside (VP-16)) and decrease the intracellular drug concentration, so that the cytotoxicity is decreased or complete lost.

For the reasons described above, the experimental data of the interaction between P-GP and the compound is used to evaluate the effective degree of the compound on the inhibitory activity of the proliferation of the tumor cells in the central nervous system.

6.1 The evaluation of the potential of the compound as the P-gp substrate, which took MDCKII-MDR1 cells as the model and Verapamil as the inhibitor.

Preparation of the cells for the penetration test:

(1) MDCKII-MDR1 cells were cultured in the cell culture bottle. The conditions of the incubator were 37°C, 5% of $CO_2$, and 95% of the relative humidity. When the cell growth confluence was up to 70-90%, the cells were inoculated to the transwell.

(2) Before the cell inoculation, 50 μL of the culture medium was respectively added to every well of the upper compartment of the transwell, and 25 mL of the culture medium was added to the lower compartment. After the culture plate was incubated in the incubator under 37°C and 5% $CO_2$ for 1 hour, it may be used to inoculate the cells.

(3) After the cells were incubated, the cell suspension was sucked and transferred to a round bottom centrifuge tube, and the tube was centrifuged at 120 g for 5 min.

(4) Cells were resuspended in the culture medium, and the final concentration was 1.56×106 cells/mL. The cell suspension was transferred to the upper compartment of the 96-well transwell, wherein each upper compartment contains 50 μL of the cell suspension. The final inoculum density was $5.45 \times 10^5$ cells/$cm^2$.

(5) The culture medium was replaced after inoculating for 48 h. After culturing for 4-8 days, the medium was changed every other day.

(6) The process of replacing the culture medium was as the follow: The transwell and the receiving plate were separated. The medium in the receiving plate was discarded, and then the medium in the transwell was discarded. Finally, 75 μL of fresh medium was added to each transwell and 25 mL of fresh medium was added to the receiving plate.

Evaluation of the cell monolayer membrane integrity:

(1) The MDCKII-MDR1 cells should be totally confluent and complete the differentiation after culturing for 4-8 days. After that, the MDCKII-MDR1 cells can be used for the penetration test.

(2) Millipore was used to measure the resistance of monolayer membrane, and the resistance of every well was recorded.

(3) After the measuring, the transwell culture plate was put back to the incubator.

(4) The calculation of the resistance: measured resistance (ohms) × area of the membrane = TEER value (ohm·$cm^2$). If TERR value was < 42 ohm·$cm^2$, the well would not be used in the penetration test.

Penetration test of the drug:

(1) The transwell culture plate of the MDCKII-MDR1 cells was taken out from the incubator. The cell monolayer membrane was rinsed two times with the buffer solution and incubated at 37 °C for 30 min.

(2) The transport rate that the compound was transported from the top-end to the base-end was tested. 75 μL of the buffer solution contained the sample was added to each upper compartment (top-end), and 235 μL of the buffer solution was added to each lower compartment (base-end).

(3) The transport rate that the compound was transported from the base-end to the top-end was tested. 75 μL of the buffer solution was added to each upper compartment (top-end), and 235μL of the buffer solution contained the sample was added to each lower compartment (base-end).

(4) 50 μL of the sample was transferred from the working fluid preparation plate to 200 μL of the acetonitrile containing the internal standard (100 nM Alprazolam, 200 nM Labetalol, 200 nM caffeine and 2 μM Ketoprofen), and the mixture was tested as the dosing sample at 0 min.

(5) For testing the transportation of the subjest under the condition that the P-GP inhibitor Verapamil was added, it is necessary to add Verapamil to the buffer salts of the donor and the acceptor of the transwell of MDCKII-MDR1 cells, and the final concentration was 100 μM.

(6) The upper and lower transits were merged up and then incubated at 37°C for 2 h.

(7) After the incubation was finished, 50μL of the samples from each well of the upper compartment and the lower compartment of the transwell culture plate were respectively added to new sample tubes. 200 μL of the acetonitrile containing the internal standard (100 nM Alprazolam, 200 nM Labetalol, 200 nM caffeine and 2 μM Ketoprofen) was added to each sample tube. After the sample tubes were vortexed for 10 min, they were centrifuged at 3220 g for 30 min. 100 μL of the supernatant was taken and diluted with the same volume of the water to perform the LC-MS/MS analysis. Each sample was adopted to the three parallel incubation.

(8) After the cell monolayer membrane was incubated for 2 h, its integrity was evaluated by the leakage assay using the fluorescence yellow, wherin the fluorescence yellow storage solution was diluted by the buffer solution to the final concentration of 100 μM/L. 100 μL of the fluorescence yellow solution was added to each well of the upper compartment of the transwell, and 300 μL of the buffer was added to each well of the lower compartment of the transwell. After the transwell was incubated at 37°C for 30 min, 80 μL of the solution from each well of the upper compartment and the lower compartment was respectively transferred to a new 96-well plate. Microplate reader was used to perform the fluorimetry with the excitation wavelength of 480 nm and the emission wavelength of 530 nm.

Data analysis:

The peak area was calculated by the result of the ion chromatography. The permeability coefficient (Papp, unit: cm/s$\times 10^{-6}$) of the compound was calculated by the formula:

$$P_{app} = \frac{V_A}{Area \times time} \times \frac{[drug]_{acceptor}}{[drug]_{initial,donor}}$$

In the formula, "VA" refers to the volume of the solution on the acceptor (Ap→Bl was 0.235 mL, and Bl→Ap was 0.075 mL), "Area" refers to the area of the permeable membrane of the 96-well transwell (0.143 $cm^2$), "time" refers to the incubation time (in second), "[drug]acceptor" refers to the drug concentration of the acceptor after the incubation, and "[drug]initial, donor" refers to the initial concentration of the drug administration before the incubation.

The efflux ratio was calculated by the following formula:

$$Efflux\ Ratio = \frac{P_{app\ (B-A)}}{P_{app\ (A-B)}}$$

In the formula, "Papp (B-A)" refers to the permeability coefficient from the base-end to the top-end, and the term "Papp (A-B)" refers to the permeability coefficient from the top-end to the base-end.

The recovery was calculated by the following formula:

$$Recovery\% = \frac{[drug]_{acceptor} \times V_A + [drug]_{donor} \times V_D}{[drug]_{initial,donor} \times V_D} \times 100$$

In the formula, "VA" refers to the volume of the solution on the acceptor (mL), "VD" refers to the volume of the solution on the donor (mL), "[drug]acceptor" refers to the drug concentration of the acceptor after the incubation, "[drug]donor" refers to the drug concentration of the donor after the incubation, and the term "[drug]initial, donor" refers to the initial concentration of the drug administration before the incubation.

The fluorescence value LY Leakage of the cell monolayer membrane was calculated by the following formula:

$$\text{LY Leakage} = \left(\frac{I_{acceptor} \times 0.3}{I_{acceptor} \times 0.3 + I_{donor} \times 0.1}\right) \times 100$$

In the formula, "Iacceptor" refers to the fluorescence intensity of the acceptor (0.3 mL), "Idonor" refers to the fluorescence intensity of the donor (0.1 mL). The result is expressed by %LY and LY<1.5% denotes that the cell monolayer membrane is intact.

The efflux ratios of different compounds that were in the presence of or in the absence of P-glycoprotein inhibitor, Verapamil, were obtained after respectively testing a part of the prepared compounds. The test data are shown in Table 13 below.

Table 13: Penetration test data for drugs

| Compound | verapamil (μM) | $P_{app}$ (A-B) ($10^{-6}$, cm/s) | $P_{app}$ (B-A) ($10^{-6}$, cm/s) | Efflux Ratio | Recovery (%) | |
|---|---|---|---|---|---|---|
| | | | | | AP-BL | BL-AP |
| Metoprolol | 0 | 27.57 | 26.51 | 0.96 | 96.18 | 98.46 |
| prazosin | 0 | 10.99 | 31.84 | 2.90 | 90.69 | 78.53 |
| prazosin | 100 | 30.36 | 17.65 | 0.58 | 94.19 | 88.52 |
| Imatinib | 0 | 1.89 | 30.49 | 16.13 | 79.04 | 63.40 |
| Imatinib | 100 | 21.14 | 19.68 | 0.93 | 83.02 | 87.60 |
| B | 0 | 18.36 | 11.91 | 0.65 | 59.31 | 58.81 |
| B | 100 | 22.69 | 12.42 | 0.55 | 65.20 | 68.27 |
| C | 0 | 18.77 | 11.99 | 0.64 | 57.81 | 58.37 |
| C | 100 | 21.69 | 12.52 | 0.58 | 61.55 | 66.29 |

Conclusion: The three key parameters of Papp (A-B), Papp (B-A), Efflux Ratio of compound

B/C in the presence and absence of P-gp inhibitor verapamil are very close to each other; therefore, compound B and C are not substrates for Pgp.

6.2 Blood-brain barrier experiments

Three CD-1 rats were administered 1 mg/kg of the test compound intravenously at each time point. Blood and brain tissue were collected at 5 min, 15 min, 1 h, 2 h, 4 h and 8 h after administration. The concentrations of the test compounds in the samples were determined by LC/MS method and the results are shown in Tables 14 and 15 below.

Table 14: Concentrations of test substances in blood samples at different time points after intravenous administration of compound B to CD-1 mice

| Route | Time (h) | Animal ID | Brain (ng/g) | Plasma (ng/mL) | Ratio (Brain/ Plasma) | Mean |
|---|---|---|---|---|---|---|
| IV | 0.083 | 1 | 916.57 | 393.31 | 2.33 | 2.25 |
| | | 2 | 992.31 | 454.21 | 2.18 | |
| | | 3 | 1038.97 | 465.00 | 2.23 | |
| | 0.25 | 4 | 498.99 | 340.59 | 1.47 | 1.59 |
| | | 5 | 480.24 | 289.25 | 1.66 | |
| | | 6 | 420.87 | 256.49 | 1.64 | |
| | 1 | 7 | 88.14 | 69.98 | 1.26 | 1.39 |
| | | 8 | 104.66 | 67.46 | 1.55 | |
| | | 9 | 103.20 | 75.23 | 1.37 | |
| | 2 | 10 | 9.00 | 6.54 | 1.38 | 1.34 |
| | | 11 | 13.14 | 9.83 | 1.34 | |
| | | 12 | 15.08 | 11.45 | 1.32 | |
| | 4 | 13 | 1.34 | 1.13 | 1.19 | NA |
| | | 14 | BLOQ | 0.52 | NA | |
| | | 15 | BLOQ | 0.28 | NA | |
| | 8 | 16 | BLOQ | BLOQ | NA | NA |
| | | 17 | BLOQ | BLOQ | NA | |
| | | 18 | BLOQ | BLOQ | NA | |

Table 15: Concentrations of test substances in blood samples at different time points after intravenous administration of compound C to CD-1 mice

| Route | Time (h) | Animal ID | Brain (ng/g) | Plasma (ng/mL) | Ratio (Brain/ Plasma) | Mean |
|---|---|---|---|---|---|---|
| IV | 0.083 | 1 | 37729 | 45685 | 0.83 | 1.07 |
| | | 2 | 414.53 | 417.49 | 0.99 | |
| | | 3 | 541.99 | 390.41 | 1.39 | |
| | 0.25 | 4 | 146.13 | 178.95 | 0.82 | 0.86 |
| | | 5 | 131.63 | 132.02 | 100 | |
| | | 6 | 120.75 | 154.92 | 0.78 | |
| | 1 | 7 | 28.63 | 54.81 | 0.52 | 0.54 |
| | | 8 | 18.01 | 25.61 | 0.70 | |
| | | 9 | 15.01 | 37.23 | 0.40 | |
| | 2 | 10 | 6.31 | 4.69 | 1.35 | 1.11 |
| | | 11 | 1.90 | 2.16 | 0.88 | |
| | | 12 | BLOQ | 1.64 | NA | |
| | 4 | 13 | BLOQ | BLOQ | NA | NA |
| | | 14 | BLOQ | BLOQ | NA | |
| | | 15 | BLOQ | BLOQ | NA | |
| | 8 | 16 | BLOQ | BLOQ | NA | NA |
| | | 17 | BLOQ | BLOQ | NA | |
| | | 18 | BLOQ | BLOQ | NA | |

The corresponding results of the concentrations of compounds B and C versus time in blood and brain tissue were made into a curve graph, as shown in Figure 25.

The corresponding results of the ratio of the concentration of compound B to that of compound C versus time in brain tissue and blood were made into a curve graph, as shown in Figure 26.

The PK (pharmacokinetic) parameters obtained from the above tests were listed in Tables 16 and 17.

Table 16: PK parameters obtained by testing in plasma

| PK parameters | Unit | B Compound | C Compound |
|---|---|---|---|
| Cl_obs | mL/min/kg | 57.63 | 87.92 |
| T1/2 | h | 0.45 | 0.3 |
| C0 | ng/mL | 531.77 | 694.23 |
| AUClast | h*ng/mL | 28879 | 188.34 |
| AUCInf | h*ng/mL | 289.2 | 189.57 |
| MRTInf_obs | h | 0.46 | 0.33 |
| Vss_obs | L/kg | 1.59 | 1.73 |

Table 17: PK parameters obtained by testing in brain tissue

| PK parameters | Unit | B Compound | C Compound |
|---|---|---|---|
| T1/2 | h | 0.33 | 0.35 |

(continued)

| PK parameters | Unit | B Compound | C Compound |
|---|---|---|---|
| Tmax | h | 008 | 0.08 |
| Cmax | ng/g | 982.61 | 445 |
| AUClast | h*ng/g | 429.34 | 136.59 |
| AUCInf | h*ng/g | 435.32 | 138.68 |
| MRTInf_obs | h | 0.41 | 0.34 |
| AUCInf Ratio (Brain/Plasma) | - | 149 | 0.73 |

Conclusion: Compounds B and C may be detected in brain tissue, showing that both compounds can penetrate the blood-brain barrier.

**VII. Metabolic stability evaluation experiments**

7.1 Stability experiments of liver microsomes

1) Two individual experiments were performed.

1a) With NADPH: 10 μL of 20 mg/mL liver microsomes and 40 μL of 10 mM NADPH were added to the culture solution. The final concentrations of microsomes and NADPH were 0.5 mg/mL and 1 mM, respectively.

1b) Without NADPH: 10 μL of 20 mg/mL liver microsomes and 40 μL of ultrapure $H_2O$ were added to the culture solution. The final concentration of microsomes was 0.5 mg/mL.

2) The reaction was started by adding 4 μL of 200 μM test compound solution at a final concentration of 2 μM or control compound (verapamil) solution and incubated at 37°C.
3) 50 μL of aliquots were taken from the reaction solution at 0 min and 30 min. The reaction was terminated by adding 4 volumes of cold acetonitrile and four internal standards (IS, 100 nM alprazolam, 200 nM caffeine, 200 nM labetalol and 2 μM ketoprofen). The samples were centrifuged at 3, 220 × g for 40 min. The aliquots of 100 μL of supernatant were mixed with 100 μL of ultrapure $H_2O$ and then used for LC-MS/MS analysis. All experiments were performed in duplicate. The test results are shown in Table 18.

Table 18: Remaining amount of compound A in the stability experiments of liver microsomes after 30 minutes

| Compound | Remaining percentage after 30 minutes | | | | | | | | | |
|---|---|---|---|---|---|---|---|---|---|---|
| | Human | | Monkey | | Dog | | Rat | | Mouse | |
| | with NADPH | without NADPH | with NADPH | without NADPH | with NADPH | without NADPH | with NADPH | without NADPH | with NADPH | without NADPH |
| verapamil | 15.55 | 98.27 | 0.19 | 92.44 | 15.73 | 97.58 | 1.13 | 89.24 | 2.91 | 93.95 |
| Compound C | 96.39 | 82.15 | - | - | 31.16 | 101.62 | 71.05 | 111.15 | 102.89 | 103.07 |

The test data was made into a curve graph as shown in Figure 27.

Compound A is relatively stable in human and mouse liver microsomes, but not stable in rat and canine liver microsomes.

For compound D, the experimental procedure was similar and the results of the test are shown in Table 19 below.

Table 19: Remaining amount of compound D in the stability experiments of liver microsomes after 30 minutes

| Compound | Remaining percentage after 30 minutes | | | | | | | | | |
|---|---|---|---|---|---|---|---|---|---|---|
| | Human | | Monkey | | Dog | | Rat | | Mouse | |
| | with NADPH | without NADPH | with NADPH | without NADPH | with NADPH | without NADPH | with NADPH | without NADPH | with NADPH | without NADPH |
| verapamil | 2.11 | 102.23 | 0.23 | 95.67 | 0.52 | 100.86 | 0.33 | 102.96 | 0.16 | 96.40 |
| Compound D | 74.28 | 107.67 | 0.86 | 95.89 | 9.97 | 99.10 | 6.81 | 103.00 | 32.37 | 96.37 |

7.2 Plasma stability test

1) Plasma preparation

The frozen plasma was immediately melted in a water bath of 37 °C. The plasma was centrifuged at 3, 220g for 10 minutes to remove the blood clots and the supernatant was collected into a new test tube. The pH of the plasma was checked and recorded. Note: a. Only plasma that has been melted no more than twice since arrival was used; b. Only plasma in the range of pH 7 to pH 8 was used.

2) Preparation of experimental solutions

1 mM of working solutions of the test compound and the control compound mevinolin were prepared in DMSO. 1 mM of the working solution of the control compound solution in propane was prepared in acetonitrile. 4 μL of the working solution was added to 796 μL of pre-incubated plasma to achieve 5 μM of final concentration. The final concentration of solvent was 0.5%.

3) Test procedure for plasma stability

The aliquots of 50 μL of the standard addition plasma were added to new test tubes and incubated in the water bath of 37 °C at approximately 60 rpm for 2 hours. The assay is performed in duplicate. At the end of the two hour incubation, 300 μL of quenched solution at room temperature (acetonitrile containing internal standards (acetonitrile, 100 nM Alprazolam, 500 nM Labetalol and 2 μM ketoprofen)) was added to terminate the reaction. Samples at time 0 were prepared by adding 50 μL of standard addition plasma to the new test tubes with 300 μL of quenched solution at room temperature. The test tubes were mixed by vortex for 5 minutes. The samples in the plates were centrifuged at 3, 220 × g for 30 minutes at 4°C to precipitate the proteins. 100 μL of supernatant was transferred to a new plate. The supernatant was diluted with 100 μL of water, mixed well and analysed using LC-MS/MS.

The test results after 30 minutes are shown in Table 20 below.

Table 20: Remaining percentage of compound A in the plasma of different species after 30 minutes

| Compound | Species | Remaining percentage |
|---|---|---|
| Propanthine | Human | 0.07 |
| Lovastatin | Rat | 0.21 |
| Propanthine | Mouse | 5.33 |
| Lovastatin | Dog | 37.6 |
| Propanthine | Monkey | 48.11 |
| Compound A | Human | 68.68 |
| Compound A | Rat | 52.11 |
| Compound A | Mouse | 55.07 |
| Compound A | Dog | 85.04 |

The test data was made into a curve graph, as shown in Figure 28.

For compound D, the experimental procedure was similar and the results of the test are shown in Table 21 below.

Table 21: Remaining percentage of compound D in the plasma of different species after 30 minutes

| Compound | Species | Remaining percentage |
|---|---|---|
| Propanthine | Human | 3.32 |
| Lovastatin | Rat | 0.19 |
| Propanthine | Mouse | 1.98 |
| Lovastatin | Dog | 58.73 |
| Propanthine | Monkey | 54.38 |

(continued)

| Compound | Species | Remaining percentage |
|---|---|---|
| Compound D | Human | 97.06 |
| Compound D | Rat | 85.49 |
| Compound D | Mouse | 87.35 |
| Compound D | Dog | 91.95 |
| Compound D | Monkey | 87.66 |

Conclusion: Compound D was relatively stable in canine plasma and secondarily in human plasma. It was relatively unstable in rat and mouse plasma.

The metabolism data of compound A in the blood of different animals over 2 hours were further tested and the results are shown in Table 22 below.

Table 22: Metabolism data of compound A in the plasma of different animals over 2 hours

| Species | Remaining percentage (%) | | | | | | t1/2 (min) |
|---|---|---|---|---|---|---|---|
| | 0 min | 15 min | 30 min | 60 min | 90 min | 120 min | |
| Mouse | 100 | 104±2.96 | 97.7±4.71 | 86.6±3.16 | 76.9±2.40 | 66.4±0.993 | 189±13.5 |
| Rat | 100 | 101±1.33 | 100±2.23 | 88.9±0.537 | 84.6±1.20 | 69.0±1.10 | 226±9.73 |
| Dog | 100 | 107±2.54 | 105±1.27 | 101±5.20 | 105±3.67 | 98.9±0.476 | >373* |
| Monkey | 100 | 97.9±3.72 | 107±2.78 | 103±4.19 | 92.3±2.15 | 87.4±1.59 | >373* |
| Human | 100 | 108±0.773 | 102±3.85 | 99.2±3.39 | 93.8±3.12 | 89.7±4.43 | >373* |

The remaining percentage of compound A in the plasma of mouse, rat, dog, monkey and human at 120 min was 66.4%, 69.0%, 98.9%, 87.4% and 89.7%, respectively. The half-life of compound A *in vitro* in the plasma of mouse, rat, dog, monkey and human was 189 min, 226 min, >373 min, >373 min and >373 min, respectively. The experimental results showed that compound A was stable in the plasma of dog, monkey and human for at least 2 hours at 37°C, while it was unstable in the plasma of mouse and rat.

**VIII. Maximal tolerance dose (MTD) test**

1. Compound B

Experimental procedure

18 ICR mice, 9 females/9 males, were randomly divided into compound B 10mg/kg group, 20mg/kg group and 40mg/kg group according to gender and body weight, with 3 females/3 males in each group. The drug was administered by intravenous injection, and the groups of compound B 10mg/kg, 20mg/kg and 40mg/kg were administered compound B at concentrations of 1, 2 and 4 mg/ml, respectively, with a volume of 10 ml/kg and doses of 10, 20 and 40 mg/kg, respectively. It was administered once a day for 5 days, and recovered for 2 weeks after the last administration.

The animals were closely observed for 2 h after administration on the administration day and once a day in the morning and once in the afternoon on the non-administration day. Body weight and dietary intake were measured twice a week during the test period. Dead animals and all surviving animals after 14 days from the last administration were subjected to autopsy. The autopsy was performed on the pathological organs, and histopathological examination of the pathological organs was performed.

Experimental Results

(1) Animal death conditions

Three of the six animals from the compound B 40mg/kg group died during the trial period, and the time of death was concentrated in the recovery period of 2-6 days.

(2) Clinical symptoms of animals after administration

During the trial period, the animals in the compound B 10mg/kg group and the compound B 20mg/kg group were in good condition in general; the animals in the compound B 40mg/kg group intermittently appeared unstable gait, deepening respiration, short breath and reduced spontaneous activity, and during the recovery period of 2-6 days, individual dying animals appeared reduced spontaneous activity, piloerection, roachback and spasticity.

(3) Effects on animal body weight

After mice were administered compound B by intravenous injection, the body weight of the animals increased slowly. The body weight of female animals was reduced on day 3 during administration and on day 2 during recovery as compared to that of pre-administration. On day 5 during recovery, the body weight gain resumed. The body weight of surviving animals in 40 mg/kg group of male animals was reduced on day 5 during recovery.

(4) Effect on animal dietary intake

The mean dietary intake of animals in each dosage group was not significantly different from that of normal mice (background data from the present laboratory).

(5) Pathological examination results

Tissues were removed from dead animals for examination. At the end of the recovery observation period, no significant macropathological changes were observed on autopsy of the surviving animals.

Experimental Conclusion

The maximal tolerance dose of single-cycle compound B administered intravenously to ICR mice under the conditions of the present test was 20 mg/kg.

2. Compound C

Experimental Procedure

18 ICR mice, 9 females/9 males, were randomly divided into compound C 10mg/kg group, 20mg/kg group and 40mg/kg group according to gender and body weight, with 3 females/3 males in each group. The drug was administered by intravenous injection, and the groups of compound C 10mg/kg, 20mg/kg and 40mg/kg were administered compound C at concentrations of 1, 2 and 4 mg/ml, respectively, with a volume of 10 ml/kg and doses of 10, 20 and 40 mg/kg, respectively. It was administered once a day for 5 continuous days, and recovered for 1 week after the last administration. After continuous administration for 5 days, there was no animal death in any of the dosage groups. To explore the maximal tolerance dose, two cycles of administration were required. The dosage was adjusted and the administration was continued for 5 days. The animals were observed for 14 consecutive days after the final administration. The dosage was adjusted as shown in Table 23 below.

Table 23: Dosage adjustment for MTD experiments of compound C

| Original dosage group | Dosage adjusted in second cycle | Group identification |
|---|---|---|
| Compound C 10 mg/kg group | 80 mg/kg | Compound C 80 mg/kg group |
| Compound C 20 mg/kg group | 60 mg/kg | Compound C 60 mg/kg group |
| Compound C 40 mg/kg group | 40 mg/kg | Compound C 40 mg/kg group |

The animals were closely observed for 2 h after administration on the administration day and once a day in the morning and once in the afternoon on the non-administration day. Body weight and dietary intake were measured twice a week during the test period. Dead animals and all surviving animals after 14 days from the last administration were subjected to autopsy. The autopsy was performed on the pathological organs, and histopathological examination of the pathological organs was performed.

Experimental results

(1) Animal death conditions

During the trial period, 1/6 animals from the compound C 10/80 mg/kg group (indicating that the first cycle was administered at 10 mg/kg and then the second cycle continued at 80 mg/kg) died; 3/6 animals from the compound C 20/60 mg/kg group died; and 1/6 animals from the compound C 40/40 mg/kg group died. The time of death concentrated in the recovery period of 3-7 days.

(2) Clinical symptoms of animals after administration

During the first cycle of administration, the animals in the compound C 10/80 mg/kg group were in good condition in general; the animals in the compound C 20/60 mg/kg group appeared unstable after administration; and the animals in the compound C 40/40 mg/kg group intermittently appeared unstable gait, short respiration and increased spontaneous activity after administration from the immediate to 5 min post-administration.

During the second cycle of administration, the animals in the compound C 10/80 mg/kg group intermittently appeared abnormal gait, decreased muscle tone, decreased spontaneous activity, irregular breath, unstable gait, short breath, piloerection, twitching, deepening breath, prone, and roachback from the immediate to 1h post-administration; the animals in the compound C 20/60 mg/kg group intermittently appeared deepening breath, decreased spontaneous activity, unstable gait, irregular breath, increased spontaneous activity, abnormal gait, and prone from immediate to 30min post-administration; the animals in the compound C 40/40 mg/kg group intermittently appeared unstable gait, deepening breath, short breath, increased spontaneous activity, irregular breath, and unstable gait from immediateto 10-30 min post-administration; and the animals in the compound C 10/80 mg/kg group and compound C 20/60 mg/kg group appeared salivating and moist around the mouth during the recovery period of 3-4 days.

(3) Effect on body weight of animals

After mice were administered compound C by intravenous injection, the body weight of the animals increased slowly.

For male animals, it was found on day 3 of administration during the first cycle that: in the 10 & 40 mg/kg groups, body weight decreased compared to the pre-administration; and in the 20 mg/kg group, body weight increased slowly.

For female animals, on day 3 of administration and day 1 of recovery during the first cycle, body weight was reduced compared to the pre-administration. On day 5 of recovery, body weight growth resumed.

For all animals in the administration group (regardless of sex), body weight was reduced in the 20/60 mg/kg group and 40/40 mg/kg group during the second cycle compared to the pre-administration at day 5 of recovery.

(4) Effect on animal dietary intake

The mean dietary intake of animals in each dosage group was not significantly different from that of normal mice (background data from the present laboratory).

(5) Pathological examination results

Tissues were removed from dead animals for examination. At the end of the recovery observation period, no significant macropathological changes were observed on autopsy of the surviving animals.

Experimental conclusion

The maximal tolerance dose of compound C intravenous administered to ICR mice under the conditions of the present test was >40 mg/kg during the first cycle; and the maximal tolerance dose of compound C administered during the second cycle was <40 mg/kg. In conclusion, the maximal tolerance dose of compound C was in the range of 20-40 mg/kg, similar to or slightly higher than that of compound B.

3. Comparison of MTD of compounds B and C with TH-2565/2566

In the prior literature (WO2016210175A1, CN108024974A), the MTD test results of compound TH-2565/2566 in mice were disclosed as 2mg/kg, respectively. The MTD results of anti-cancer compounds B and C disclosed in the present invention as tested and those of compound TH-2565/2566 in the literature were listed.

Table 24: Comparison of MTD results of compounds B and C with TH-2565/2566

| Compound Code | Compound Structure | MTD Result (mg/kg) |
|---|---|---|
| TH2565 | | 2 |
| TH2566 | | 2 |
| Compound B | 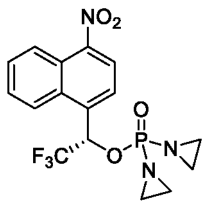 | 20 |
| Compound C | 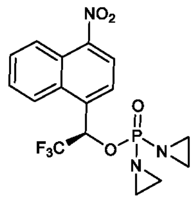 | 20-40 |

The results of the maximal tolerance dose test indicate that the anticancer compound A provided by the present invention has stronger safety for animals, i.e. it is possible that larger dosages can be used in clinical treatment to achieve better therapeutic results.

Further, the prior literature (WO2016210175A1, CN108024974A) as a comparison discloses that compound TH-2565/2566 is the overall optimal compound among the anti-cancer compounds with a benzene ring structure. By comparison, it can be presumed that the compound with new structure of the present invention obtained through new structural modifications has a higher MTD, i.e. it can improve the safety and clinical treatment dosage of the new compound.

## Claims

**1.** A compound having any one of the following structural formula, or a pharmaceutically acceptable salt, a prodrug, a solvate or an isotopic variant thereof,

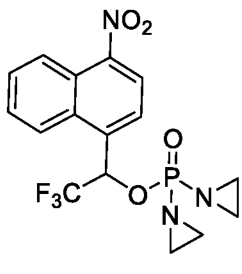 , 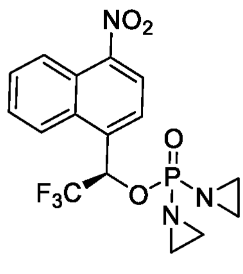 , 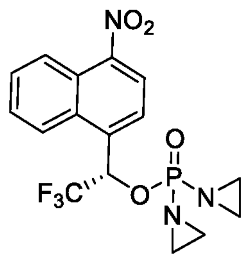 ,

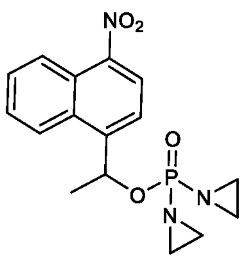 , 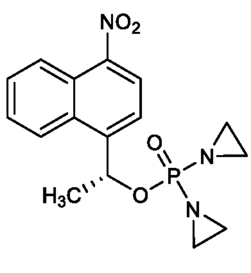 , 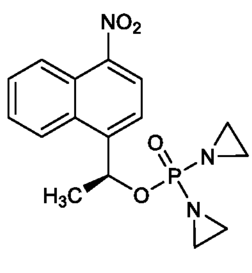 .

2. The compound according to claim 1, wherein the structural formula of the isotopic variant is as follows:

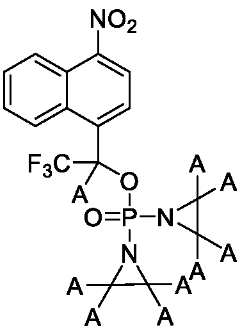 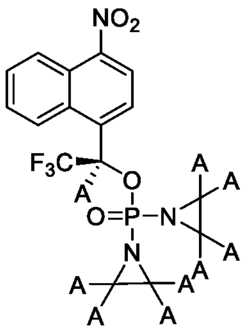 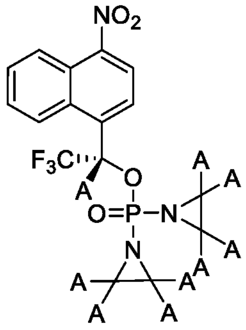 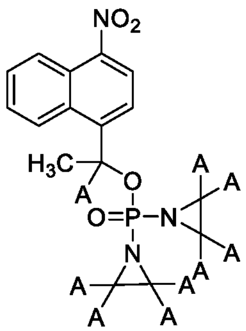

wherein each A is independently H or D, and at least one of the nine A is D.

3. The compound according to claim 1, wherein the isotopic variant corresponds to a deuterated compound with the following structures:

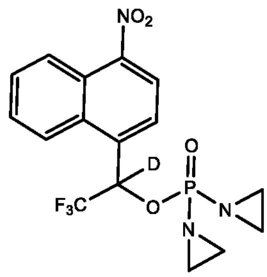 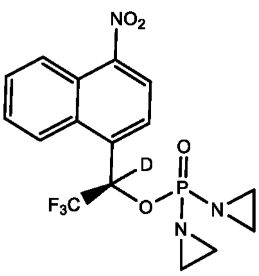 , 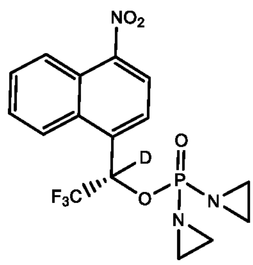 , 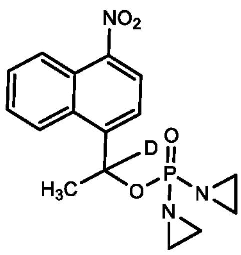 ,

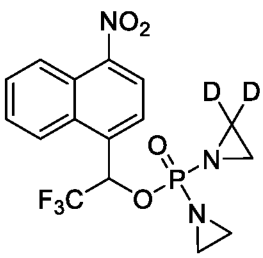 , 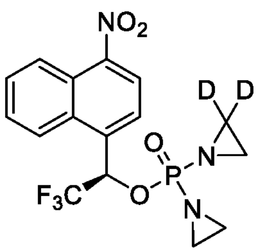 , 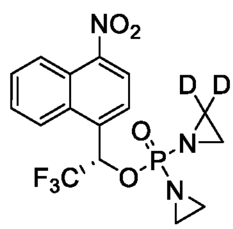 , 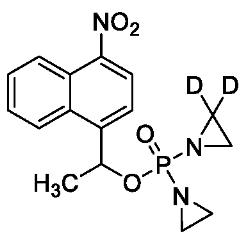 ,

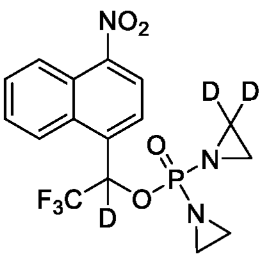 , 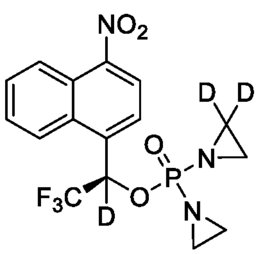 , 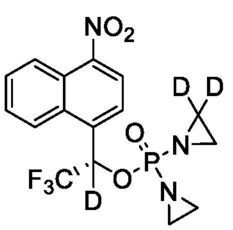 , 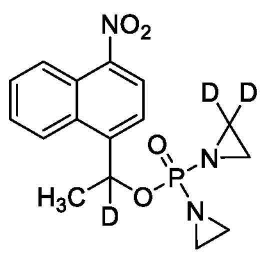 ,

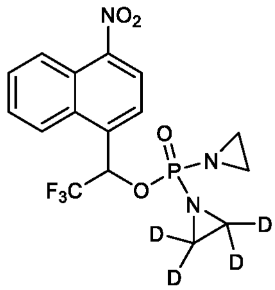 , 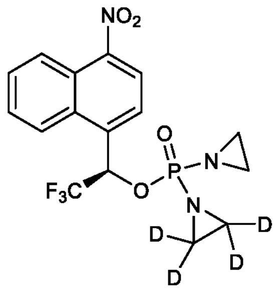 , 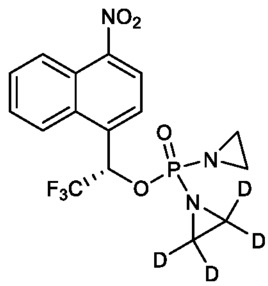 , 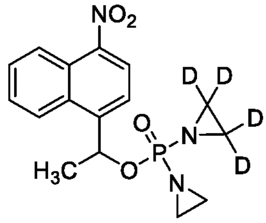 ,

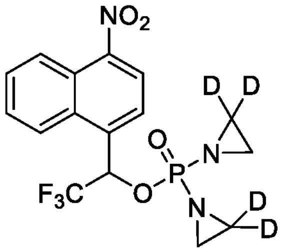 , 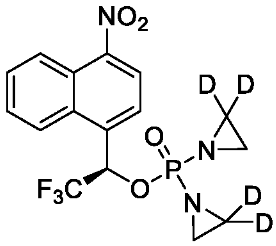 , 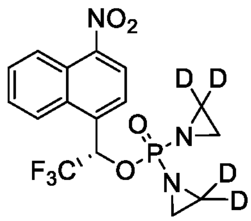 , 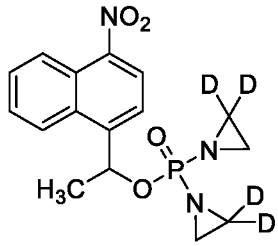 ,

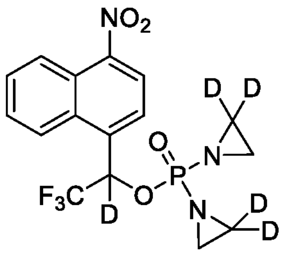 , 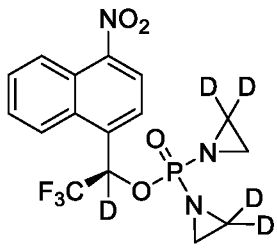 , 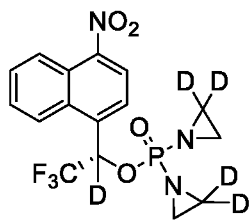 , 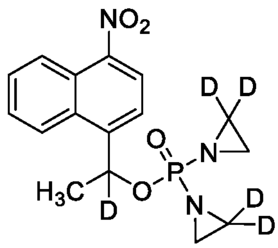

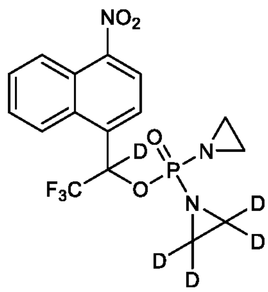 , 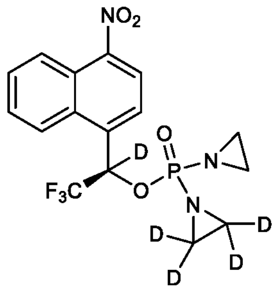 , 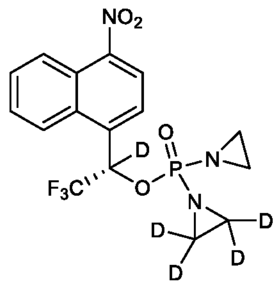 , 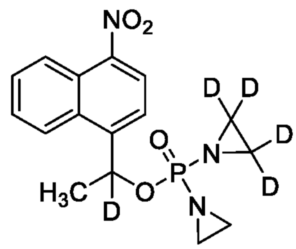

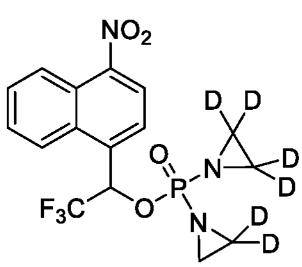 , 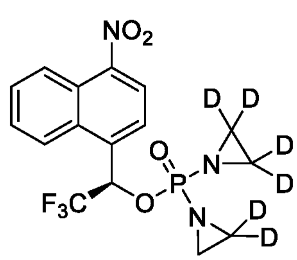 , 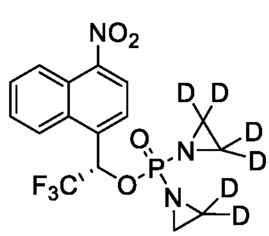 ,

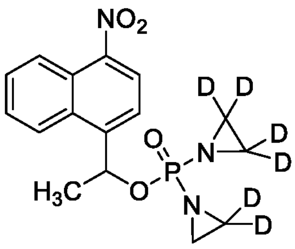

,

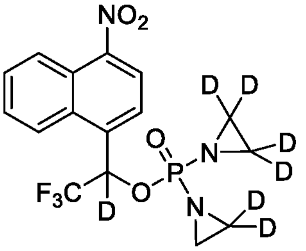

,

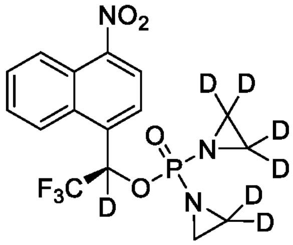

,

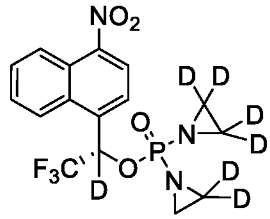

,

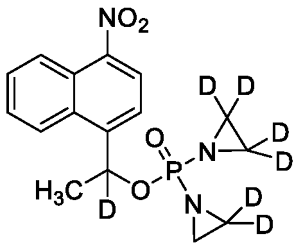

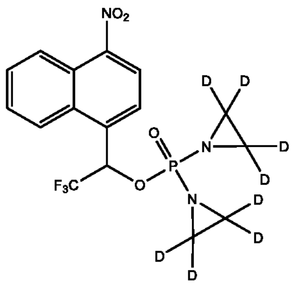

,

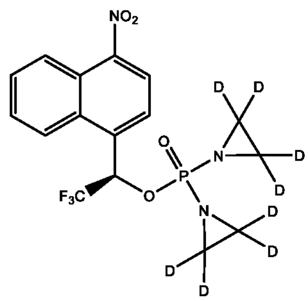

,

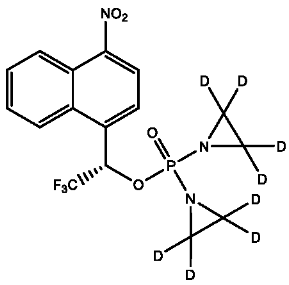

,

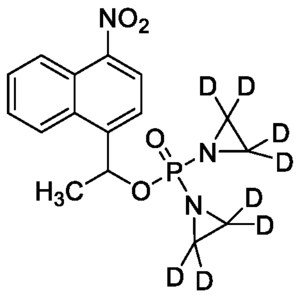

,

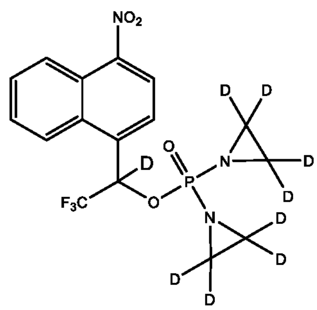

,

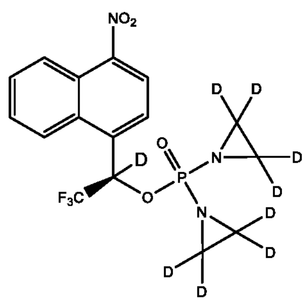

,

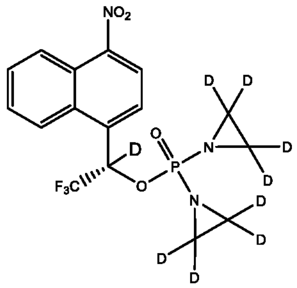 , 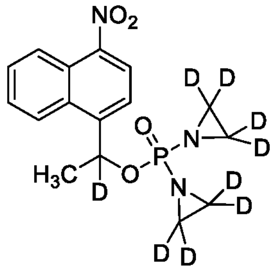 .

4. A medicament comprising the compound of any one of claims 1 to 3 or a pharmaceutically acceptable salt, a prodrug, a solvate or an isotopic variant thereof.

5. Use of the medicament of claim 4 for the treatment of cancer, tumor, conditions caused by cancer or tumor, or cell proliferative diseases.

6. The use according to claim 5, wherein the cancer or tumor is non-small cell lung cancer, pancreatic cancer, colorectal cancer, liver cancer, or gastric cancer.

7. The use according to claim 5, wherein the cancer or tumor is primary brain cancer or tumor, or metastatic cancer or tumor that metastasizes to the brain.

8. The use according to claim 5, wherein the patient with cancer or tumor is a patient with impaired DNA repair.

9. The use according to claim 8, wherein the impaired DNA repair is one or more of impaired homologous recombination DNA repair enzymes, impaired nucleotide excision repair enzymes, impaired non-homologous end joining enzymes, impaired base excision repair enzymes, impaired mismatch repair enzymes, or at least one impaired repair enzyme in a fanconi anemia pathway.

10. The use according to claim 5, wherein the patient with cancer or tumor is a patient with BRCA2 gene mutation.

11. Use of the compound of any one of claims 1 to 3, or a pharmaceutically acceptable salt, a prodrug, a solvate or an isotopic variant thereof in the manufacture of a medicament for the treatment of cancer, tumor, conditions caused by cancer or tumor, or cell proliferative diseases.

12. The use according to claim 11, wherein the cancer or tumor is non-small cell lung cancer, pancreatic cancer, colorectal cancer, liver cancer, or gastric cancer.

13. The use according to claim 11, wherein the cancer or tumor is primary brain cancer or tumor, or metastatic cancer or tumor that metastasizes to the brain.

14. The use according to claim 11, wherein the patient with cancer or tumor is a patient with impaired DNA repair.

15. The use according to claim 14, wherein the impaired DNA repair is one or more of impaired homologous recombination DNA repair enzymes, impaired nucleotide excision repair enzymes, impaired non-homologous end joining enzymes, impaired base excision repair enzymes, impaired mismatch repair enzymes, or at least one impaired repair enzyme in a fanconi anemia pathway.

16. The use according to claim 11, wherein the patient with cancer or tumor is a patient with BRCA2 gene mutation.

17. A combined drug, which comprises the compound of any one of claims 1 to 3 or a pharmaceutically acceptable salt, a prodrug, a solvate or an isotopic variant thereof and

a. a conventional chemotherapeutic drug;
b. an anti-angiogenic drug;
c. a cell checkpoint inhibitor; or
d. an immunosuppressive agent.

**18.** A combined therapy for the treatment of cancers or tumors, wherein the therapy comprises administering to the patient a drug containing the compound of any one of claims 1 to 3 or a pharmaceutically acceptable salt, a prodrug, a solvate or an isotopic variant thereof; and

a. administering a conventional chemotherapeutic drug;
b. administering an anti-angiogenic drug;
c. administering a cell checkpoint inhibitor; or
d. administering an immunosuppressive agent.

**19.** A method for preparing any one of the compounds of the following structure I, wherein the method comprises subjecting compound I-l to a ring closure reaction to give the corresponding compound I:

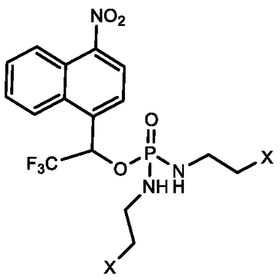
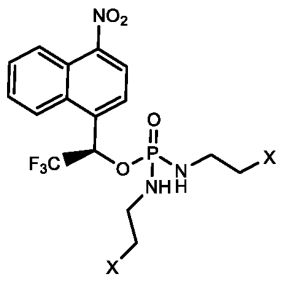
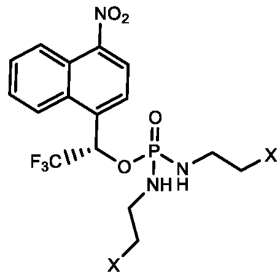
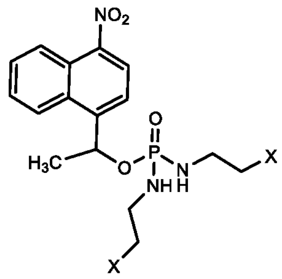

I-1

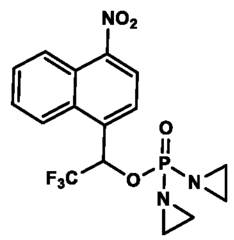
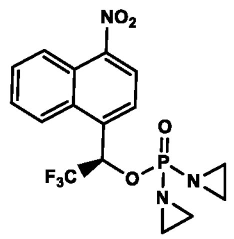
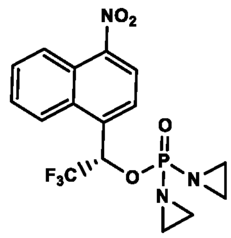
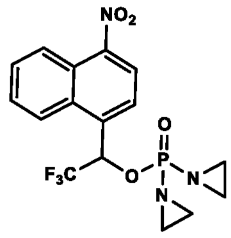

I

wherein X is independently F, Cl, Br, or I.

**20.** The method according to claim 19, wherein the ring closure reaction uses silver oxide or silver nitrate as a catalyst, with or without the addition of a base.

**21.** The method according to claim 19, wherein all of X is Br.

**22.** The method according to claim 20, wherein the organic base is N, N-diisopropylethylamine DIEA or triethylamine TEA.

**23.** A compound having the following structure I-1:

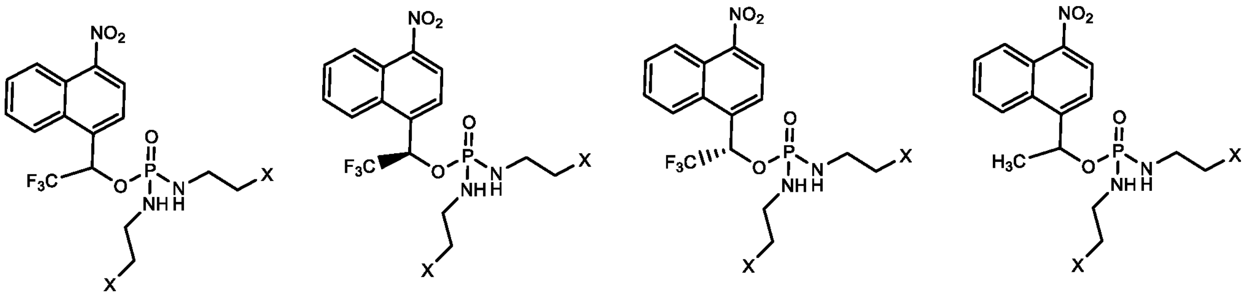

I-1

wherein X is independently F, Cl, Br, or I.

**24.** Use of the compound of claim 23 as an intermediate for the synthesis of the compound of claim 1.

**25.** A method for the preparation of a compound of the following structure I-1, wherein the method comprises first reacting a compound I-2 as the starting reactant with phosphorus oxychloride $POCl_3$ respectively to obtain an intermediate, then second reacting the intermediate with haloethanamine or the hydrohalide salt of haloethanamine, and finally obtaining the corresponding compound I-1:

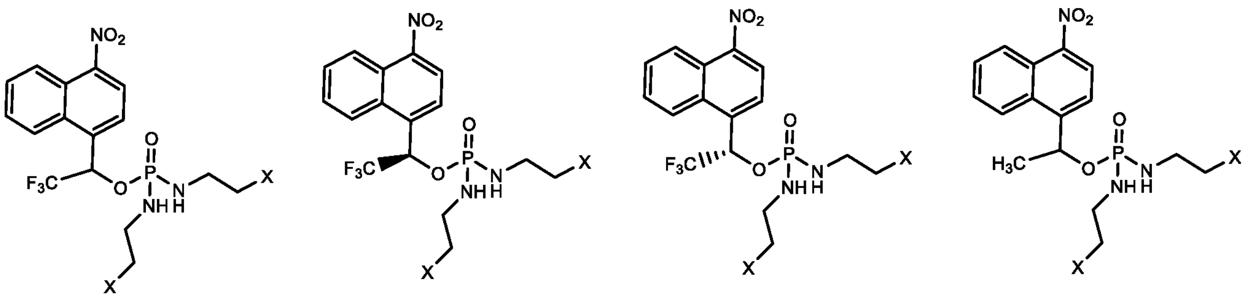

I-1

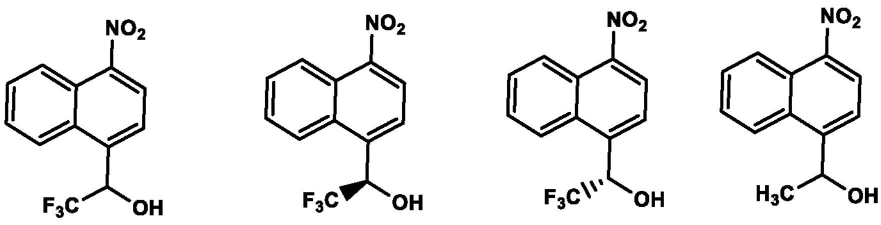

I-2

wherein X is independently F, Cl, Br, or I.

**26.** The method according to claim 25, wherein the haloethylamine is bromoethylamine and the hydrohalide of haloethylamine is the hydrobromide of bromoethylamine.

**27.** A method for preparing a compound of the following structure I-2, wherein the method comprises:

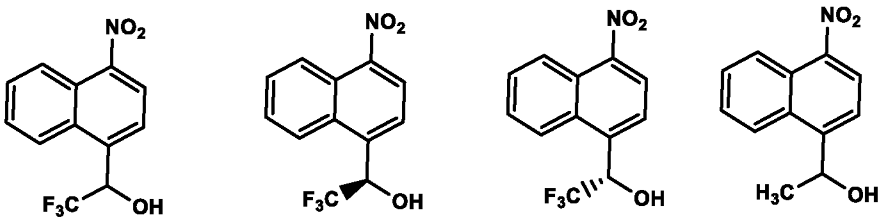

I-2

reacting compound I-3 with $TMSCF_3$ in contact; hydrolyzing after deprotection operation to obtain the racemic alcohols I-2-1 and I-2-4; and performing chiral resolution operation on the racemic alcohol I-2-1 to obtain chiral alcohol I-2-2 and chiral alcohol I-2-3, respectively,

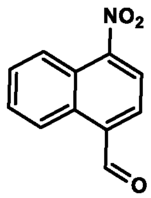

I-3

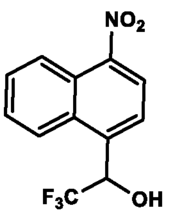
I-2-1

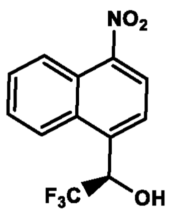
I-2-2

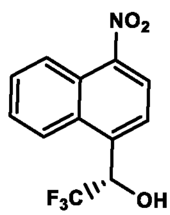
I-2-3

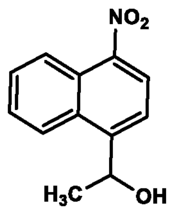
I-2-4

.

**28.** The method according to claim 27, wherein the deprotection reagent used in the deprotection operation is tetrabutylammonium fluoride TBAF, and ammonium fluoride, aqueous ammonium chloride or hydrochloric acid was used for the hydrolysis operation.

**29.** The method according to claim 27, wherein the method of chiral resolution includes crystallization method and chemical resolution method.

**30.** A method for separating the racemate in the compound according to claim 1 to obtain one of the enantiomers or to increase the concentration of any enantiomeric excess of the compound in the mixture, wherein the method comprises the steps of: a) subjecting the racemic compound to an optical analysis, wherein by chiral chromatography comprising stationary phase and mobile phase, wherein the stationary phase comprises silica gel impregnated with a functionalized polysaccharide, and wherein the mobile phase comprises an alcohol and a further solvent.

**31.** The method according to claim 30, wherein the alcohol is ethanol and the further solvent is n-hexane.

**32.** A method for preparing a deuterated compound of the following structure I-4, wherein the method comprises subjecting compound I-5 to a ring closure reaction to give the corresponding compound I-4

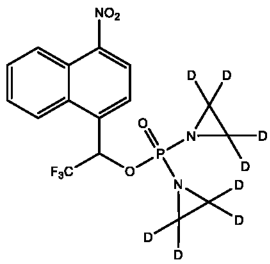
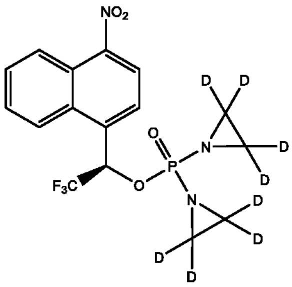
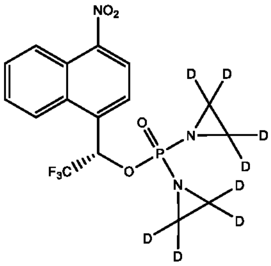
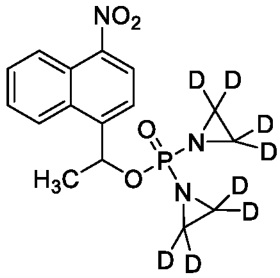

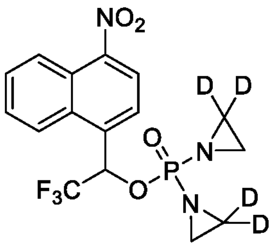
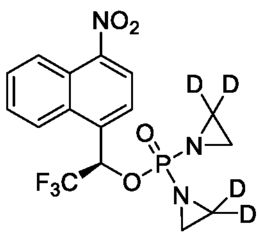
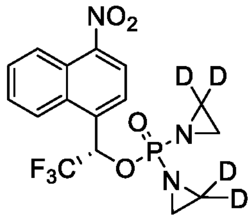
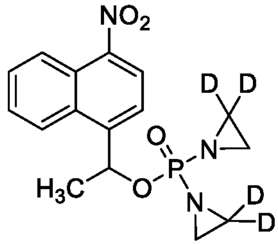

I-4

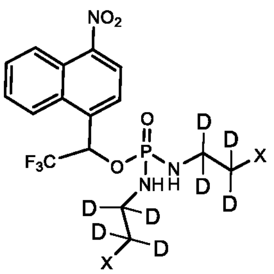
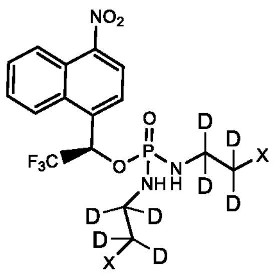
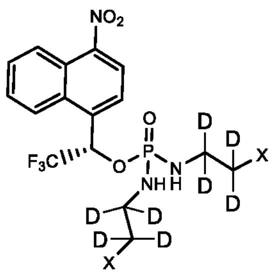
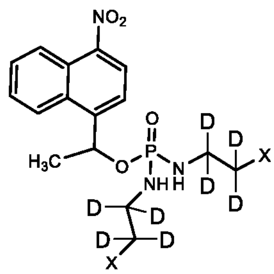

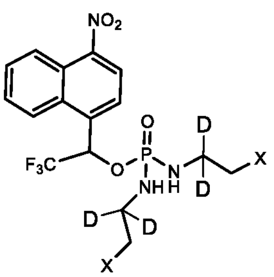
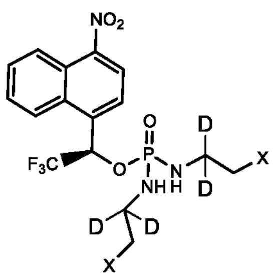
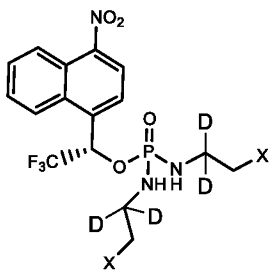
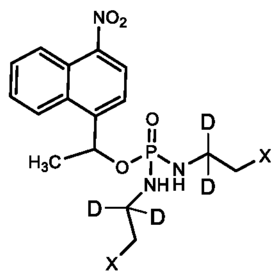

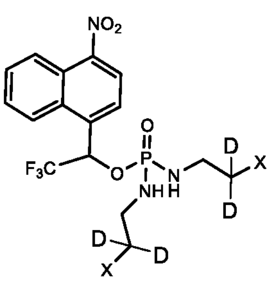
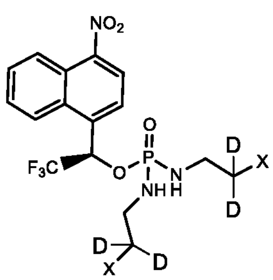
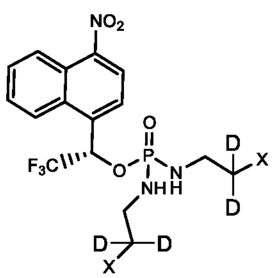
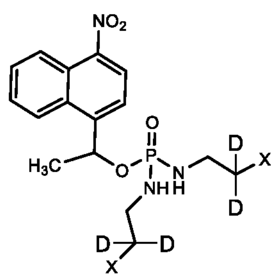

I-5

wherein X is independently F, Cl, Br, or I, preferably Br;
the ring closure reaction uses the silver oxide or silver nitrate as a catalyst, with or without the addition of an organic base.

**33.** A method for the preparation of a deuterated compound of the following structure I-5, wherein the method comprises first reacting a compound I-2 as the starting reactant to react with phosphorus oxychloride $POCl_3$ respectively to obtain an intermediate, then second reacting the intermediate with the corresponding deuterated haloethanamine I-6 or the hydrohalide salt of deuterated haloethanamine, and finally obtaining the corresponding compound I-5:

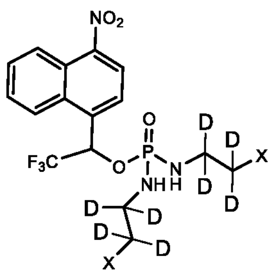
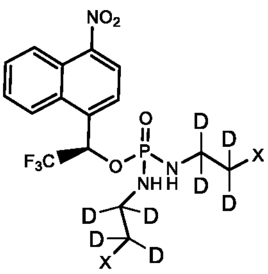
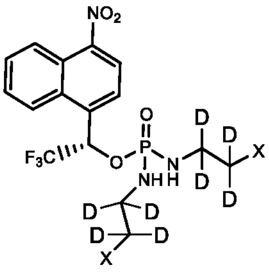
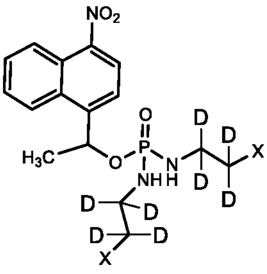
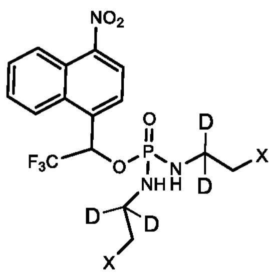
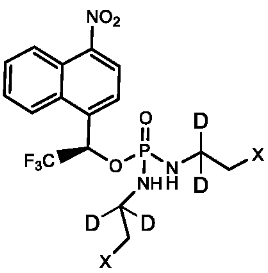
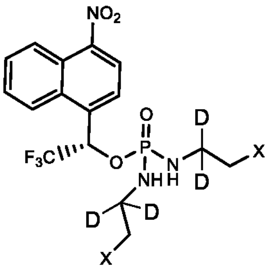
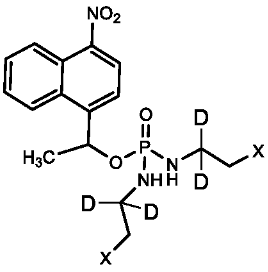
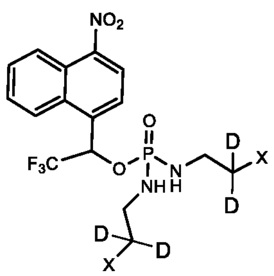
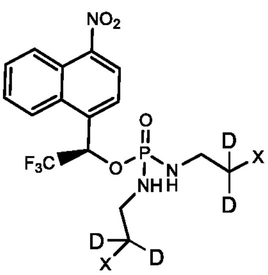
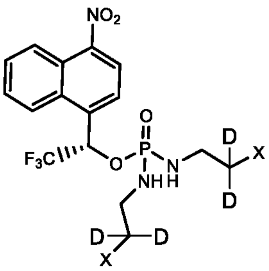
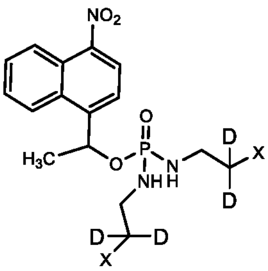

I-5

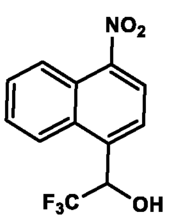
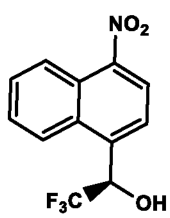
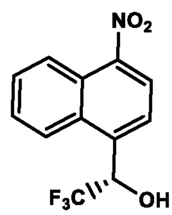
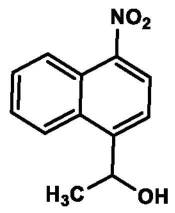

I-2

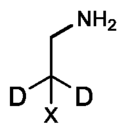 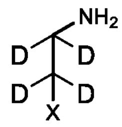

I-6

wherein X is independently F, Cl, Br, or I;

the halogen in the hydrohalide salt of the deuterated haloethylamine is the same as the halogen in the deuterated haloethylamine;

preferably, the haloethylamine is bromoethylamine and the hydrohalide salt of the haloethylamine is the hydrobromide salt of the bromoethylamine.

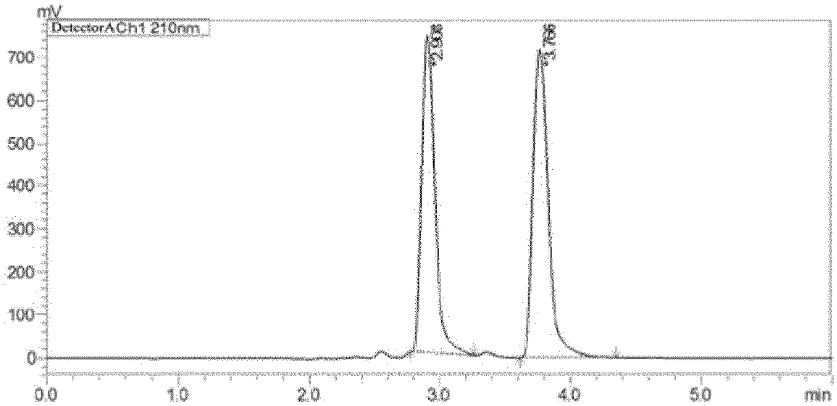


Figure 1

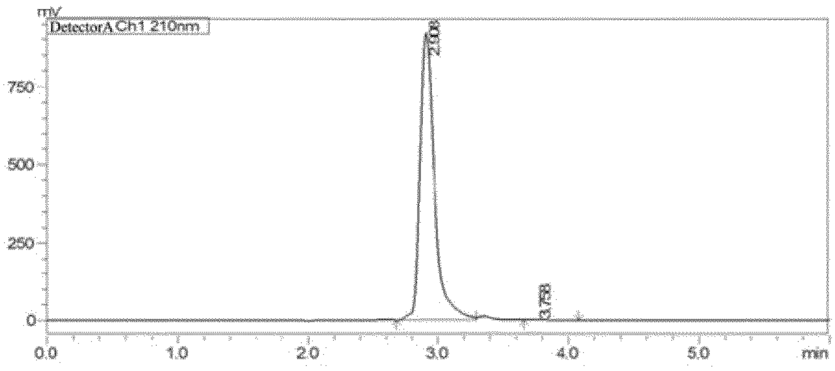


Figure 2

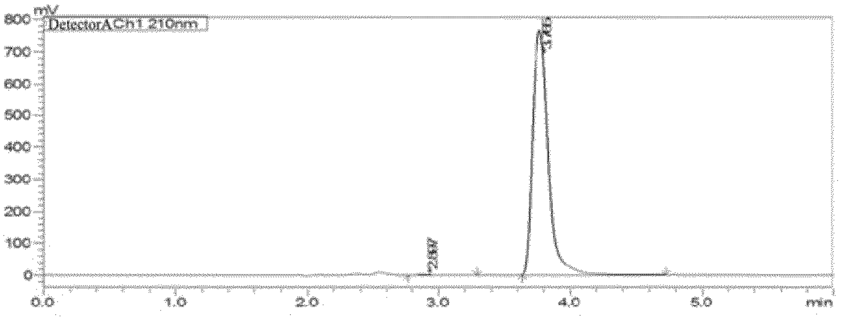


Figure 3

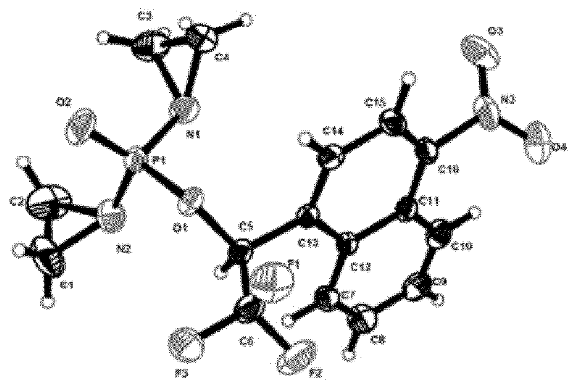

Figure 4

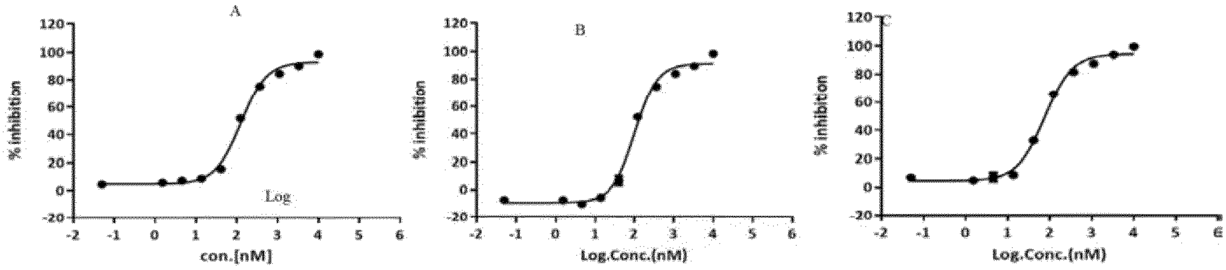


Figure 5

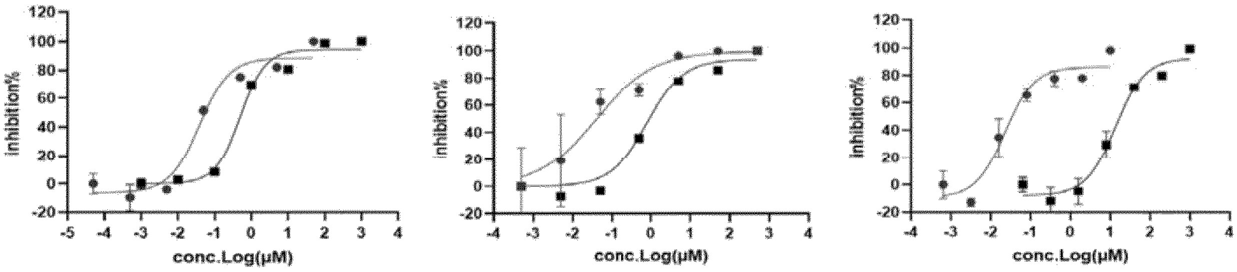


Figure 6

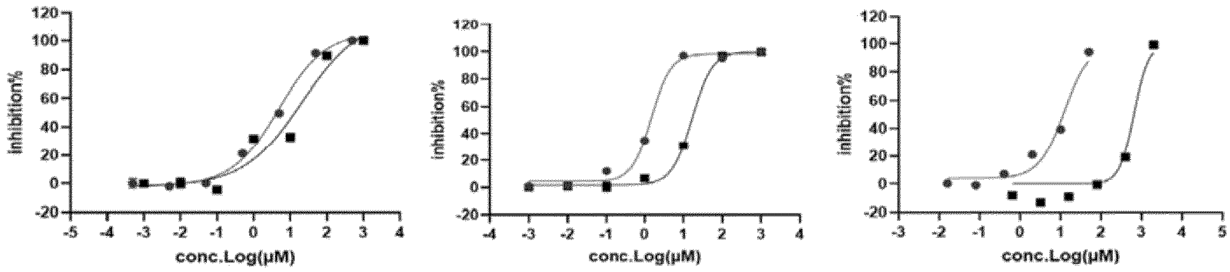


Figure 7

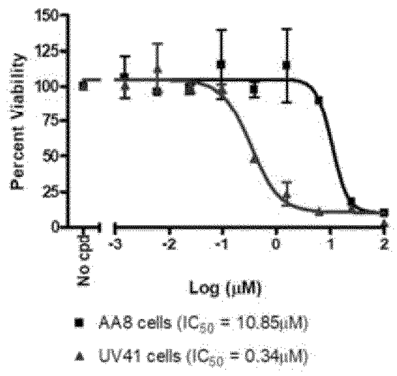


Figure 8

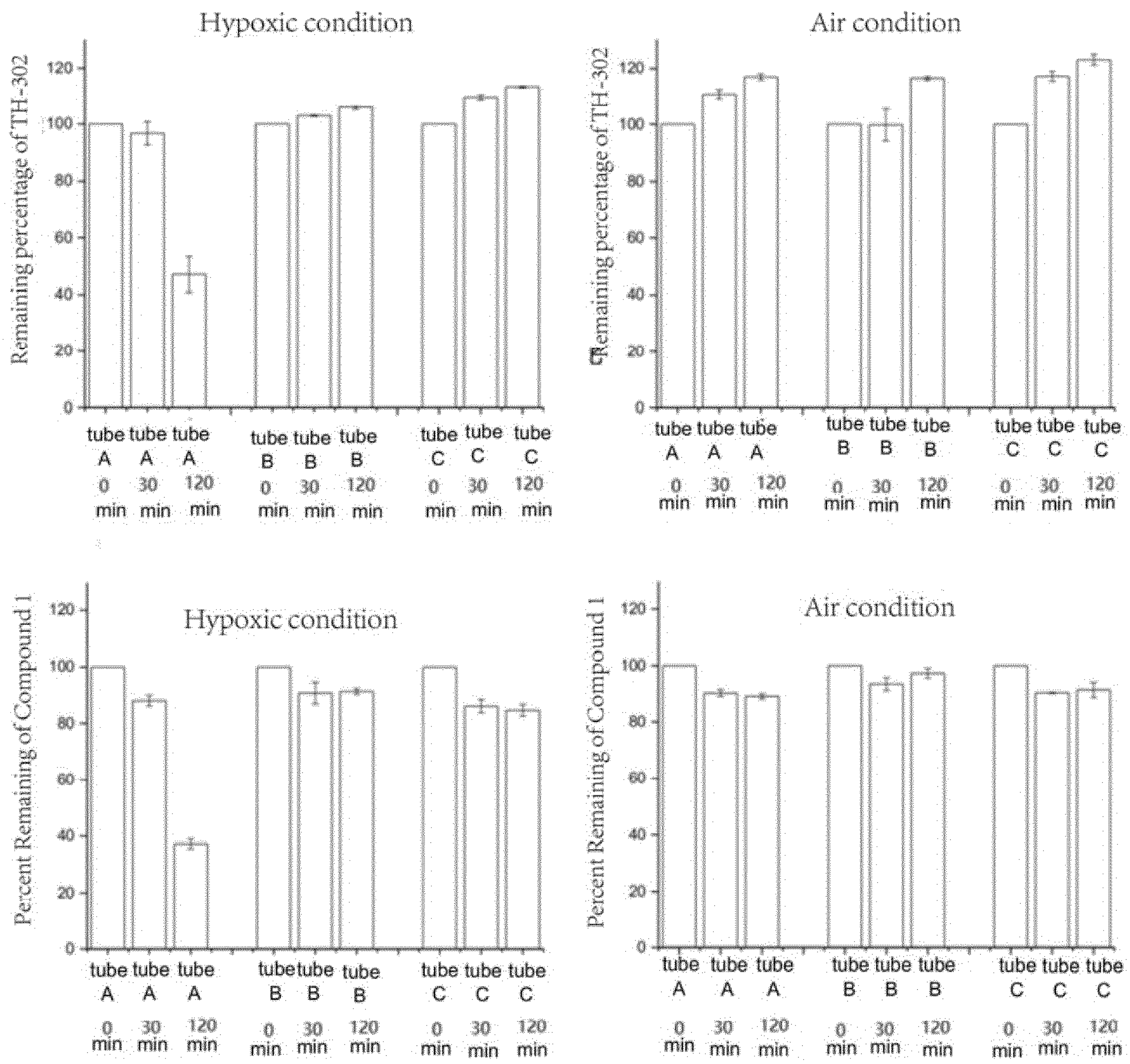


Figure 9

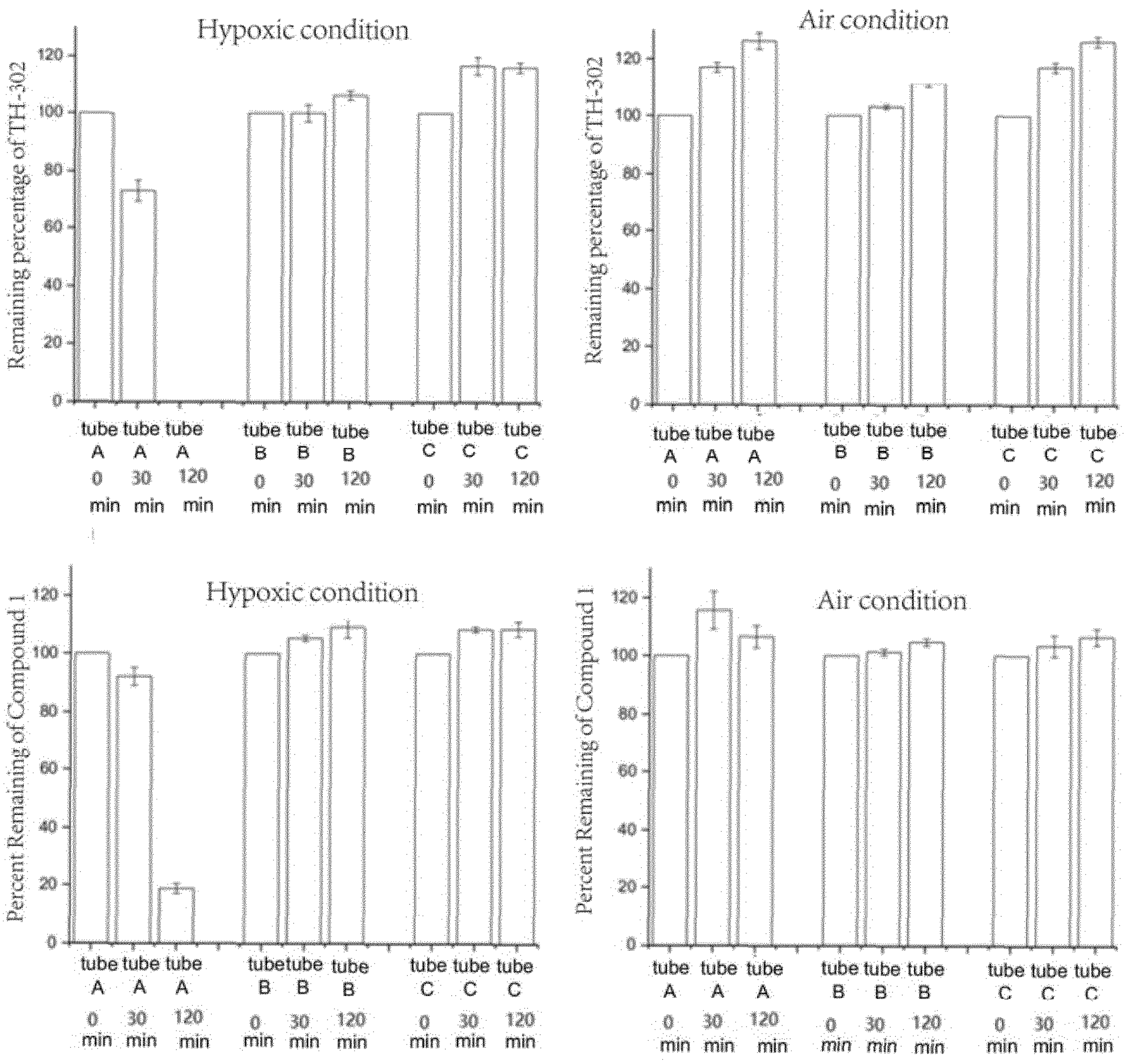


Figure 10

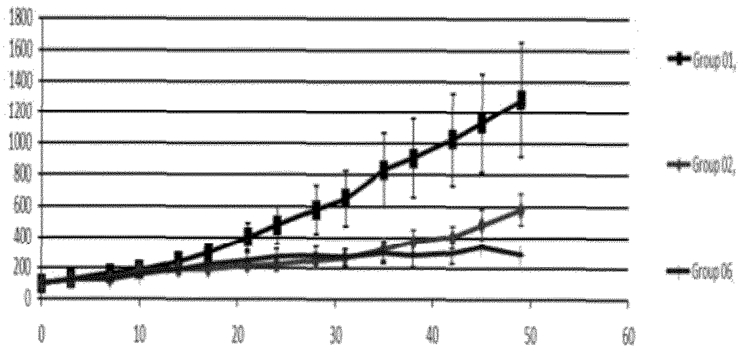


Figure 11

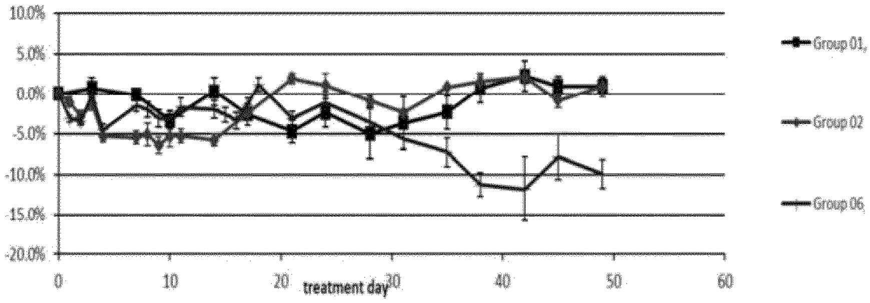


Figure 12

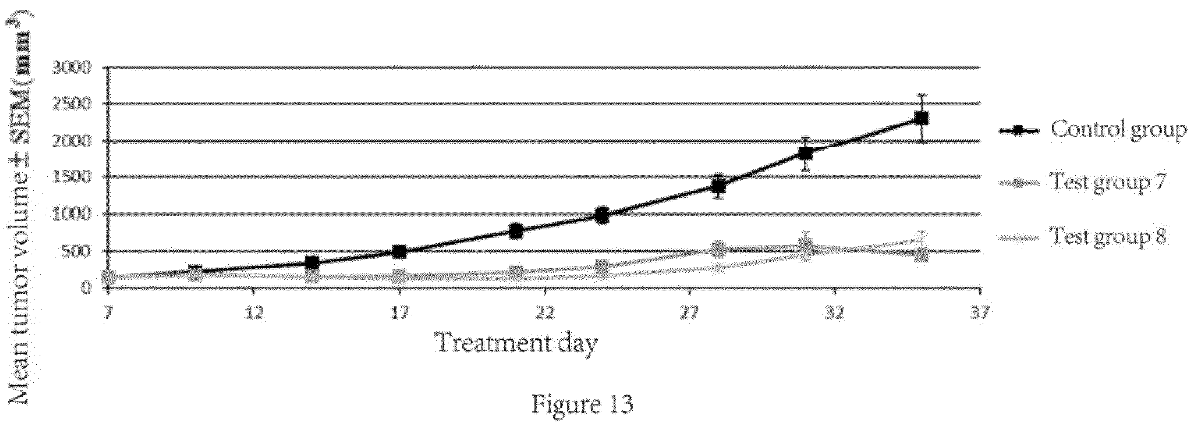


Figure 13

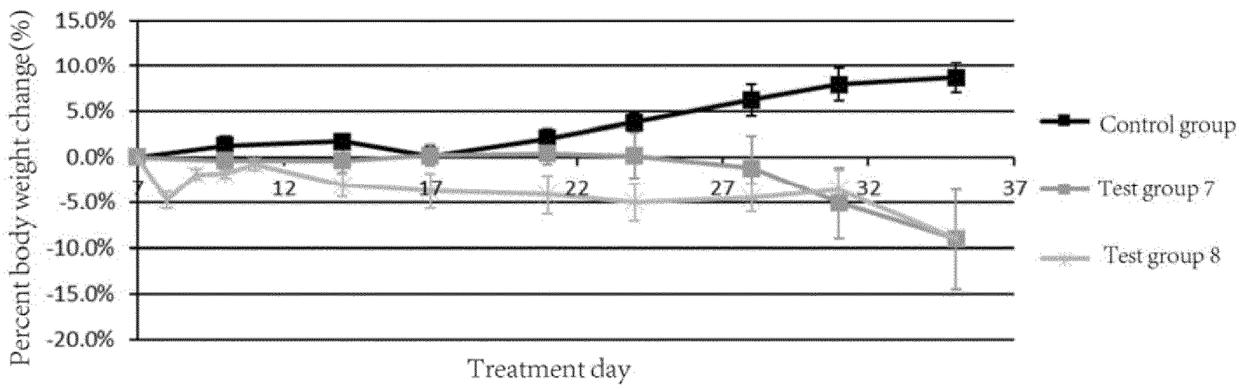


Figure 14

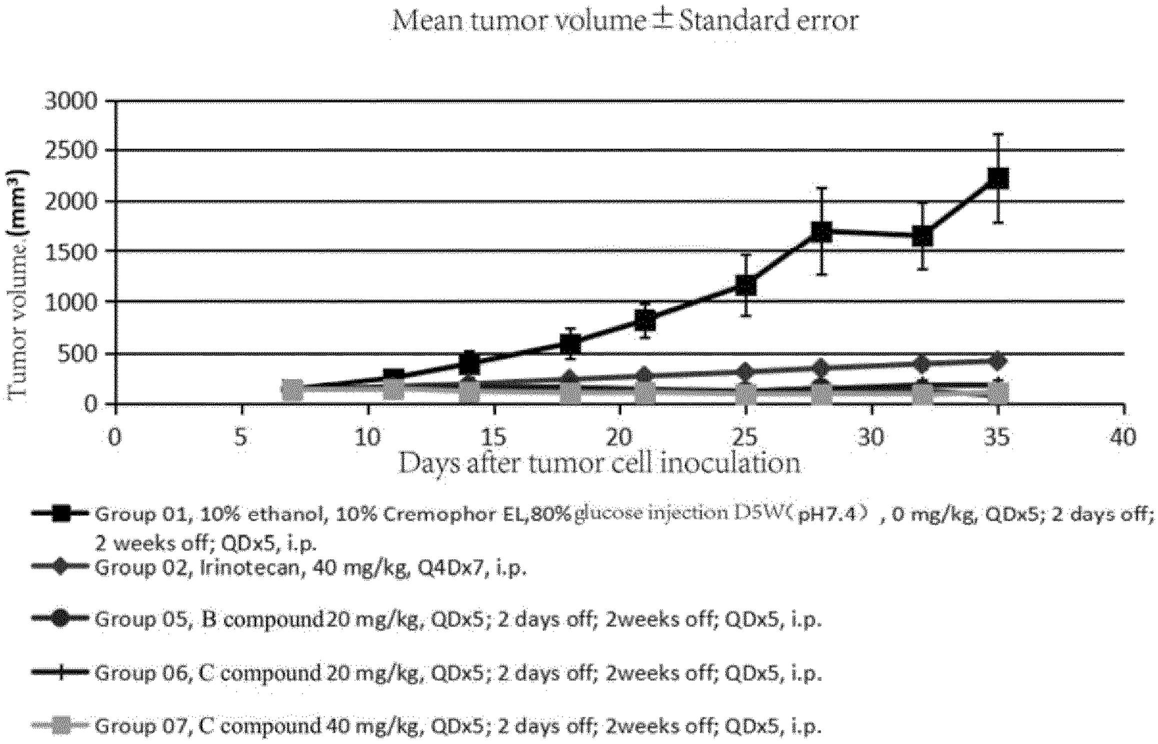


Figure 15

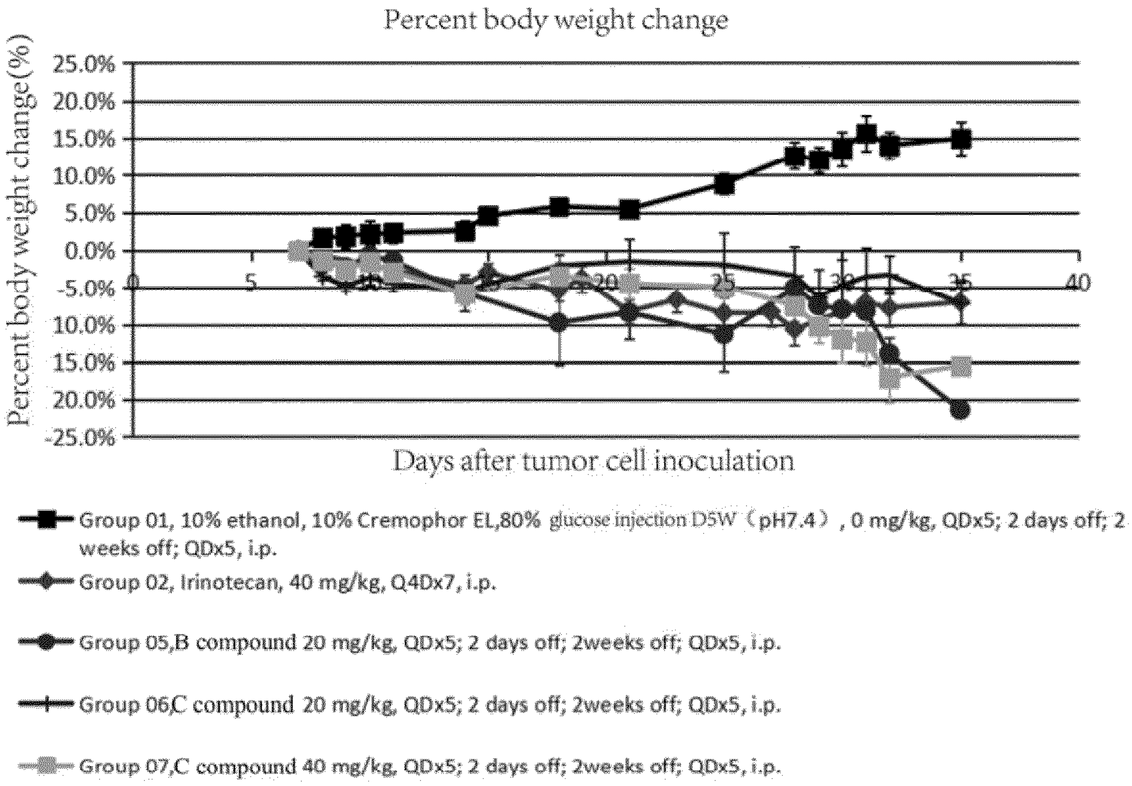


Figure 16

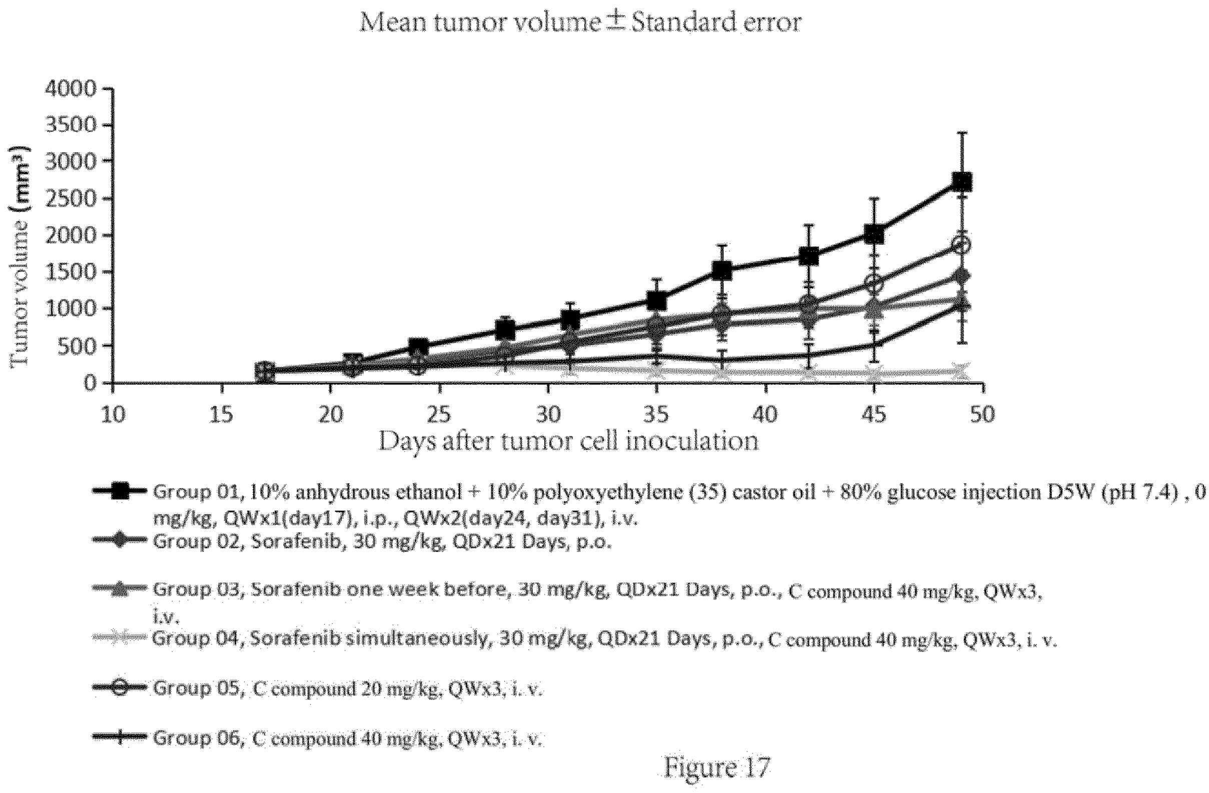


Figure 17

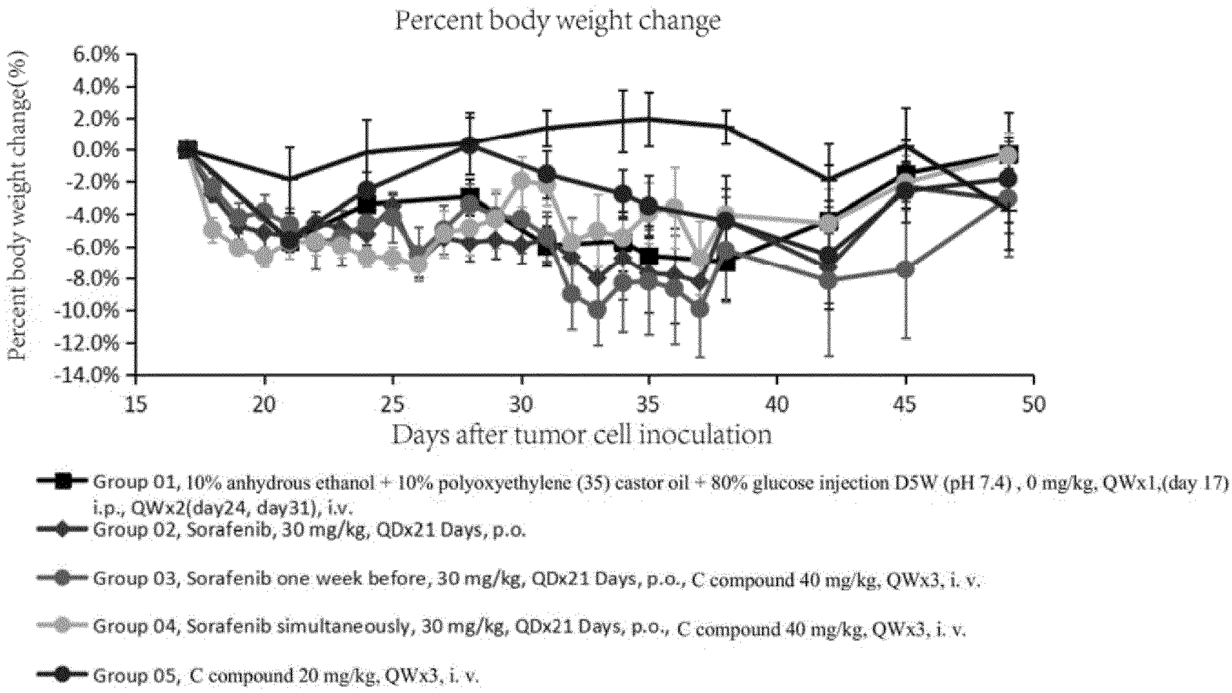


Figure 18

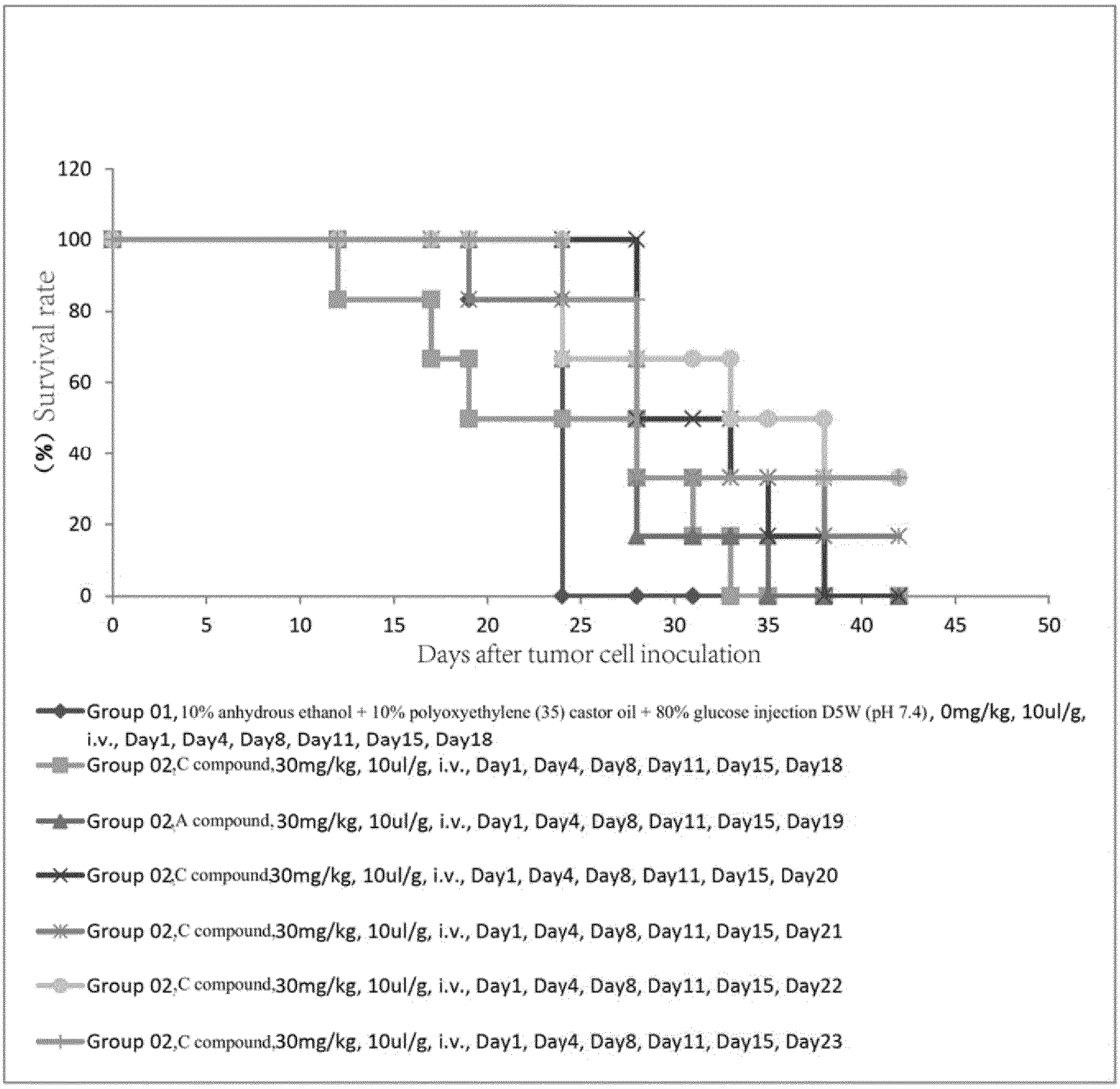
120
100
80
60
40
20
0
(%) Survival rate
0
5
10
15
20
25
30
35
40
45
50
Days after tumor cell inoculation
Group 01, 10% anhydrous ethanol + 10% polyoxyethylene (35) castor oil + 80% glucose injection D5W (pH 7.4), 0mg/kg, 10ul/g, i.v., Day1, Day4, Day8, Day11, Day15, Day18
Group 02,C compound,30mg/kg, 10ul/g, i.v., Day1, Day4, Day8, Day11, Day15, Day18
Group 02,A compound, 30mg/kg, 10ul/g, i.v., Day1, Day4, Day8, Day11, Day15, Day19
Group 02,C compound30mg/kg, 10ul/g, i.v., Day1, Day4, Day8, Day11, Day15, Day20
Group 02,C compound,30mg/kg, 10ul/g, i.v., Day1, Day4, Day8, Day11, Day15, Day21
Group 02,C compound,30mg/kg, 10ul/g, i.v., Day1, Day4, Day8, Day11, Day15, Day22
Group 02,C compound,30mg/kg, 10ul/g, i.v., Day1, Day4, Day8, Day11, Day15, Day23

Figure 19

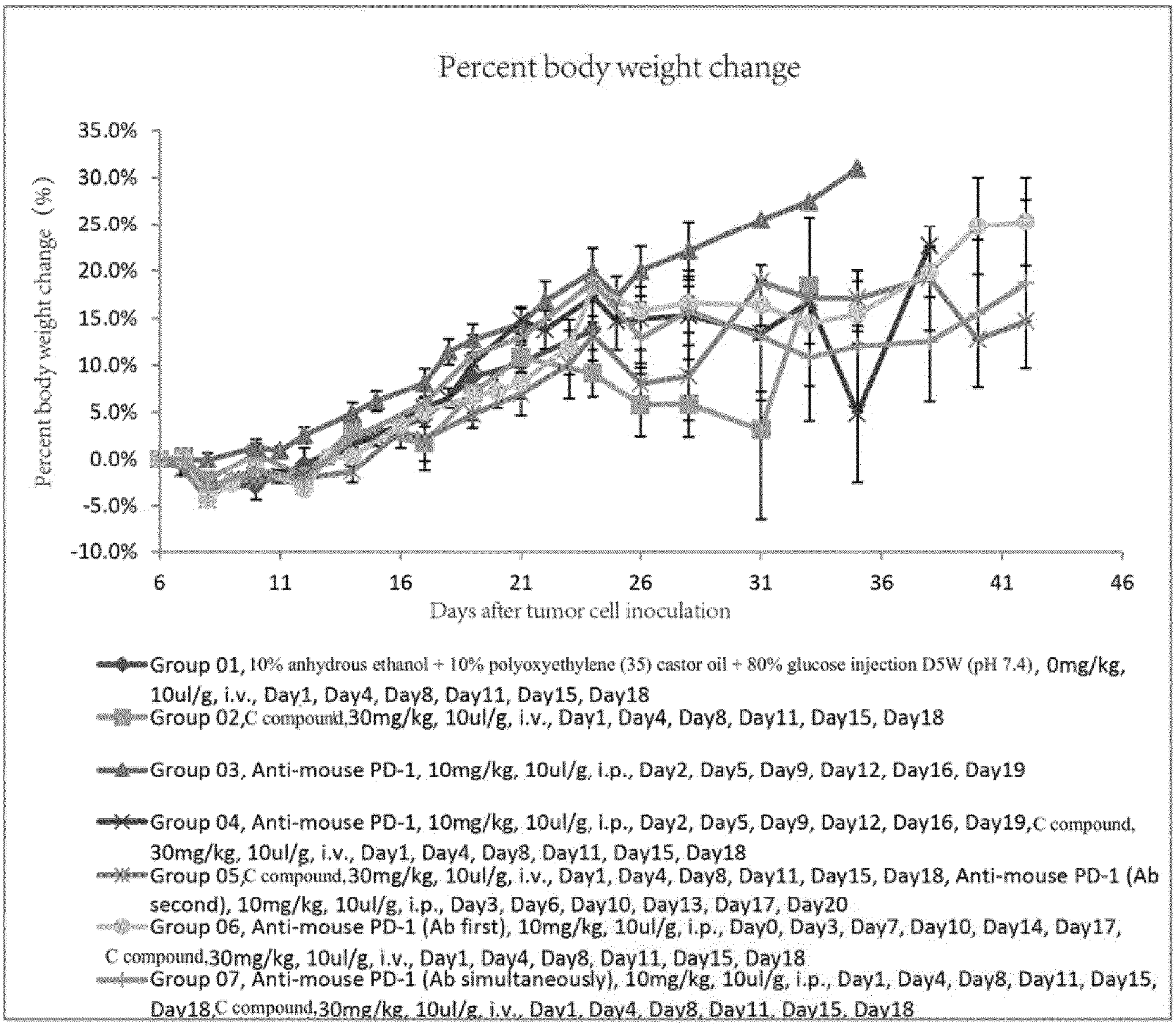
Percent body weight change
35.0%
30.0%
25.0%
20.0%
15.0%
10.0%
5.0%
0.0%
-5.0%
-10.0%
Percent body weight change (%)
6
11
16
21
26
31
36
41
46
Days after tumor cell inoculation
Group 01, 10% anhydrous ethanol + 10% polyoxyethylene (35) castor oil + 80% glucose injection D5W (pH 7.4), 0mg/kg, 10ul/g, i.v., Day1, Day4, Day8, Day11, Day15, Day18
Group 02, C compound, 30mg/kg, 10ul/g, i.v., Day1, Day4, Day8, Day11, Day15, Day18
Group 03, Anti-mouse PD-1, 10mg/kg, 10ul/g, i.p., Day2, Day5, Day9, Day12, Day16, Day19
Group 04, Anti-mouse PD-1, 10mg/kg, 10ul/g, i.p., Day2, Day5, Day9, Day12, Day16, Day19, C compound, 30mg/kg, 10ul/g, i.v., Day1, Day4, Day8, Day11, Day15, Day18
Group 05, C compound, 30mg/kg, 10ul/g, i.v., Day1, Day4, Day8, Day11, Day15, Day18, Anti-mouse PD-1 (Ab second), 10mg/kg, 10ul/g, i.p., Day3, Day6, Day10, Day13, Day17, Day20
Group 06, Anti-mouse PD-1 (Ab first), 10mg/kg, 10ul/g, i.p., Day0, Day3, Day7, Day10, Day14, Day17, C compound, 30mg/kg, 10ul/g, i.v., Day1, Day4, Day8, Day11, Day15, Day18
Group 07, Anti-mouse PD-1 (Ab simultaneously), 10mg/kg, 10ul/g, i.p., Day1, Day4, Day8, Day11, Day15, Day18, C compound, 30mg/kg, 10ul/g, i.v., Day1, Day4, Day8, Day11, Day15, Day18

Figure 20

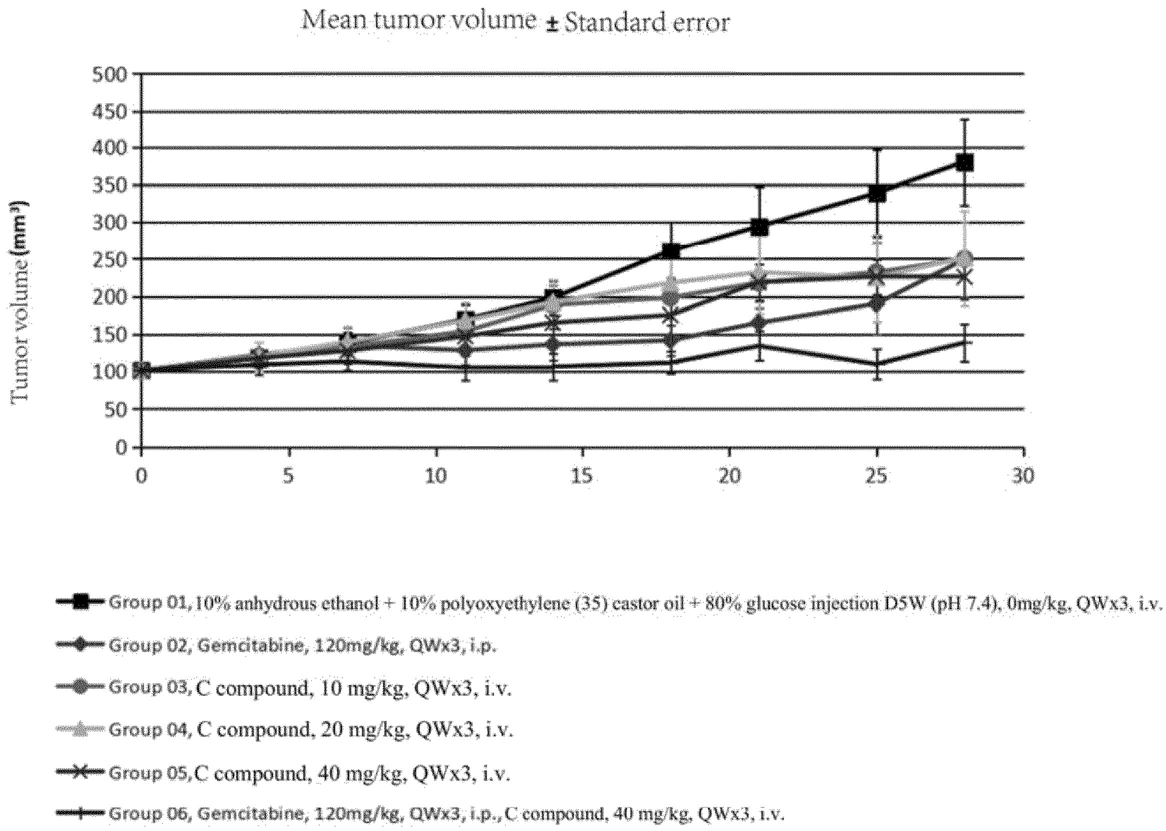


Figure 21

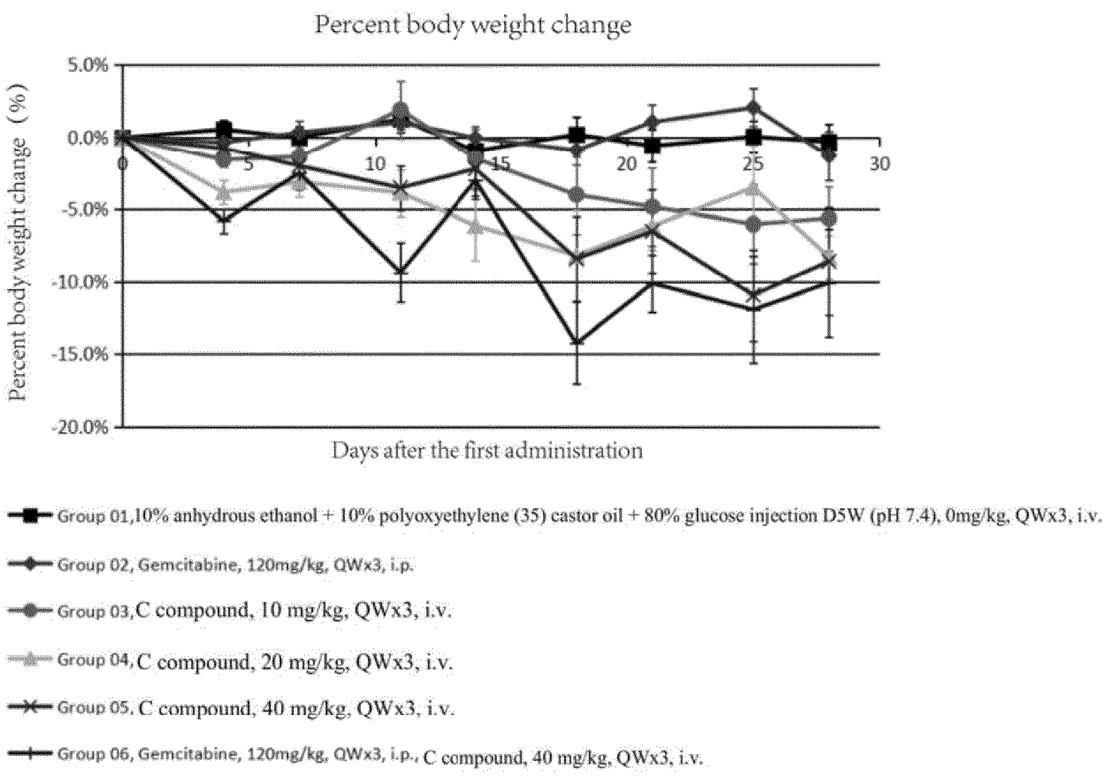


Figure 22

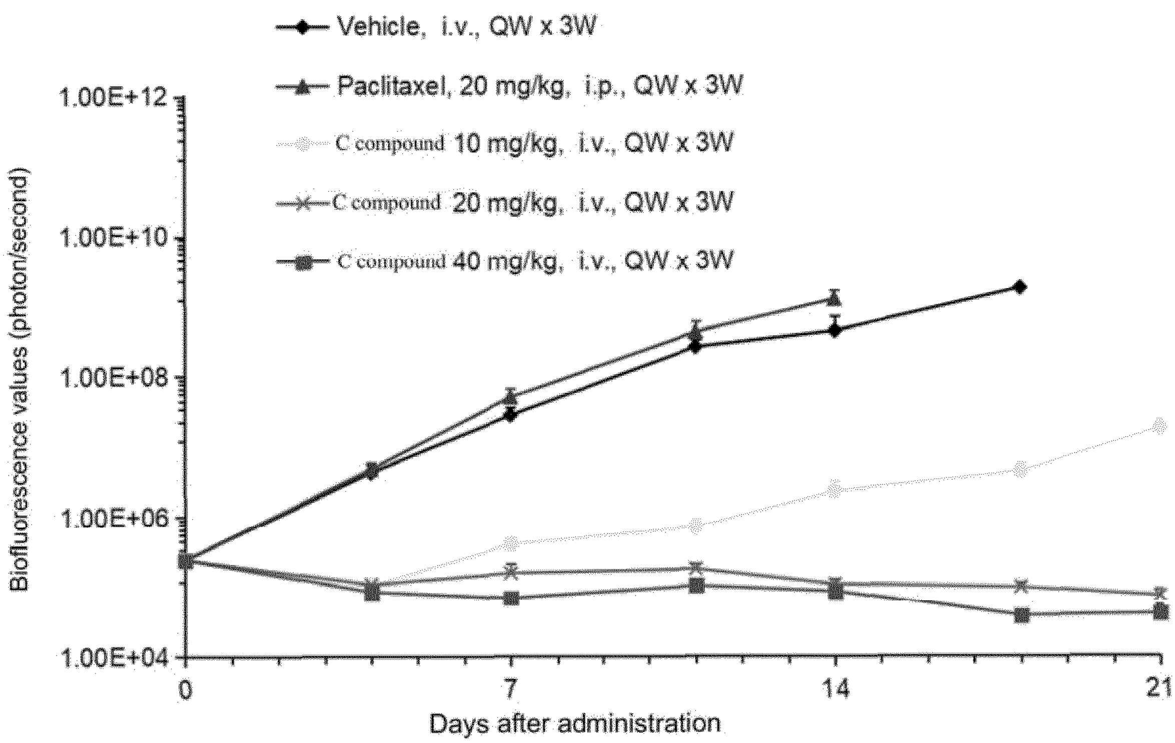


Figure 23

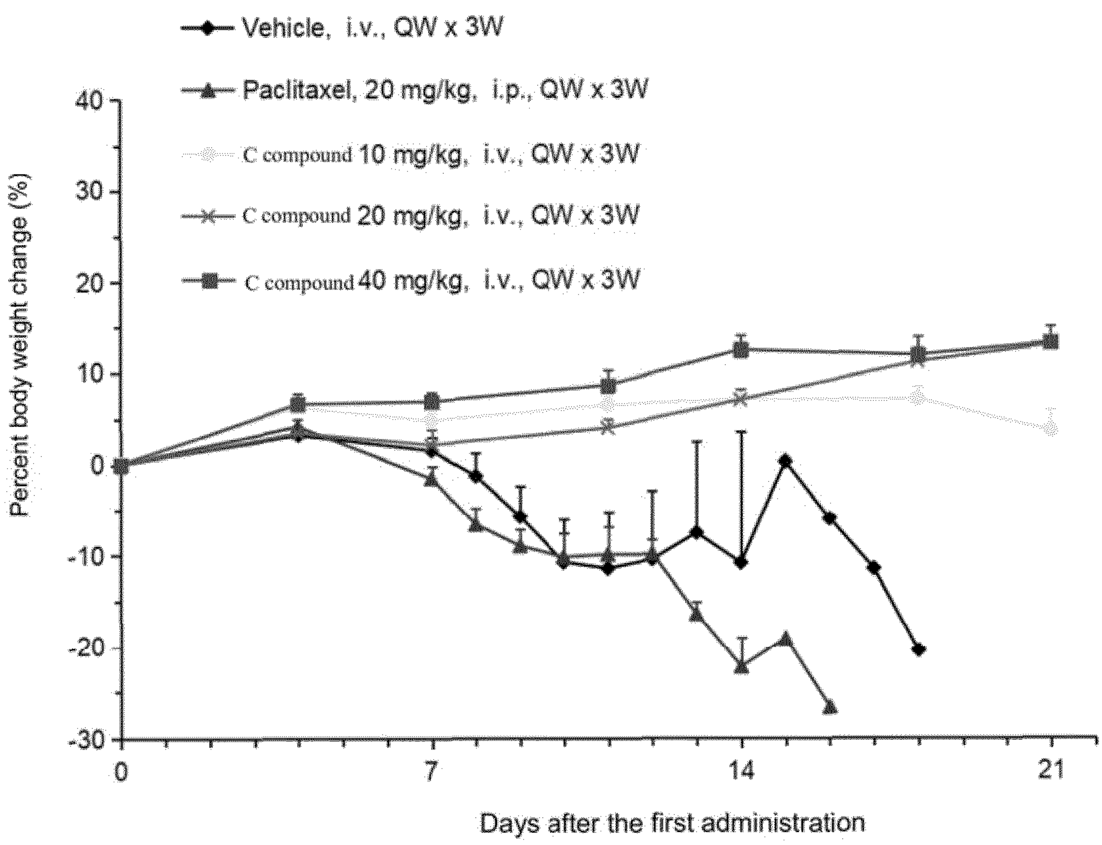


Figure 24

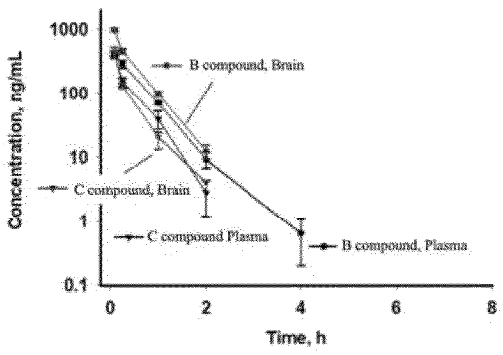


Figure 25

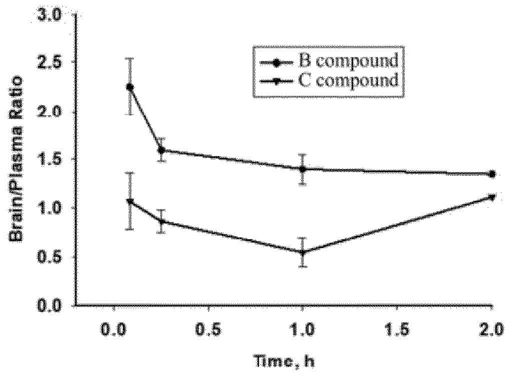


Figure 26

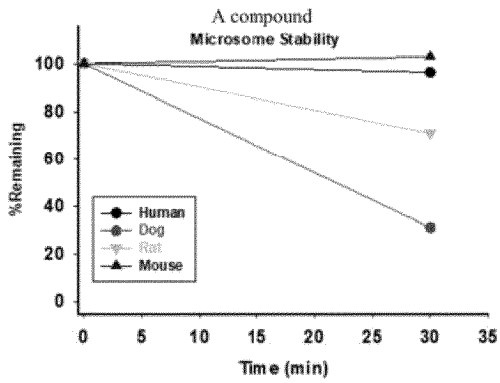


Figure 27

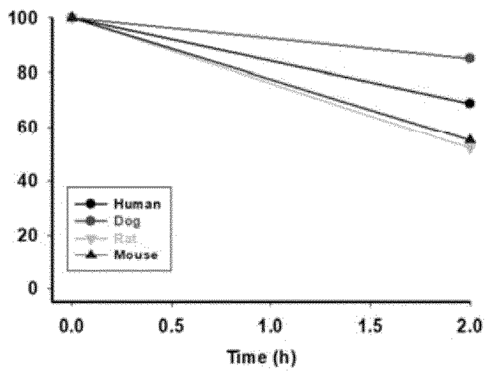


Figure 28

## INTERNATIONAL SEARCH REPORT

International application No.
**PCT/CN2021/101870**

**A. CLASSIFICATION OF SUBJECT MATTER**

C07F 9/564(2006.01)i； A61K 31/06(2006.01)i； A61K 31/664(2006.01)i； A61P 35/00(2006.01)i

According to International Patent Classification (IPC) or to both national classification and IPC

**B. FIELDS SEARCHED**

Minimum documentation searched (classification system followed by classification symbols)

C07F; A61K; A61P

Documentation searched other than minimum documentation to the extent that such documents are included in the fields searched

Electronic data base consulted during the international search (name of data base and, where practicable, search terms used)

DWPI; SIPOABS; CNABS; CNTXT; CNKI; STNext: 深圳艾欣达伟医药; 李安蓉; 烷化剂; 乏氧; 同位素; 氘; structure search; alkylating agents; anoxia; isotopes; deuterium; structure search

**C. DOCUMENTS CONSIDERED TO BE RELEVANT**

| Category* | Citation of document, with indication, where appropriate, of the relevant passages | Relevant to claim No. |
|---|---|---|
| X | CN 108024974 A (MOLECULAR TEMPLATES, INC.) 11 May 2018 (2018-05-11) description paragraphs 10-11, 33, 35, 93, embodiment 1 | 1, 4-24, 27-31 |
| Y | CN 108024974 A (MOLECULAR TEMPLATES, INC.) 11 May 2018 (2018-05-11) description paragraphs 10-11, 33, 35, 93, embodiment 1 | 2-18, 25-26, 32-33 |
| Y | CN 108290911 A (THRESHOLD PHARMACEUTICALS, INC.) 17 July 2018 (2018-07-17) claims 1, 10-11; description paragraph 0041, embodiment 2 | 2-18, 25-26, 32-33 |
| A | CN 107530556 A (THRESHOLD PHARMACEUTICALS, INC.) 02 January 2018 (2018-01-02) claims 1-24, embodiment 1 | 1-33 |
| A | CN 107118231 A (PEKING UNIVERSITY) 01 September 2017 (2017-09-01) claims 1-7 | 1-33 |
| A | WO 2019062919 A1 (OBI PHARMA, INC. et al.) 04 April 2019 (2019-04-04) entire document | 1-33 |
| A | WO 0104130 A1 (PURDUE RESEARCH FOUNDATION et al.) 18 January 2001 (2001-01-18) claims 1-33 | 1-33 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2021** | **26 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/101870**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **5-10,18**
because they relate to subject matter not required to be searched by this Authority, namely:

[1] Claims 5 -10 and 18 relate to a method for treating cancer, a tumor, a disorder caused by a cancer or a tumor, or a cell proliferative disease, and therefore do not comply with PCT Rule 39.1 (iv). A search has been carried out on the basis of the subject matter title referring to a drug and a compound drug according to claim 4 for preparing drugs for treating cancer, a tumor, or a disease caused by cancer or a tumor, or a cell proliferative disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/101870**

| Patent document cited in search report | | | Publication date (day/month/year) | Patent family member(s) | | | Publication date (day/month/year) |
|---|---|---|---|---|---|---|---|
| CN | 108024974 | A | 11 May 2018 | JP | 2018527301 | A | 20 September 2018 |
| | | | | US | 2018169064 | A1 | 21 June 2018 |
| | | | | EP | 3313385 | A1 | 02 May 2018 |
| | | | | US | 10668047 | B2 | 02 June 2020 |
| | | | | EP | 3313385 | B8 | 21 April 2021 |
| | | | | CA | 2990665 | A1 | 29 December 2016 |
| | | | | TW | 201713646 | A | 16 April 2017 |
| | | | | EP | 3313385 | A4 | 23 January 2019 |
| | | | | US | 2020085786 | A1 | 19 March 2020 |
| | | | | WO | 2016210175 | A1 | 29 December 2016 |
| | | | | EP | 3313385 | B1 | 03 March 2021 |
| | | | | AU | 2016282785 | A1 | 15 February 2018 |
| CN | 108290911 | A | 17 July 2018 | CN | 108290911 | B | 08 May 2020 |
| | | | | BR | 112017025778 | A2 | 14 August 2018 |
| | | | | TW | 201726695 | A | 01 August 2017 |
| | | | | ES | 2781398 | T3 | 01 September 2020 |
| | | | | CA | 2990696 | A1 | 26 May 2017 |
| | | | | EP | 3390415 | A4 | 22 May 2019 |
| | | | | KR | 101985778 | B1 | 04 June 2019 |
| | | | | IL | 256569 | D0 | 28 February 2018 |
| | | | | EP | 3390415 | A1 | 24 October 2018 |
| | | | | AU | 2016357728 | B2 | 27 August 2020 |
| | | | | KR | 20170130615 | A | 28 November 2017 |
| | | | | BR | 112017025778 | B1 | 16 March 2021 |
| | | | | JP | 2018517710 | A | 05 July 2018 |
| | | | | TW | I702222 | B | 21 August 2020 |
| | | | | JP | 6695360 | B2 | 20 May 2020 |
| | | | | WO | 2017087428 | A1 | 26 May 2017 |
| | | | | HK | 1250988 | A1 | 18 January 2019 |
| | | | | EP | 3390415 | B1 | 26 February 2020 |
| | | | | AU | 2016357728 | A1 | 30 November 2017 |
| CN | 107530556 | A | 02 January 2018 | JP | 6704421 | B2 | 03 June 2020 |
| | | | | SG | 11201707293V | A | 30 October 2017 |
| | | | | EP | 3277380 | A4 | 01 August 2018 |
| | | | | KR | 20190072688 | A | 25 June 2019 |
| | | | | KR | 102034618 | B1 | 21 October 2019 |
| | | | | SG | 10201913462 X | A | 30 March 2020 |
| | | | | WO | 2016145092 | A1 | 15 September 2016 |
| | | | | AU | 2016229136 | B2 | 09 August 2018 |
| | | | | CN | 107530556 | B | 10 April 2020 |
| | | | | EP | 3747508 | A1 | 09 December 2020 |
| | | | | KR | 20170128280 | A | 22 November 2017 |
| | | | | US | 10364261 | B2 | 30 July 2019 |
| | | | | EP | 3277380 | B1 | 16 September 2020 |
| | | | | IL | 254377 | D0 | 30 November 2017 |
| | | | | AU | 2016229136 | A1 | 05 October 2017 |
| | | | | KR | 20190072689 | A | 25 June 2019 |
| | | | | JP | 2018513876 | A | 31 May 2018 |
| | | | | BR | 112017019287 | A2 | 02 May 2018 |
| | | | | US | 2019225633 | A1 | 25 July 2019 |

Form PCT/ISA/210 (patent family annex) (January 2015)

**INTERNATIONAL SEARCH REPORT**
**Information on patent family members**

International application No.
**PCT/CN2021/101870**

| Patent document cited in search report | | | Publication date (day/month/year) | Patent family member(s) | | | Publication date (day/month/year) |
|---|---|---|---|---|---|---|---|
| | | | | US | 2018044360 | A1 | 15 February 2018 |
| | | | | TW | 201706262 | A | 16 February 2017 |
| | | | | CA | 2979251 | C | 23 March 2021 |
| | | | | EP | 3277380 | A1 | 07 February 2018 |
| | | | | CA | 2979251 | A1 | 15 September 2016 |
| | | | | KR | 102189066 | B1 | 09 December 2020 |
| | | | | US | 10766914 | B2 | 08 September 2020 |
| | | | | TW | I674258 | B | 11 October 2019 |
| CN | 107118231 | A | 01 September 2017 | CN | 107118231 | B | 07 June 2019 |
| WO | 2019062919 | A1 | 04 April 2019 | TW | 201919644 | A | 01 June 2019 |
| WO | 0104130 | A1 | 18 January 2001 | US | 7304046 | B2 | 04 December 2007 |
| | | | | US | 2003008850 | A1 | 09 January 2003 |
| | | | | US | 6656926 | B2 | 02 December 2003 |
| | | | | US | 2004176332 | A1 | 09 September 2004 |
| | | | | AU | 5935600 | A | 30 January 2001 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016210175 A1
- CN 108024974 A


### Non-patent literature cited in the description


- *CHEMICAL ABSTRACTS,* 918633-87-1
- **VANHOEFER U ; CAO S ; HARSTRICK A ; SEEBER S ; RUSTUM YM.** Comparative antitumor efficacy of docetaxel and paclitaxel in nude mice bearing human tumor xenografts that overexpress the multidrug resistance protein(MRP). *Ann Oncol,* 1997, vol. 8, 1221-1228
- **BIGIONI M ; BENZO A ; IRRISSUTO C ; LOPEZ G ; CURATELLA B ; MAGGI CA ; MANZINI S ; CREA A ; CAROLI S ; CUBADDA F.** Antitumor effect of combination treatment with Sabarubicin (MEN 10755) and cis-platin (DDP) in human lung tumor xenograft. *Cancer Chemother Pharmacol,* 2008, vol. 62, 621-629
- **HUBER PE ; BISCHOF M ; JENNE J ; HEILAND S ; PESCHKE P ; SAFFRICH R ; GRONE HJ ; DEBUS J ; LIPSON KE ; ABDOLLAHI A.** Trimodal cancer treatment: beneficial effects of combined antiangiogenesis, radiation, and chemotherapy. *Cancer Res,* 2005, vol. 65, 3643-3655
- **HOUGHTON JA ; CHESHIRE PJ ; HALLMAN JD, 2ND ; LUTZ L ; LUO X ; LI Y ; HOUGHTON PJ.** Evaluation of irinotecan in combination with 5 -fluorouracil or etoposide in xenograft models of colon adenocarcinoma and rhabdomyosarcoma. *Clin Cancer Res,* 1996, vol. 2, 107-118
- **KRAUS-BERTHIER L ; GUILBAUD N ; JAN M ; SAINT-DIZIER D ; ROUILLON MH ; BURBRIDGE MF ; PIERRE A ; ATASSI G.** Experimental antitumor activity of S 16020-2 in a panel of human tumors. *Eur J Cancer,* 1997, vol. 33, 1881-1887
- **MERRIMAN RL ; HERTEL LW ; SCHULTZ RM ; HOUGHTON PJ ; HOUGHTON JA ; RUTHERFORD PG ; TANZER LR ; BODER GB ; GRINDEY GB.** Comparison of the antitumor activity of gemcitabine and ara-C in a panel of human breast, colon, lung and pancreatic xenograft models. *Invest New Drugs,* 1996, vol. 14, 243-247
- **TEICHER BA ; CHEN V ; SHIH C ; MENON K ; FORLER PA ; PHARES VG ; AMSRUD T.** Treatment regimens including the multitargeted antifolate LY231514 in human tumor xenografts. *Clin Cancer Res,* 2000, vol. 6, 1016-1023
- **MIDDLETON MR ; THATCHER N ; MCMURRY TB ; MCELHINNEY RS ; DONNELLY DJ ; MARGISON GP.** Effect of O6-(4-bromothenyl)guanine on different temozolomide schedules in a human melanoma xenograft model. *Int J Cancer,* 2002, vol. 100, 615-617
- **LIU et al.** TH-302, a hypoxia-activated prodrug with broad in vivo preclinical combination therapy efficacy: optimization of dosing regimens and schedules. *Cancer Chemother Pharmacol,* 2012, vol. 69, 1487-1498
- **CHANG et al.** Sorafenib (BAY43-9006) inhibits tumor growth and vascularization and induces tumor apoptosis and hypoxia in RCC xenograft models. *Cancer Chemother Pharmacol,* 2007, vol. 59 (5), 561-74
- **SU D ; STAMATAKIS L ; SINGER EA ; SRINIVASAN R.** Renal cell carcinoma: molecular biology and targeted therapy. *Curr Opin Oncol,* 2014, (26), 321-7
- **SUN et al.** Combination treatment with hypoxia-activated prodrug evofosfamide (TH-302) and mTOR inhibitors results in enhanced antitumor efficacy in preclinical renal cell carcinoma models. *Am J Cancer Res.,* 2015, (5), 2139
- **MENG et al.** Enhancement of hypoxia-activated prodrug TH-302 anti-tumor activity by Chk1 inhibition. *BMC Cancer,* 2015, vol. 15, 422
- **WANG F Z ; FEI H R ; CUI, Y J et al.** The checkpoint 1 kinase inhibitor LY2603618 induces cell cycle arrest, DNA damage response and autophagy in cancer cells. *Apoptosis,* 2014, (19), 1389-1398
- **BALSINA et al.** Breaching the DNA damage checkpoint via PF-00477736, a novel small-molecule inhibitor of checkpoint kinase. *Mol Cancer Ther,* 2008, vol. 7 (8), 2394-404
- **MORGAN et al.** Mechanism of radiosensitization by the Chk1/2 inhibitor AZD7762 involves abrogation of the G2 checkpoint and inhibition of homologous recombinational DNA repair. *Cancer Researh,* 2010, (70), 4972
- **JAYAPRAKASH et al.** Targeted hypoxia reduction restores T cell infiltration and sensitizes prostate cancer to immunotherapy. *JCI,* 2018, (128), 5137

- **CURRAN et al.** PD-1 and CTLA-4 combination blockade expands infiltrating T cells and reduces regulatory T and myeloid cells within B16 melanoma tumors. *Proc Natl Acad Sci USA.,* 2010, vol. 107 (9), 4275-4280
- **NOGRADY.** Medicinal Chemistry A Biochemical Approach. Oxford University Press, 1985, 388-392
- **SUN J D ; LIU Q ; WANG J et al.** Selective tumor hypoxia targeting by hypoxia-activated prodrug TH-302 inhibits tumor growth in preclinical models of cancer.[J. *Clinical Cancer Research An Official Journal of the American Association for Cancer Research,* 2012, vol. 18 (3), 758-70
- **THOMPSON LH et al.** Repair of DNA adducts in asynchronous CHO cells and the role of repair in cell killing and mutation induction in synchronous cells treated with 7-bromomethylbenz[a]anthracene. *Somatic Cell Mol. Genet.,* 1984, vol. 10, 183-194
- **THOMPSON LH et al.** Genetic diversity of UV-sensitive DNA repair mutants of Chinese hamster ovary cells. *Proc. Natl. Acad. Sci. USA,* 1981, vol. 78, 3734-3737
- **HOY CA et al.** Defective DNA cross-link removal in Chinese hamster cell mutants hypersensitive to bifunctional alkylating agents. *Cancer Res.,* 1985, vol. 45, 1737-1743
- **BUSCH D et al.** Summary of complementation groups of UV-sensitive CHO cell mutants isolated by large-scale screening. *Mutagenesis,* 1989, vol. 4, 349-354
- **BESSHO T et al.** Initiation of DNA interstrand cross-link repair in humans: the nucleotide excision repair system makes dual incisions 5'' to the cross-linked base and removes a 22- to 28-nucleotide-long damage-free strand. *Mol. Cell. Biol.,* 1997, vol. 17, 6822-6830
- **THOMPSON LH et al.** Hypersensitivity to mutation and sister-chromatid-exchange induction in CHO cell mutants defective in incising DNA containing UV lesions. *Somatic Cell Genet.,* 1982, vol. 8, 759-773
- **JESSICA D. SUN ; QIAN LIU ; DHARMENDRA AHLUWALIA ; DAMIEN J. FERRARO ; YAN WANG ; DON JUNG ; MARK D. MATTEUCCI ; CHARLES P. HART.** Comparison of hypoxia-activated prodrug evofosfamide (TH-302) and ifosfamide in preclinical non-small cell lung cancer models[J. *Cancer Biology & Therapy,* 2016, vol. 17 (4), 371-380